(19) Europäisches Patentamt
European Patent Office
Office européen des brevets

(11) EP 3 213 240 B1

(12) **EUROPEAN PATENT SPECIFICATION**

(45) Date of publication and mention of the grant of the patent:
**20.01.2021 Bulletin 2021/03**

(21) Application number: **15820585.6**

(22) Date of filing: **27.10.2015**

(51) Int Cl.:
***G16B 15/00*** *(2019.01)* ***C07K 14/005*** *(2006.01)*
***C12N 7/00*** *(2006.01)* ***A61K 35/76*** *(2015.01)*
***G16B 50/00*** *(2019.01)* ***G16B 30/00*** *(2019.01)*

(86) International application number:
**PCT/IB2015/002153**

(87) International publication number:
**WO 2016/067094 (06.05.2016 Gazette 2016/18)**

(54) **METHODS AND SYSTEMS FOR IDENTIFYING LIGAND-PROTEIN BINDING SITES**

VERFAHREN UND SYSTEME ZUR IDENTIFIZIERUNG VON LIGANDENPROTEINBINDUNGSSTELLEN

MÉTHODES ET SYSTÈMES D’IDENTIFICATION DE SITES DE LIAISON LIGAND-PROTÉINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2014 US 201462068861 P**
**13.11.2014 US 201462079130 P**

(43) Date of publication of application:
**06.09.2017 Bulletin 2017/36**

(60) Divisional application:
**20213133.0**

(73) Proprietor: **King Abdullah University of Science and Technology**
**Thuwal 23955-6900 (SA)**

(72) Inventors:
- **GAO, Xin**
  **Thuwal 23955-6900 (SA)**
- **NAVEED, Hammad**
  **Thuwal 23955-6900 (SA)**

(74) Representative: **Ipsilon**
**Le Centralis**
**63, avenue du Général Leclerc**
**92340 Bourg-la-Reine (FR)**

(56) References cited:
**US-A1- 2013 280 238**

- **JOE DUNDAS ET AL: "Structural Signatures of Enzyme Binding Pockets from Order-Independent Surface Alignment: A Study of Metalloendopeptidase and NAD Binding Proteins", JOURNAL OF MOLECULAR BIOLOGY, ACADEMIC PRESS, UNITED KINGDOM, vol. 406, no. 5, 3 December 2010 (2010-12-03), pages 713-729, XP028144534, ISSN: 0022-2836, DOI: 10.1016/J.JMB.2010.12.005 [retrieved on 2010-12-09] cited in the application**
- **PAUL ASHFORD ET AL: "Visualisation of variable binding pockets on protein surfaces by probabilistic analysis of related structure sets", BMC BIOINFORMATICS, BIOMED CENTRAL, LONDON, GB, vol. 13, no. 1, 14 March 2012 (2012-03-14) , page 39, XP021124561, ISSN: 1471-2105, DOI: 10.1186/1471-2105-13-39**
- **WALLQVIST ANDERS ET AL: "Establishing connections between microarray expression data and chemotherapeutic cancer pharmacology", MOLECULAR CANCER THERAPEUTICS, vol. 1, no. 5, March 2002 (2002-03), pages 311-320, XP002756182, ISSN: 1535-7163**
- **WILLIAM R PITT ET AL: "Polyphony: superposition independent methods for ensemble-based drug discovery", BMC BIOINFORMATICS, BIOMED CENTRAL, LONDON, GB, vol. 15, no. 1, 30 September 2014 (2014-09-30), page 324, XP021199013, ISSN: 1471-2105, DOI: 10.1186/1471-2105-15-324**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ANATHE O. M. PATSCHULL ET AL: "In Silico Assessment of Potential Druggable Pockets on the Surface of [alpha]1-Antitrypsin Conformers", PLOS ONE, vol. 7, no. 5, 8 May 2012 (2012-05-08), page e36612, XP055262913, DOI: 10.1371/journal.pone.0036612
- STEFAN HENRICH ET AL: "Computational approaches to identifying and characterizing protein binding sites for ligand design", JOURNAL OF MOLECULAR RECOGNITION, 1 January 2009 (2009-01-01), pages n/a-n/a, XP55052028, ISSN: 0952-3499, DOI: 10.1002/jmr.984
- HAMMAD NAVEED ET AL: "An integrated structure- and system-based framework to identify new targets of metabolites and known drugs", BIOINFORMATICS., 18 August 2015 (2015-08-18), page btv477, XP55262669, GB ISSN: 1367-4803, DOI: 10.1093/bioinformatics/btv477

## Description

### Field of the Invention

The invention provides a novel integrated structure and system-based approach for ligand (e.g. drug) target prediction that enables the large-scale discovery of new targets for existing drugs. Novel computer-readable storage media and computer systems are also provided.

Methods and systems of the invention use novel sequence order-independent structure alignment, divisive hierarchical clustering, and probabilistic sequence similarity techniques to construct a probabilistic pocket ensemble (PPE) that captures even promiscuous structural features of different binding sites for a drug on known targets. The ligand's (e.g. drug's) PPE is combined with an approximation of the drug delivery profile to facilitate large-scale prediction of novel drug-protein interactions with several applications to biological research and drug development.

In a cross-validation study, exemplary methods of the invention predicted the known targets of eleven drugs with 63% sensitivity and 81% specificity. Using these novel methods, novel targets for these drugs were predicted, and two targets of high pharmacological interest (the nuclear receptor PPARγ, and the oncogene Bcl-2) were validated through *in vitro* binding experiments.

### Background of the Invention

Most metabolites and pharmaceutical drugs bind to more than one protein [1], resulting in a phenotype composed of many molecular (side) effects. For the pharmaceutical industry, predicting and minimizing the off-target effects is important because these cause low efficacy and high toxicity resulting in the high failure rate of new drugs in clinical trials [2-4]. Recent studies estimate that each drug on average binds to at least six known targets and many others are yet to be discovered [5, 6]. Thus, knowledge of off-target effects can help reducing drug resistance and provide opportunities for multi-target drug development [7]. Moreover, off-target ligands for a given binding site may inspire "drug repositioning", where a drug already approved for one condition is redirected to treat another condition, thus overcoming delays and cost associated with clinical trials and drug approval [8]. Therefore, predicting off-target binding sites to comprehensively understand the side effects of the drugs and exploit drug development/repositioning opportunities is central for rapid and cost-efficient drug development.

Apart from drug development, the identification of all cellular targets of a given biological cofactor, metabolite or other small-molecules is of great importance in understanding cellular function and dysfunction, for example in metabolome-target interactions and associated diseases. Finally, identification of possible targets for environmental pollutants may help understand and avoid health hazards from released chemicals.

Computational methods to predict new targets for existing endogenous or administered small-molecule compounds are therefore of high biological and pharmacological value. (For simplicity, we herein refer to all these compounds collectively as "drugs", in the meaning of "a small-molecule chemical substance with effects on a biological system".) These methods can be divided into three broad categories; structure based, expression based, and ligand based.

Structure-based methods utilize information from the drug targets by employing binding site similarity or molecular docking [9-12]; expression-based methods exploit the molecular activity perturbation signatures as a result of the activity of the drugs [13-19]; and ligand-based methods utilize the chemical and structural properties of the drug [20-22] to discover new targets. In addition to these methods, novel targets for existing drugs have also been predicted using side effect similarity [23], genome-wide association studies [24], and medical genetics [25]. Moreover, there are other methods that directly link drugs with diseases without specifying the drug targets by using drug-drug and disease-disease similarity measures [26].

Recently, methods that combine information from multiple sources have been introduced and are likely to be more successful in the future [27]. However, most of these methods do not provide an assessment of their performance by predicting the known drug targets (sensitivity analysis), only Chang *et al.* [11] and Li *et al.* [12] reported a true positive prediction rate, which, however, remains relatively low (29% and 49%, respectively). Moreover, a high-throughput framework based on structural information is still missing, and current methods do not capture satisfactorily the structural flexibility of a drug resulting in several conformationally different interactions with different targets.

Thus, the need exists for methods of predicting off-target binding sites to comprehensively understand the side effects of the drugs and exploit drug development/repositioning opportunities is central for rapid and cost-efficient drug development

### Summary of the Invention

The scope of the invention is defined by the appended claims We have discovered a novel integrated structure and system-based approach for ligand (e.g. drug) target prediction (iDTP) that enables the large-scale discovery of new

targets for existing drugs. Our novel method integrates structural signatures of small-molecule compounds with their tissue delivery profiles. If the required structural information is available, iDTP can be applied to all types of small molecules, including metabolites, cofactors and approved or experimental drugs.

Methods of the invention construct the structural signatures of a drug using sequence order-independent structure alignment, divisive hierarchical clustering, and probabilistic sequence similarity. This enables our claimed methods to capture features related to promiscuous target interactions and structural flexibility of a drug. The drug delivery profile is approximated by averaging the mRNA expression of all known protein targets. Through the combination of these orthogonal sources of information, iDTP enables large-scale computational prediction of novel drug targets, as supported by computational and experimental validation. Application of iDTP allowed us to propose a novel cellular target for coenzyme A (CoA), a novel druggable pocket and lead compound for Bcl-2, and plausible mechanistic information for the inhibition of CYP2E1 by Trolox.

In one embodiment, the invention provides a method for identifying a ligand binding site of a protein, the method comprising the steps of:

(a) generating stochastic basis sets of values representative of putative ligand-protein binding sites by (1) identifying information in a protein structure database, the information corresponding to known protein signature pockets and representing at least protein signature pocket atomic coordinates and conformation, and (2) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and known protein signature pockets;

(b) generating basis sets of values representative of nucleotide expression levels of genes that are known targets of the drug;

(c) selecting overlapping values from the basis sets generated in steps (a) and (b) as information which correlates to at least the atomic sequence and conformation of one or more ligand-protein binding sites; and

(d) validating the one or more ligand-protein binding sites determined in step (c) by measuring the affinity of the ligand for the one or more ligand-protein binding sites.

The term "basis sets" is used herein has the same meaning as in Dundas, et al. [29], *viz*. a "*basis set* of signature pockets, which represent the ensemble of differently sampled conformations for a functional family of proteins. As a basis set of signatures can represent many possible variations in shapes and chemical textures, it can represent structural features of an enzyme function with complex binding activities, and can also be used to accurately predict enzymes function." *See* Detailed Description of the Invention. As illustrated in *Dundas* [29], in selecting values from a hierarchical tree, "different basis sets of signature pockets can be produced at different levels of structural similarity by raising or lowering a similarity threshold. A low threshold will produce more signature pockets. As the threshold is raised, fewer signature pockets will be created. A single signature pocket can in principle be created to represent the full surface pocket data set by raising the threshold." The use of basis sets in linear algebra is explained in a variety of sources, including Http://mathworld.wolfram.com/VectorSpaceBasis.html.

In a preferred embodiment, the "nucleotide expression levels of genes" is an mRNA expression level of genes, the samples are tissue samples and:

(1) the distance function defined by the structural similarity and likely sequence similarity of putative ligand-protein binding sites and known protein signature pockets is represented by the formula:

$$Score = Structural\ similarity + \alpha*(1\text{-}Sequence\ similarity)$$

$$Structural\ similarity = \frac{RMSD}{N^{1/3}}$$

$$Sequence\ similarity = \frac{Sequence\ score}{Best\ sequence\ score}$$

$$Sequence\ score = \sum_i (AtomFreq_i + ResFreq_i)$$

$$Best\ sequence\ score = \sum_i (MaxAtomFreq_i + MaxResFreq_i)$$

where $\alpha$ is 1.2, *RMSD* is the root mean square distance after the alignment, *N* is the number of positions aligned, *AtomFreqi* / *ResFreqi* is the frequency of aligned atom/residue at position *i,* and *MaxAtomFreqi* / *MaxResFreqi* is the highest frequency of any atom/residue at position *i* and the summation is over all the aligned positions; and

(2) the distance function defined by the structural similarity, likely sequence similarity of putative ligand-protein binding sites and known protein signature pockets and relative tissue mRNA expression levels in the predetermined number of tissues is represented by the formula:

$$Score = Structural\ similarity + \alpha*(1\text{-}Sequence\ similarity) + \beta*Tissue\ expression$$

$$Tissue\ expression = 1\text{-}\frac{Number\ of\ tissues\ with\ matching\ expression}{Total\ number\ of\ tissues}$$

wherein a and the structural similarity term and sequence similarity term are as defined above and $\beta$ is a value between 0.1 and 0.9.

The CASTp pre-calculated database is one example of a preferred source of information regarding a protein's surface pockets and voids. Preferably, more than 30 apo structures per ligand are used to construct the probabilistic pocket ensemble and the known ligand-protein binding sites are determined by identifying the three largest pockets of the first chain of the protein's three-dimensional structure.

Preferably, the number of atoms in the putative ligand-protein binding sites is between about 10 to 500, more preferably 20, 30, 40 or 50 to 100, 200, 300 or 400, still more preferably about 50 to about 100 and mRNA expression levels of genes that have been correlated to known ligand-protein binding sites in a predetermined number of tissues are average values and the known ligand-protein binding sites mapped to more than one gene. Average mRNA expression levels can be determined using Uniprot ID mapping and in a preferred method, the affinity of the ligand for the predicted ligand-protein binding sites is measured using fluorescence anisotropy.

In another embodiment, the invention provides a method for identifying protein binding sites for a novel ligand, the method comprising the steps of:

(a) evaluating the binding of the novel ligand to one or more known protein binding sites, selecting protein binding sites which bind the novel ligand within a specified selectivity and identifying (preferably by X-ray crystallography) protein signature pockets which bind the ligand within a specified selectivity;

(b) generating stochastic basis sets of values representative of putative ligand-protein binding sites by (1) identifying in a protein structure database information corresponding to the protein signature pockets identified in step (a), the information representing at least protein signature pocket atomic coordinates and conformation, and (2) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and the protein signature pockets identified in step (a);

(c) generating stochastic basis sets of values representative of nucleotide (preferably mRNA) expression levels of genes that have been correlated to known ligand-protein binding sites in a predetermined number of biological samples (preferably tissues or cells) by mapping known ligand-protein binding site information to a gene name and identifying in a gene-sample expression database information representative of nucleotide (preferably mRNA) expression levels corresponding to the gene;

(d) selecting overlapping values from the basis sets generated in steps (b) and (c) as information which correlates to at least the atomic sequence and conformation of one or more ligand-protein binding sites; and

(e) validating the one or more ligand-protein binding sites determined in step (d) by measuring the affinity of the ligand for the one or more ligand-protein binding sites.

In a preferred embodiment of the above method for identifying protein binding sites for a novel ligand, the samples are tissue samples and the "nucleotide expression levels of genes" is a mRNA expression level of genes and:

(1) the distance function defined by the structural similarity and likely sequence similarity of putative ligand-protein binding sites and protein signature pockets identified in step (a) is represented by the formula:

$$Score = Structural\ similarity + \alpha * (1\text{-}Sequence\ similarity)$$

$$Structural\ similarity = \frac{RMSD}{N^{1/3}}$$

$$Sequence\ similarity = \frac{Sequence\ score}{Best\ sequence\ score}$$

$$Sequence\ score = \sum_i (AtomFreq_i + \mathrm{Res}Freq_i)$$

$$Best\ sequence\ score = \sum_i (MaxAtomFreq_i + Max\mathrm{Res}Freq_i)$$

where $\alpha$ is 1.2, *RMSD* is the root mean square distance after the alignment, *N* is the number of positions aligned, *AtomFreqi* / *ResFreqi* is the frequency of aligned atom/residue at position *i*, and *MaxAtomFreqi* / *MaxResFreqi* is the highest frequency of any atom/residue at position *i* and the summation is over all the aligned positions; and

(2) the distance function defined by the structural similarity, likely sequence similarity of putative ligand-protein binding sites and protein signature pockets identified in step (a) and relative tissue mRNA expression levels in the predetermined number of tissues is represented by the formula:

$$Score = Structural\ similarity + \alpha * (1\text{-}Sequence\ similarity) + \beta * Tissue\ \exp ression$$

$$Tissue\ \exp ression = 1 - \frac{Number\ of\ tissues\ with\ matching\ \exp ression}{Total\ number\ of\ tissues}$$

wherein $\alpha$ and the structural similarity term and sequence similarity term are as defined above and $\beta$ is a value between 0.1 and 0.9.

In other preferred methods, the ligand is selected from the group consisting of a small molecule or a nucleic acid and at least one step of the method is done *in silico.*

The invention also provides non-transitory computer-readable storage media with an executable program stored thereon, and computer systems comprising a processor, a memory and non-transitory computer-readable storage media, to implement the methods of the invention.

In still another embodiment, the invention provides a network system for identifying a ligand binding site of a protein, the network system comprising:

(a) computer system comprising a processor, a memory and a non-transitory computer-readable storage medium that stores instructions that, when executed by the processor, cause the system to:

(1) generate stochastic basis sets of values representative of putative ligand-protein binding sites by (i) identifying information in a protein structure database, the information corresponding to known protein signature pockets and representing at least protein signature pocket atomic coordinates and conformation, and (ii) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and known protein signature pockets;

(2) generate stochastic basis sets of values representative of nucleotide expression levels of genes that have been correlated to known ligand-protein binding sites in a predetermined number of biological samples (preferably tissue or cell samples) by mapping known ligand-protein binding site information to a gene name and identifying in a gene- biological sample expression database information representative of nucleotide expression levels corresponding to the gene; and

(3) select overlapping values from the basis sets generated in steps (1) and (2) as information which correlates to at least the atomic coordinates and conformation of one or more ligand-protein binding sites; and

(b) a high-throughput assay which is networked to the computer system for the transmission of information representative of validated ligand-protein binding sites determined by measuring the affinity of the ligand for the predicted ligand-protein binding sites.

In still another embodiment, the invention provides a network system for identifying protein binding sites for a novel ligand, the system comprising:

(a) a high-throughput assay which evaluates the binding of the novel ligand to one or more known protein binding sites, selects protein binding sites which bind the novel ligand within a specified selectivity and identifies information representing at least the atomic coordinates and conformation of protein signature pockets which bind the ligand within a specified selectivity;
(b) a computer system which is networked to the high-throughput assay for the receipt of information representing at least the atomic coordinates and conformation of protein signature pockets which bind the ligand within a specified selectivity, the computer system comprising a processor, a memory and a non-transitory computer-readable storage medium that stores instructions that, when executed by the processor, cause the system to:

(1) generate stochastic basis sets of values representative of putative ligand-protein binding sites by performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and identified protein signature pockets;

(2) generate stochastic basis sets of values representative of nucleotide expression levels of genes that have been correlated to known ligand-protein binding sites in a predetermined number of biological samples by mapping known ligand-protein binding site information to a gene name and identifying in a gene-biological sample expression database information representative of nucleotide expression levels corresponding to the gene; and

(3) select overlapping values from the basis sets generated in steps (b) and (c) as information which correlates to at least the atomic coordinates and conformation of one or more ligand-protein binding sites; and

(c) a high-throughput assay which is networked to the computer system for the transmission of information representative of ligand-protein binding site affinity and which validates the one or more ligand-protein binding sites

determined in step (b)(2) by measuring the affinity of the ligand for the one or more ligand-protein binding sites.

Beyond revealing biologically relevant interactions between small-molecules (ligands, cofactors or metabolites) and cellular proteins, methods and systems of the invention can establish metabolite-protein pairs for large-scale metabolic analyses, or predict possible targets for chemical small-molecule pollutants, such as bisphenols. Drug discovery applications identify possible lead compounds and novel druggable protein binding pockets, and provide insights into the binding mechanism of known drugs for which the drug:target complex structure has not been determined.

These and other aspects are described further in the Detailed Description of the Invention.

**Brief Description of the Figures**

**Figure 1:** The *PPE* of Formic Acid, β-D-glucose, and Phosphoaminophosphonic Acid-Adenylate Ester (Top view: a-c, Side view: d-f). Each position is represented by the label of the atom with the highest frequency. The atoms are gray scale coded as C: light gray; O: medium gray, N: dark gray.

**Figure 2(a):** Thermal shift assays on hPPARγ-LBD. Melting temperatures (Tm) calculated from thermal denaturation curves of hPPARγ-LBD in presence of varying molar excess of Rosiglitazone or Coenzyme A. Rosiglitazone displays a protective effect (raise of Tm) against thermal denaturation, in contrary of Coenzyme A which displays a destabilizing effect (decrease of Tm). **Figure 2(b):** The predicted CoA binding site overlaps with the ligand binding site on hPPARγ-LBD. The figure is based on the crystal structure of hPPARγ-LBD (light and medium grays) bound to rosiglitazone (darker gray rings; PDB id 4EMA). The predicted CoA binding pocket is a circular central zone.

**Figure 3:** Fluorescence anisotropy on hPPARγ-LBD. Dissociation constants (Kd) measured from fluorescence anisotropy titrations between fluoresceine-labelled PGC1-NR2 or N-CORNR2 (NCoR RID2) or S-CORNR2 (SMRT RID2) peptides and hPPARγ-LBD in the absence of ligand or in the presence of (A) 10 molar excess, and (B,C,D) increasing molar excess of Rosiglitazone, Coenzyme A or CD5477.

**Figure 4(a):** Change in thermal stability of Bcl-2 in the presence of CoA at various concentrations, the Bax-BH3 peptide (as positive control) and the scrambled LD4 peptide (as negative control) measured by change in the aggregation temperature of Bcl-2 in the presence of Ligands. **Figure 4(b):** Thermal stability of Bcl-2 in the presence and absence of CoA measured and the change in aggregation temperature $\Delta T_{agg}$ was plotted against the concentration of CoA. The $K_d$ value as determined using the single binding site model is 0.32 ± 0.13 mM. **Figure 4(c):** Comparison of Tryptophan Fluorescence quenching by various concentrations of CoA, Bax-BH3 peptide and Scramble LD4 peptide. 0.25 mM CoA were as effective in quenching tryptophan fluorescence as were 400 nM Bax-BH3 peptide. **Figure 4(d):** Tryptophan Relative Fluorescence of Bcl-2 in the presence of increasing concentrations of CoA. The $K_d$ calculated using a single binding site model was 0.38 ± 0.08 mM.

**Figure 5(a):** Increase in the anisotropy of the fluorescein labeled Bax-BH3 peptide is plotted against the Bcl-2 concentration. The Kd value was obtained by fitting the data to single binding site model and it was about 127.90 ± 21.02 nM. **Figure 5(b):** The predicted CoA binding site (dark gray scale) is adjacent to the Bax-BH3 (medium gray scale) binding site on Bcl-2 (light gray scale). The Bax-BH3 peptide binds in the largest pocket (darkest gray scale). The figure was built based on the crystal structure 2XA0.

**Figure 6(a):** Structural alignment of the *PPE* of 2'-Monophosphadenosine 5'-Diphosphoribose with Kinesin-like protein KIF11 (pdb id:2Q2Z, medium gray scale) and Collagenase 3-Inhibitor 24f complex (pdb id:3ELM, dark gray scale); **Figure 6(b)** Structural alignment of the *PPE* of 2'-Monophosphadenosine 5'-Diphosphoribose with Kinesin-like protein KIF11 (medium gray scale) and Collagenase 3-Pyrimidinedicarboxamide complex (pdb id:1XUC, dark gray scale); **Figure 6(c)** Structural alignment of the *PPE* of 2'-Monophosphadenosine 5'-Diphosphoribose with Kinesin-like protein KIF11 (medium gray scale) and C-C motif chemokine 4 (pdb id:1JE4, dark gray scale). This illustrates that the predicted novel targets spatially align with distinct parts of the *PPE.*

**Figure 7:** Fluorescence polarization of 20nM Bax-BH3 in the presence of 400nM Bcl-2 was titrated against increasing concentrations of CoA. The data show that CoA is unable to displace the Bax-BH3 from Bcl-2, as explained by non-overlapping CoA and Bax-BH3 binding sites on Bcl-2, as predicted by our model.

**Figure 8:** Validated targets and structures determined in accordance with methods of the invention.

**Figure 9:** Further validated targets and structures determined in accordance with methods of the invention.

**Figure 10(a):** aDDPs of 11 drugs investigated in this study. **Figure 10(b):** aDDP of CoA (1XVT) and the mRNA expression profile of four known CoA targets (ACAT2, HMGCR, KAT2B, CRAT), the Pearson correlation coefficient of aDDP of CoA with its known targets is 0.56. **Figure 10(c):** aDDP of b-D-glucose (1PIG) and the mRNA expression profile of five known b-D-glucose targets (ASPA, GNDPA, PYGM, NUDT9, PYGL). **Figure 10(d):** The mRNA expression profile of RGS 10 matches the aDDP of CoA in 65/79 tissues, while the mRNA expression profile of AMD1matches the aDDP of CoA in 46/79 tissues. In this case, RGS 10 will be preferredover AMD1 as the predicted target of CoA. Gray scale code: dark gray (low expression), light gray (medium expression) and medium gray (high expression). Y-axis has the 79 human tissues.

**Figure 11** is a flowchart showing steps in the iDTP method of the present invention.

**Detailed Description of the Invention**

The following terms shall be used throughout the specification to describe the present invention. Where a term is not specifically defined herein, that term shall be understood to be used in a manner consistent with its use by those of ordinary skill in the art.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention. In instances where a substituent is a possibility in one or more Markush groups, it is understood that only those substituents which form stable bonds are to be used.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

It must be noted that as used herein and in the appended claims, the singular forms "a," "and" and "the" include plural references unless the context clearly dictates otherwise.

Furthermore, the following terms shall have the definitions set out below.

The following abbreviations are used herein. iDTP: integrated structure and system-based approach for drug target prediction; PPE: probabilistic pocket ensemble; aDDP: approximated drug delivery profile; NR: nuclear receptor; PPAK$\gamma$: peroxisome proliferator-activated receptor gamma; Bcl-2: B-cell lymphoma 2; and CoA: coenzyme A.

The term "patient" or "subject" is used throughout the specification within context to describe an animal, generally a mammal, especially including a domesticated animal and preferably a human, to whom a treatment, including prophylactic treatment (prophylaxis) is provided. For treatment of those infections, conditions or disease states which are specific for a specific animal such as a human patient, the term patient refers to that specific animal. In most instances, the patient or subject is a human patient of either or both genders.

The term "effective" is used herein, unless otherwise indicated, to describe an amount of a compound or component which, when used within the context of its use, produces or effects an intended result, whether that result relates to the prophylaxis and/or therapy of an infection and/or disease state or as otherwise described herein. The term effective subsumes all other effective amount or effective concentration terms (including the term "therapeutically effective") which are otherwise described or used in the present application.

The term "compound" is used herein to describe any specific compound or bioactive agent disclosed herein, including any and all stereoisomers (including diasteromers), individual optical isomers (enantiomers) or racemic mixtures, pharmaceutically acceptable salts and prodrug forms. The term compound herein refers to stable compounds. Within its use in context, the term compound may refer to a single compound or a mixture of compounds as otherwise described herein.

In accordance with the present invention there may be employed conventional molecular biology, microbiology, and recombinant DNA techniques within the skill of the art. Such techniques are explained fully in the literature. *See, e.g.,* Sambrook et al, 2001, "Molecular Cloning: A Laboratory Manual"; Ausubel, ed., 1994, "Current Protocols in Molecular Biology" Volumes I-III; Celis, ed., 1994, "Cell Biology: A Laboratory Handbook" Volumes I-III; Coligan, ed., 1994, "Current Protocols in Immunology" Volumes I-III; Gait ed., 1984, "Oligonucleotide Synthesis"; Hames & Higgins eds., 1985, "Nucleic Acid Hybridization"; Hames & Higgins, eds., 1984,"Transcription And Translation"; Freshney, ed., 1986, "Animal Cell Culture"; IRL Press, 1986, "Immobilized Cells And Enzymes"; Perbal, 1984, "A Practical Guide To Molecular Cloning."

A "biological sample" can be a tissue sample or a cell sample.

"Basis set" is defined above and is further discussed hereinafter.

As used herein, the terms "nucleotide" and "polynucleotide" refer respectively to monomeric or polymeric form of nucleotides of any length, either ribonucleotides or deoxynucleotides, and include both double- and single-stranded DNA and RNA. A nucleotide or polynucleotide may include nucleotide sequences having different functions, such as coding regions, and non-coding regions such as regulatory sequences (e.g., promoters or transcriptional terminators). A polynucleotide can be obtained directly from a natural source, or can be prepared with the aid of recombinant, enzymatic, or chemical techniques. A nucleotide or polynucleotide can be linear or circular in topology. A nucleotide or polynucleotide can be, for example, a portion of a vector, such as an expression or cloning vector, or a fragment.

As used herein, the term "polypeptide" refers broadly to a polymer of two or more amino acids joined together by peptide bonds. The term "polypeptide" also includes molecules which contain more than one polypeptide joined by a disulfide bond, or complexes of polypeptides that are joined together, covalently or noncovalently, as multimers (e g., dimers, tetramers). Thus, the terms peptide, oligopeptide, and protein are all included within the definition of polypeptide and these terms are used interchangeably. It should be understood that these terms do not connote a specific length of a polymer of amino acids, nor are they intended to imply or distinguish whether the polypeptide is produced using recombinant techniques, chemical or enzymatic synthesis, or is naturally occurring.

A "ligand" can be any natural or synthetic moiety, including but not limited to a small molecule, an antibody, a nucleic acid, an amino acid, a protein (e.g. an enzyme) or a hormone that binds to a cell, preferably at a receptor (binding site) located on the surface of the cell. The term "ligand" therefore includes any targeting active species (compound or moiety, e.g. antigen) which binds to a moiety (preferably a receptor) on, in or associated with a cell. In some embodiments, a ligand is a peptide, a polypeptide including an antibody or antibody fragment, an aptamer, or a carbohydrate, among other species which bind to a targeted cell.

"Binding site" as used herein is not limited to receptor protein surface areas that interact directly with ligands, but also includes any atomic sequence, whether or not on the surface of a receptor, that is implicated (by affecting conformation or otherwise) in ligand binding. A purely illustrative list of binding sites that can be identified using methods and systems of the invention includes transmembrane receptors (including: G protein-coupled receptors (GPCRs; e.g. muscarinic acetylcholine receptor, adenosine receptors, adrenoreceptors, GABA receptors, angiotensin receptors, cannaboid receptors, cholecystokinin receptors, dopamine receptors, glucagons receptors, metabotropic glutamate receptors, histamine receptors, olfactory receptors, opiod receptors, rhodosin receptors, secretin receptors, serotonin receptors, somatostatin receptors, calcium-sensing receptor, chemokine receptors, sphingosine-1-phosphate (SIP) receptors); receptor tyrosine kinases (e.g. erythropoietin receptor, insulin receptor, insulin-like growth factor 1 receptor, Eph receptors); guanylyl cyclase receptors (e.g. receptors for natriuretic peptides, guanylin receptor); and ionotropic receptors (e.g. nicotinic acetylcholine receptor, glycine receptor, 5-HT, receptor, P2X receptors).

"Measuring the affinity of the ligand for the one or more ligand-protein binding sites" can be done using any number of techniques that are well-known to those of ordinary skill in the art, including but not limited to fluorescence detection techniques (described in greater detail hereinafter), NMR methods, X-ray crystallography, thermodynamic binding assays and whole cell-ligand binding assays. Preferably, fluorescence detection techniques are used. Conventionally, a receptor's (protein's) binding site is defined by areas of a protein tertiary structure that account for ligand-protein specificity and affinity, and that comprise atomic sequences that interact with the ligand through electrostatic interactions, hydrophobic interactions, hydrogen bonding or Van der Waals interactions. An affinity of the ligand for one or more ligand-protein binding sites represents a non-covalent attractive force between the protein and ligand.

As explained in U.S. Patent Application Document No. 20130280238, "association and dissociation reactions between a ligand and a binding site, and are characterized by the so-called 'dissociation constant' that reflects a degree of dissociation between the ligand and the binding site at equilibrium. Dissociation constants are typically expressed in units of concentration, with the lower concentrations reflecting higher affinity between the protein and the ligand, which can be also described as tighter binding. Another way to describe affinity is to use a degree of saturation of the binding site, or the total number of binding sites that are occupied by ligands per unit time. High affinity ligands reside in the binding site longer than lower affinity ligands.

Various methods exist for measuring and describing binding affinity between a protein and its ligand. For example, the dissociation constant can be determined based on the measurements of the amount of a complex formed over a range of starting concentrations of a ligand. In the biomedical field, measures of biological activity of a particular ligand are often used as substitute measures of binding affinity. For example, if a ligand inhibits a particular biological response, a 50% inhibitory concentration, or $IC_{50}$ may be used. For enzymes, the Michaelis constant, or $K_m$, is often used as a measure of a substrate's affinity for an enzyme's active site. It is to be understood that the methods of the present invention are not limited by any particular measure of binding affinity, and can employ a variety of measures of binding affinity. The selection of a particular measure of binding affinity depends on the specific application of the methods of the present invention, the type of protein and/or ligand, the experimental data available potentially available, and other factors."

Thus, in "selecting protein binding sites which bind the novel ligand within a specified selectivity", the specified selectivity can be any value consistent with the manner in which protein-ligand affinity is determined.

As explained in U.S. Patent Application Document No. 20140275487 "'conformation" or 'conformational state' of a protein refers generally to the range of structures that a protein may adopt at any instant in time. One of skill in the art will recognize that determinants of conformation or conformational state include a protein's primary structure as reflected in a protein's amino acid sequence (including modified amino acids) and the environment surrounding the protein. The conformation or conformational state of a protein also relates to structural features such as protein secondary structures (e.g., α-helix, β-sheet, among others), tertiary structure (e.g., the three dimensional folding of a polypeptide chain), and quaternary structure (e.g., interactions of a polypeptide chain with other protein subunits)."

"Atomic coordinates" represent a set of three-dimensional co-ordinates for atoms within a molecular structure as determined through X-ray crystallography or other techniques well-known to those of ordinarily skill in the art.

"Protein signature pockets" are determined through sequence-order independent surface alignments across the functional pockets of a family of protein structure as described in Dundas, et al., "Structural Signatures of Enzyme Binding Pockets from Order-Independent Surface Alignment: A Study of Metalloendopeptidase and NAD Binding Proteins", J Mol Biol. Mar 11, 2011; 406(5): 713-729 ("Dundas *et al.*") [29], and identify structurally preserved atoms across a family of protein structures that are functionally related. As described by Dundas *et al.,* "since more than one signature pocket may result for a single functional class, the signature pockets can be organized into a basis set of pockets for that functional family. These signature pockets of the binding surfaces then can be used for scanning a protein structure database for function inference."

Dundas, in "Protein Function Prediction for Omics Era" (D. Kihara ed.; Springer Science & Business Media, Apr 19, 2011), describes the determination of "protein signature pockets" in further detail in the paragraph below (citations, figure references and figures omitted). This excerpt also explains, in relevant context, the concept of "basis sets" and related use of "pair-wise sequence order-independent structure alignment", "hierarchical clustering", and (non-stochastic) "sequence similarity operations".

"A signature pocket is derived from an optimal alignment of precomputed surface pockets in a sequence-order-independent fashion, in which atoms and residues are aligned based on their spatial correspondence when maximal similarity is obtained, regardless how they are ordered in the underlying primary sequences. Our method does not require the atoms of the signature pocket to be present in all member structures. Instead, signature pockets can be created at varying degrees of partial structural similarity, and can be organized hierarchically at different level of binding surface similarity.

The input to the signature pocket algorithm is a set of functional pockets from a pre-calculated database of surface pockets and voids on proteins, such as those contained in the CASTp database. The algorithms begins by performing all vs all pair-wise sequence order independent structural alignment on the input functional surface pockets. A distance score, which is a function of the RMSD and the chemistry of the paired atoms from the structural alignment, is recorded for each aligned pair of functional pockets. The resulting distance matrix is then used by an agglomerative clustering method, which generates a hierarchical tree. The signature of the functional pockets can then be computed using a recursive process following the hierarchical tree. The process begins by finding the two closest siblings (pockets $S_A$ and $S_B$), and combining them into a single surface pocket structure $S_{AB}$. Because of the recursive nature of this algorithm, either of the two structures being combinedmay themselves already be a combination of several structures. When combining the two structures, we follow the criteria listed below:

1. If two atoms were considered equivalent in a structural alignment, a single coordinate is created in the new structure to represent both atoms. The new coordinate is calculated by averaging the coordinates of all underlying atoms that are currently represented by the two coordinates to be averaged.

2. If no equivalence was found for an atom during the structural alignment, the coordinates of that atom are transferred directly into the new pocket structure. During each step in combining two surface pockets, a count of the number of times that an atom at the position *i* was present in the underlying set of pockets is recorded, which is then divided by the number of the constituent pockets. This is the *preservation ratio* r (*i*). In addition, the mean distance of the coordinates of the aligned atoms to their geometric center is recorded as the *location variation v*. At the end of each step, the new structure $S_{AB}$ replaces the two structures $S_A$ and $S_B$ in the hierarchical tree, and the process is repeated on the updated hierarchical tree. At a specific height of the hierarchical tree, different signature pockets can be created with different extents of structural preservation by selecting a r threshold value. The signature pocket algorithm can be terminated at any point during its traversal of the hierarchical tree.

A single signature pocket that represents all surface pockets in the data set can be generated by raising the threshold even further. Since clusters from the hierarchical tree represent a set of surface pockets that are similar within certain threshold, if a stopping threshold is chosen such that there exist multiple clusters in the hierarchical tree, a

signature pocket will be created for each cluster. The set of signature pockets from different clusters collectively form a *basis set* of signature pockets, which represent the ensemble of differently sampled conformations for a functional family of proteins. As a basis set of signatures can represent many possible variations in shapes and chemical textures, it can represent structural features of an enzyme function with complex binding activities, and can also be used to accurately predict enzymes function."

As explained further in the Examples herein, "pair-wise sequence order-independent structure alignment, divisive hierarchical clustering, and probabilistic sequence similarity operations" steps of methods of the invention combine certain aspects of the aforementioned Dundas *et al.* data mining-optimization operations with novel methodologies that prove better suited to the *in silico* identification of novel ligand-protein binding sites. For example, *Dundas et al* requires high quality, manually curated enzyme binding sites and is not suitable for high throughput studies. We modified the method described by Dundas *et al.* and used minimal manually curated binding sites (one in most cases) to construct the probabilistic pocket ensemble for each drug. In order to extract the common structural features from the set of binding pockets, ideally a multiple structure alignment method is required. However, no such method currently exists that can handle our dataset. Therefore, following Dundas *et al.,* we have used the pairwise sequence order-independent structure alignment of surface pockets and hierarchical clustering to extract the common structural features [29].

Dundas *et al.* constructed several structural signatures corresponding to the different ligand/ligand binding site conformations at a predefined specific level of the hierarchical tree. In most cases, identifying this cut-off is not trivial and requires expert knowledge about the different conformations of the ligand/ligand binding site. In contrast, we constructed the structural signature at the root of the tree. As a result, the structural signature (or "probabilistic sequence similarity") is an ensemble of more than one unique pockets (corresponding to distinct branches in the hierarchical tree). Each position in the *PPE* has a preservation ratio (how often that particular atom was present in the underlying set of pockets [29]) of at least 0.5. To achieve a minimalistic ensemble and reduce the computational time, we increased the preservation ratio cut-off to 0.6 if the number of atoms in the *PPE* is greater than 110.

The step of "mapping known ligand-protein structures to a gene name" can be done using the UniProt Knowledgebase (UniProtKB) (including sub-parts Swiss-Prot and TrEMBL, UniParc, UniRef, and UniMes) (European Bioinformatics Institute consortium) along with the PubMed, UniProt, ChemAbstracts, PDB, InterPro and GenBank (www.ncbi.nlm.nih.gov) databases, and the protein domain databases Pfam, SMART, PROSITE, Propom, PRINTS, TIGRFAMs, PIR-SuperFamily or SUPERFAMILY can also be used.

One example of a useful "gene-sample expression database" is the database described in Su, A. et al. A gene atlas of the mouse and human protein-encoding transcriptomes. Proc Natl. Acad. Sci. USA 101, 6062-6067 (2004) [36]. The Human Protein Atlas, see www.proteinatlas.org, and Expression Atlas (http://www.ebi.ac.uk/gxa) are other examples of gene-tissue expression databases that can be used in methods of the invention. IMGT® (international ImMunoGeneTics information system® http://www.imgt.org) databases can also be used.

Additional useful gene-sample expression databases include (but are not limited to) the following listed or referenced in U.S. Patent Application Document No. 20110274692: "receptor tyrosine kinase receptors (Grassot et al., 2003, "RTKdb: database of receptor tyrosine kinase" Nucleic Acids Res 31:353-358), G protein-coupled receptors (Horn et al., 2003, "GPCRDB information system for G protein-coupled receptors" Nucleic Acids Res 31:294-297), olfactory receptors (Skoufos et al., 2000, "Olfactory receptor database: a sensory chemoreceptor resource" Nucleic Acids Res 28:341-343), thyrotropin receptor mutations (Fuhrer et al., 2003 "The thyrotropin receptor mutation database: update 2003" Thyroid 13:1123-1126), nuclear receptors (Patterson et al., 1994 "The androgen receptor gene mutations database" Nucleic Acids Res 22:3560-3562; Gottlieb et al., 1998, "The androgen receptor gene mutations database" Nucleic Acids Res 26:234-238), and endocrine disruptor receptors (Nakata et al., 1999, "Development of the receptor database (RDB): application to the endocrine disruptor problem" Bioinformatics 15:544-552) .... The Database of Ligand-Receptor Partners maintained by a group at University of California-Los Angeles (http://dip.doe-mbi.ucla.edu/dip/DLRP.cgi) contains subgroups of receptors for chemokines, TNF, fibroblast growth factor (FGF), and TGFB ligands; .... The Alliance for Cellular Signaling database contains extensive information on many signaling genes; each entry of the publication and database ...the reactome database (Joshi-Tope et al., 2005 "Reactome: a knowledgebase of biological pathways" Nucleic Acids Res 33:D428-D432) and the Human Protein Reference Database (Peri et al., 2003, "Development of human protein reference database as an initial platform for approaching systems biology in humans," Genome Res 13:2363-2371)."

The step of "identifying in a protein structure database information corresponding to known protein signature pockets, the information representing at least protein signature pocket atomic sequence and conformation" can be performed as in the above excerpt from Dundas, "Protein Function Prediction for Omics Era" (D. Kihara ed.; Springer Science & Business Media, Apr 19, 2011). The $CAST_p$ database ((http://cast.engr.uic.edu)) (Computer Atlas of Surface Topology of Proteins) is one example of such a database. $CAST_p$ can be combined with the POLYVIEW-3D server to better identify and define protein surface pockets and voids. Porollo, et al., "Versatile annotation and publication quality visualization of protein complexes using POLYVIEW-3D", BMC Bioinformatics, 2007; 8: 316. Other useful databases include but are not limited to SURFNET, LIGSITEcsc, ConCavity, APROPOS, DEPTH, fpocket and SiteMap.

"Fluorescence anisotropy" is used to characterize the extent of linear polarization of fluorescence emission, resulting from photoselection from an optically isotropic sample. "Fluorescence anisotropy measurements in solution: Methods and reference materials (IUPAC Technical Report)", Pure Appl. Chem., Vol. 85, No. 3, pp. 589-608, 2013. In addition to fluorescence anisotropy, any means of detecting a signal representative of a change in color, fluorescence, evanescence, surface plasmon resonance, electrical conductance or charge separation, ultraviolet, visible or infrared absorption, luminescence, chemiluminescence, electrochemiluminescence, fluorescence intensity, fluorescence lifetime, fluorescence polarization, fluorescence energy transfer, molecular mass, electron spin resonance, nuclear magnetic resonance, hydrodynamic volume or radius, specific gravity, scintillation, field effect resistance, electrical impedance, acoustic impedance, quantum evanescence, resonant scattering, fluorescent quenching, fluorescence correlation spectroscopy, acoustic load, acoustic shear wave velocity, binding force or interfacial stress can be used to measure the affinity of a ligand for a predicted ligand-protein binding site. *See* U.S. Patent Application Document No. 20140316116. Further, in fluorescence detection techniques, in addition to fluorescein, rhodamine, Texas red, cyanine dyes and nanogold particles coated with gold may be used.

Exemplary high-throughput assay systems include, but are not limited to, an Applied Biosystems plate-reader system (using a plate with any number of wells, including, but not limited to, a 96-well plate, a-384 well plate, a 768-well plate, a 1,536-well plate, a 3,456-well plate, a 6,144-well plate, and a plate with 30,000 or more wells), the ABI 7900 Micro Fluidic Card system (using a card with any number of wells, including, but not limited to, a 384-well card), other microfluidic systems that exploit the use of TaqMan probes (including, but not limited to, systems described in WO 04083443 A1, and published U.S. Patent Application Nos. 2003-0138829 A1 and 2003-0008308 A1), other micro card systems (including, but not limited to, WO04067175 A1, and published U.S. Patent Application Nos. 2004-083443 A1, 2004-0110275 A1, and 2004-0121364 A1), the Invader.RTM. system (Third Wave Technologies), the OpenArray.TM. system (Biotrove), systems including integrated fluidic circuits (Fluidigm), and other assay systems known in the art.

Computer systems that can be employed in implementing methods of the invention include a variety of known computer hardware systems and related software operating systems, including handheld calculators. Useful hardware systems include those with any type of suitable data processor, and linked personal computers using operating systems such as DOS®, Windows®, OS/2®, Linux®, Macintosh® and JAVA-OS® can be used. Sun Microsystems and Silicon Graphics operating UNIX® operating systems such as AIX® or SOLARIS® are also useful. Additional examples of computers include those which execute a control scheduler as a thin version of an operating system and any type of device which has a data processor associated with a memory.

Certain embodiments use computer-executable instructions implemented by a computer program that runs on a personal computer. Program modules including routines, programs, components, and data structures that perform particular tasks or implement particular abstract data types can also be used. Skilled artisans will recognize that the claimed methods and systems may use any number of computer system configurations, including hand-held devices, multiprocessor systems, microprocessor-based or programmable consumer electronics, minicomputers, mainframe computers, and the like. The invention may also be employed in distributed computing environments where tasks are performed by remote processing devices that are linked through a communications network. In a distributed computing environment, program modules may be located in both local and remote memory storage devices.

In certain embodiments, the claimed methods provide information over networks such as the Internet. For example, the components of the system may be interconnected via any suitable means including over a network. The processor may take the form of a portable processing device that may be carried by an individual user e.g. lap top, and data can be transmitted to or received from any device, such as for example, server, laptop, desktop, PDA, cell phone capable of receiving data, BLACKBERRY®, and the like. In some embodiments of the invention, the system and the processor may be integrated into a single unit. In another example, a wireless device can be used to receive information and forward it to another processor over a telecommunications network, for example, a text or multi-media message.

The functions of the processor need not be carried out on a single processing device. They may, instead be distributed among a plurality of processors, which may be interconnected over a network. Further, the information can be encoded using encryption methods, e.g. SSL, prior to transmitting over a network or remote user. The information required for decoding the captured encoded images taken from test objects may be stored in databases that are accessible to various users over the same or a different network.

In some embodiments, the data is saved to a data storage device and can be accessed through a web site. Authorized users can log onto the web site, upload scanned images, and immediately receive results on their browser. Results can also be stored in a database for future reviews.

In some embodiments, a web-based service may be implemented using standards for interface and data representation, such as SOAP® and XML®, to enable third parties to connect their information services and software to the data. This approach would enable seamless data request/response flow among diverse platforms and software applications.

One representative computer system includes networked mainframe or personal computers, including a processing unit, a system memory, and a system bus that couples various system components including the system

memory to the processing unit. The system bus may be any of several types of bus structure including a memory bus or memory controller, a peripheral bus, and a local bus using any of a variety of conventional bus architectures such as PCI, VESA, Microchannel, ISA and EISA. The system memory incorporates a read only memory (ROM) and random access memory (RAM) and the ROM stores a basic input/output system (BIOS) which contains the basic routines that helps to transfer information between elements within the personal computer.

"Non-transitory computer-readable storage media" can include a hard disk drive, a magnetic disc drive which reads from or writes to a removable disk, and an optical disk drive, e.g., for reading a CD-ROM disk or to read from or write to other optical media. The hard disk drive, magnetic disk drive, and optical disk drive may be connected to a system bus by a hard disk drive interface, a magnetic disk drive interface, and an optical drive interface. The drives and their associated computer-readable media provide non-transitory storage of data, data structure and computer-executable instructions. In addition to a hard disk, a removable magnetic disk and a CD, magnetic cassettes, flash memory card, digital video disks and Bernoulli cartridges may also be used.

Program modules stored in a RAM can encompass an operating system, an application program, an additional program module and program data. Commands may be entered through any number of known input devices, which in some embodiments are connected to the processing unit through a serial port interface that is coupled to the system bus, or through a parallel port, game port or a universal serial bus (USB). A monitor or display device may be connected to the system bus via an interface, and peripheral output devices can be used.

Computers may be networked using logical connections to one or more remote computers such as a server, a router, a peer device or other common network node. Logical connections include a local area network (LAN) and a wide area network (WAN). When a LAN networking environment is used, the computer can be connected to the local network through a network interface or adapter. When used in a WAN networking environment, the computer can include a modem or other means for establishing communications over the wide area network, such as the Internet. The modem, which may be internal or external, may be connected to the system bus via the serial port interface. In a networked environment, program modules depicted relative to the computer, or portions thereof, may be stored in the remote memory storage device.

In one example, methods and systems of the invention use an IBM compatible personal computer having at least eight megabytes of main memory and a gigabyte hard disk drive, with Microsoft Windows as the user interface and any variety of data base management software including Paradox. The application software implementing predictive functions can be written in any variety of languages, including but not limited to C++, and are stored on computer readable media as defined herein.

Preferably, the invention uses a data structure stored in a computer-readable medium, to be read by a microprocessor comprising at least one code that uniquely identifies formulae variables as disclosed herein. Computer-readable include nonvolatile, hard-coded type mediums such as read only memories (ROMs) or erasable, electrically programmable read only memories (EEPROMs), recordable type mediums such as floppy disks, hard disk drives and CD-ROMs, and transmission type media such as digital and analog communication links.

Data structures used in the invention include a collection of related data elements, together with a set of operations which reflect the relationships among the elements. A data structure can be considered to reflect the organization of data and its storage allocation within a device such as a computer. Data structures include an organization of information, usually in memory, for better algorithm efficiency, such as queue, stack, linked list, heap, dictionary, and tree, or conceptual unity and can include redundant information such as length of the list or number of nodes in a subtree. A data structure may be an external data structure or can be a passive data structure which is only changed by external threads or processes. An active or functional data structure has an associated thread or process that performs internal operations to give the external behavior of another, usually more general, data structure. A data structure also can be a persistent data structure that preserves its old versions, that is, previous versions may be queried in addition to the latest version. A data structure can be a recursive data structure that is partially composed of smaller or simpler instances of the same data structure. A data structure can also be an abstract data type, i.e., set of data values and associated operations that are precisely specified independent of any particular implementation.

In one embodiment, computer systems used in the invention include upwards of five hundred computers networked in parallel and programmed with Perl (e.g. Perl 5) or C (e.g. $C^{++}$).

Preferably, in order to compute the PPE or the structural signature for a drug, we use a high performance computer (high memory and CPU requirements); identification of the *PPE* for a drug with seventy known targets on scientific workstation can take around one week. To search a database again the PPE of a drug, a cluster of computers can be used. In some cases, it can take three or four days for 500 computers to search 75,000 protein structures against a particular PPE.

Computer systems of the invention can operate by either multithreaded parallelism in which the processor automatically generates multiple simultaneous instruction streams and multiple processors sharing a single memory execute these streams, or by distributing computing in which the processor runs multiple independent computations, or by explicit parallelism in which two or more processors with separate memories simultaneously execute instructions

stored on non-transitory computer-readable storage media.

These and other aspects of the invention are illustrated further in the following Examples.

### Example 1

### An integrated structure- and system-based framework for identification of new targets for metabolites and known drugs

### Constructing the Probabilistic Pocket Ensemble (PPE)

An inherently promiscuous drug may bind to different protein pockets with a range of features, making it difficult to establish a general description of the drug's possible binding sites. To capture the essential binding site features of a promiscuous drug, we developed a method to construct the *PPE* of this drug (see *Methods* section for details). The *PPE* represents a unified set of individual pockets that potentially bind to several conformations of the drug. Each position in the *PPE* can consist of a number of atoms from different residues. The frequency of the atoms and residues at each position is recorded and used to construct a maximum likelihood sequence similarity scoring function. This probabilistic scoring method adequately accounts for the fact that a drug can bind several pockets and a pocket can bind several drugs [28]. The *PPE*'s of formic acid, β-D-glucose, and phosphoaminophosphonic acid-adenylate ester are shown in Figure 1 where each position in the *PPE* is represented by the highest frequency atom at that position.

### Evaluation of the *PPE* and Probabilistic Scoring Function

To investigate the capacity of *PPE* to retrieve structurally similar drug binding pockets, we compared the protein pocket bound by 2'-monophosphadenosine 5'-diphosphoribose with the predicted binding pockets of the top 10 predicted targets of this compound using sequence-order independent alignment and sequence-order dependent alignment (see *Information Section* 1). Our results suggest that a combination of the minimalistic *PPE* and sequence order-independent alignment is more useful in identifying new drug targets than the combination of the complete binding pocket and sequence order-dependent structure alignment. Moreover, to determine the effectiveness of the probabilistic sequence similarity function, we compared the probabilistic function with the deterministic similarity function used by Dundas *et al.* [29] and found that the *PPE* is able to extract the non-trivial sequence and structure signatures necessary to capture the promiscuous process of the drug binding to multiple sites and the sites binding to multiple drugs (see Supplementary Information Section 1 below).

Most of the predicted novel targets spatially align with distinct parts of the *PPE*'s of the respective drugs (see Figures 6a-c), suggesting that the *PPE* is indeed an ensemble of several pockets and therefore might be able to accommodate the different conformers of each drug. These results suggest that multiple structural signatures might not be the optimal way to capture the different conformations of drugs in contrast to previous studies [29, 30] and that the problem can be effectively addressed by incorporating a probabilistic scoring function in the structural signatures as demonstrated in this study. Moreover, expert knowledge about the number of binding conformations and hence the number of structural signatures is not required in our methodology.

We next employed cross-validation to assess whether the *PPE* for each drug captures the essential features for the drug-protein interaction. We were able to predict the interaction of drugs with their known targets with an average sensitivity of 63% (Table 1). We have also constructed a negative dataset (a benchmark dataset is not available for such studies) to assess the specificity of the methodology. It is non-trivial to construct a negative dataset due to the inherent promiscuity of the drugs, their binding sites and an incomplete knowledge of targets for the drugs (see *Methods* section for details). We found that the average specificity of the method to be 81% (Table 1).

**Table 1. Dataset.** The drugs in our dataset along with their 2D structure, the PDB ID of the drug-protein complexes, the number of known targets with solved (apo) structures, the percentage of known targets predicted correctly in cross-validation (sensitivity), and the percentage of correctly predicted non-related proteins in the negative dataset (specificity).

| 2D structure | PDB (bound complex) | Drug Name | Known Targets | Sensitivity (%) | Specificity (%) |
|---|---|---|---|---|---|
| 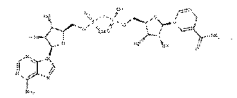 | 1OG3, 2BGL | Nicotinamide-Adenine-Dinucleotide | 129 | 74 | 89 |
| 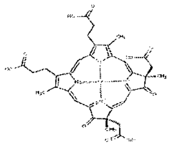 | 1GGE | Heme | 104 | 77 | 81 |
| 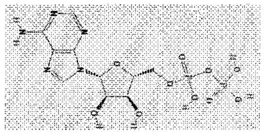 | 1UC9 | Adenosine-5'- Diphosphate | 101 | 63 | 68 |
| 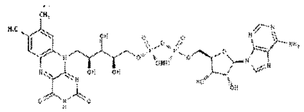 | 1FNB | Flavin-Adenine Dinucleotide | 73 | 64 | 83 |
| 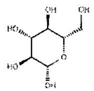 | 1PIG | β-D-Glucose | 67 | 66 | 81 |
| 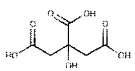 | 1HTO | Citric Acid | 62 | 65 | 62 |
| 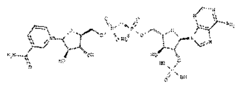 | 1DJL | 2' -Monophosphadenosine 5'-Diphosphoribose | 52 | 56 | 92 |
| 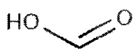 | 1B93 | Formic Acid | 47 | 70 | 84 |

(continued)

| 2D structure | PDB (bound complex) | Drug Name | Known Targets | Sensitivity (%) | Specificity (%) |
|---|---|---|---|---|---|
| 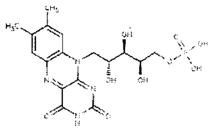 | 1SZF | Riboflavin Monophosphate | 47 | 53 | 70 |
| 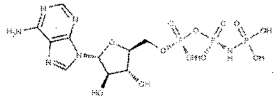 | 1TQM | Phosphoaminophosphonic Acid-Adenylate Ester | 42 | 45 | 86 |
| 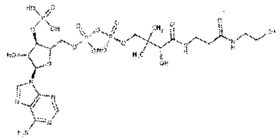 | 1XVT | Coenzyme A | 40 | 60 | 90 |
| | | **Average** | **69** | **63** | **81** |

### Integrating the Drug Delivery Profile to Reduce False Positives

To identify new drug targets with our method, we downloaded the complete CASTp database [31] that consists of 75,000 protein structures and their pockets. We extracted the three largest pockets from each of these protein structures as they account for more than 80% of the protein-small molecule binding sites [32-34]. We aligned the three largest pockets of each protein structure with the *PPE* (constructed using all the known drug targets) of each drug in our dataset using sequence order-independent structure alignment. This resulted in several thousand hits, a number similar to those of other drug repositioning studies [20]. Indeed, although our method has high sensitivity for the curated dataset, the false positive rate is expected to be higher in a general database search because our minimalistic template can align with several unrelated protein surfaces by random chance [29, 35].

To reduce the false positive rate of our method, we included an approximation for the drug delivery profile as orthogonal source of structure-independent information. Given that the actual drug tissue delivery profile is generally not available, we approximated this profile by averaging the mRNA expression profiles of all known drug targets in a set of 79 human tissues [36]. The rational was that the intracellular delivery profile of a given drug should be compatible with the mRNA expression profile of its established targets. mRNA expression profiles not only provide information about protein localization but can also provide information about protein:protein interactions and pathways [37]. Thus the comparison of the average tissue expression profile of known drug targets with the expression profiles of novel candidates is expected to reflect the likelihood for drug:target interactions in this set of tissues, and hence can be used as a proxy for the drug delivery profile (denoted herein *aDDP* for approximated Drug Delivery Profile).

We next used text mining to investigate the capacity of the *aDDP* to predict drug:target interactions. Co-citation Index [38] finds the association between two terms (in this case a drug and a gene name) by comparing the number of times the two terms appear in the abstract of studies deposited in PubMed as compared to two random terms. We found that when *aDDP* was combined with the *PPE,* the number of predictions with statistically significant co-citation index was 2-4 fold higher than using *aDDP* alone (see Supplementary Information Section 2 below). Indeed, for the combined approach the top 10 predicted targets (< 60% sequence similarity with any of the known drug targets) of β-D-glucose aligned extremely well with the *PPE* of β-D-glucose (SI Table 2). Similar results were observed for all the drugs in the dataset (see supporting information file PredTargets). Moreover, six out of the top 10 predictions have statistically significant co-citation index (p-value < 0.05). Similarly for Coenzyme A, three predictions have statistically significant co-citation index.

We found a total of 34 predicted targets with co-citation index values that had statistical significance (p-value < 0.05) for all the drugs in our dataset. For the top 10 predicted targets of all the drugs in the dataset, the range of the average sequence similarity score was between 80-87%, the range of the average RMSD was between 0.62-0.66Å, the range of the average match of mRNA expression profile was between 72-76 out of the 79 tissues, and the range of the average final score was between 0.45-0.56. These results showed that our methodology can identify novel proteins that have high structural (binding site) and system level similarity with known drug targets.

### Validation using *in vitro* binding experiments

To provide an experimental assessment of the performance of *iDTP,* we chose to test the predicted targets for coenzyme A (CoA), because this compound had the least number of known binding proteins in our dataset, and hence the least well defined *PPE* (Table 1). We used multiple *in vitro* binding experiments to test binding site and affinity for two top hits predicted by *iDTP,* namely the Peroxisome proliferator-activated receptor gamma (PPARγ) and B-cell lymphoma 2 (Bcl-2).

PPARγ is a nuclear hormone receptor regulating numerous biological functions including adipogenesis and cell differentiation. Its dysregulation is involved in the onset of diabetes and obesity [39]. The interaction of the ligand binding domain (LBD) of human PPAPγ (hPPARγ-LBD) with CoA is one of our most promising predictions, because this interaction achieved a high *iDTP* score and statistically significant co-citation index (*SI Table* 3). The pocket predicted by *iDTP* to bind CoA overlaps with the known ligand binding site of this receptor. To test this prediction *in vitro,* we first used differential scanning fluorimetry (DSF) to measure the melting temperature (*Tm*) of hPPARγ-LBD in absence or presence of CoA or rosiglitazone, an antidiabetic drug known to act as a ligand of hPPARγ-LBD (Figure 2(a) and SI Table 4). Seven molar excess of rosiglitazone displayed a protective effect, as it raised hPPARγ-LBD's *Tm* by 2°C compared to the apo-protein. On the contrary, the same amount of CoA displayed a destabilizing effect, lowering the *Tm* by 0.8°C compared to the apo-protein, suggesting a direct interaction with hPPARγ-LBD.

Next, we used fluorescence anisotropy (FA) to characterize the interaction between hPPARγ-LBD and its natural partner proteins upon CoA binding. We measured the dissociation constant ($K_d$) between hPPARγ-LBD and fluorescein-labeled peptides derived from a coactivator protein (PGC1) and two corepressor proteins (NCoR and SMRT). These experiments were performed in absence or presence of increasing molar excess of CoA or the reference hPPARγ-LBD agonist rosiglitazone or antagonist CD5477 [40]. If an increasing molar excess of the ligand has an increasing

influence on the $K_d$ of the fluorescently labeled coactivator/corepressor, then we can infer ligand binding, because the ligand disturbs binding to coactivators or corepressors. The nature of the ligand-hPPARy-LBD interaction can also be inferred: an agonist ligand would enhance binding to a coactivator, and decrease binding to a corepressor; an inverse agonist would do the contrary; a neutral antagonist would decrease binding for both coactivators and corepressors. Accordingly, adding two to ten molar excess of the agonist rosiglitazone hPPARy-LBD raised the affinity of hPPARy-LBD for the coactivator PGC1 (Figure 3(a) and (b), SI Table 5). Conversely, a two molar excess of the antagonist CD5577 lowered the affinity of hPPARy-LBD for the coactivator PGC1 (Figure 3(a) and (b), SI Table 5). Addition of two to ten molar excess of CoA lowered the affinity of hPPARy-LBD for both coactivator PGC1 (Figure 2(b)) and corepressors NCoR and SMRT (Figure 3(c) and (d), SI Table 5). Collectively, our experiments confirm a direct interaction between CoA and hPPARy-LBD in which CoA behaves as a neutral antagonist. From its potency in competing with the known ligands, and from the dose-dependent stabilization of hPPARy-LBD, we estimate the $K_d$ to be less than 500 μM.

We also tested direct binding of CoA to recombinant Bcl-2 (see supporting information file PredTargets). Bcl-2, the founding member of the Bcl-2 family of regulator proteins that regulate cell death, is an important anti-apoptotic protein and is classified as an oncogene. Using differential static light scattering (DSLS) we observed that the aggregation temperature $T_{agg}$ for 0.5 mg/ml apo-Bcl-2 was ~57°C. 400 nM of the known ligand Bax-BH3 significantly increased the $T_{agg}$ to 67°C, whereas the presence of 1 μM of scrambled LD4 peptide (as negative control) did not alter $T_{agg}$. Increasing concentrations of CoA increased $T_{agg}$ for Bcl-2 up to 62°C, suggesting a direct interaction (Figure 4(a) and (b), SI Table 6). We then established the $K_d$ of the CoA:Bcl-2 interaction to be 0.38 mM by measuring the quenching of intrinsic tryptophan fluorescence of Bcl-2 in presence of increasing CoA concentrations (Figures 4(c) and(d)). In comparison, the Bax-BH3 peptide, but not scrambled LD4 peptide, was much more potent in quenching the fluorescence of Bcl-2 (Figure 4(d)). Indeed, the affinity of Bcl-2 for a fluorescence-labeled Bax-BH3 peptide was 128 ± 21 nM under the conditions used, using FA (Figure 5(a)). The predicted CoA binding pocket by *iDTP* was adjacent to the Bax-BH binding site and without significant overlap (Figure 5(b)). The presence of CoA was therefore not expected to displace bound Bax-BH3. Indeed, even 4.7 mM CoA did not reduce the FA of Bax-BH3 (at a concentration of 20 nM, 235,000 times less than CoA), in presence of 400 nM Bcl-2, suggesting that both ligands are occupying non-overlapping binding sites (Figure 7). Thus, our *in vitro* binding experiments strongly supported our computational binding predictions.

**Discussion**

In this study, we have introduced a computational method to extract implicit structural signatures from drug binding sites of proteins to construct a *PPE.* We demonstrated that the *PPE* can reliably identify the known targets of several drugs. The *PPE* was combined with an *aDDP* as orthogonal source of structure-independent information. The resulting method, *iDTP,* enables large-scale prediction of novel targets for these drugs. We showed that the *PPE* can be constructed using as few as one structure of a drug:protein complex and a set of apo structures of other known drug-binding proteins. The construction of structural signatures for drug binding sites using sequence order-independent alignment, and the use of a probabilistic scoring function to model the drug-protein interaction allows weakly conserved but significant patterns to emerge and to be quantified.

The predictive power of our integrated method was supported by computational cross-validation and text mining. Moreover, we validated two of our predicted interactions by *in vitro* experiments. Firstly we showed that CoA indeed bound to hPPARy-LBD, with an apparent $K_d$ of less than 500 μM. The CoA binding site on hPPARy-LBD is predicted to be the receptor's ligand binding pocket, which also binds rosiglitazone and CD5477. Indeed, our observation that CoA showed characteristics of a neutral antagonist strongly suggested that CoA binds to the ligand binding pocket, as predicted. Thus CoA could trigger a conformational change that disrupts or unsettles the binding surface of coactivators/corepressors, explaining the drug's neutral antagonist nature. Of course our current data cannot strictly rule out that CoA binds to the surface where coactivators and corepressors normally bind, creating a competition for the binding site.

However, the ligand-binding pocket of hPPARy is one of the largest among the nuclear receptor protein family [41], allowing hPPARy to bind a variety of ligands, further supporting the predicted mode of action. CoA is a ubiquitous cofactor, and can reach high concentrations in eukaryotes, depending on cell type and subcellular localization (~0.14, 0.7 and 5 mM in animal cytosol, peroxysomes and mitochondria, respectively [42]). It is therefore possible that this predicted interaction plays a currently unrecognized biological role in fatty acid signaling and metabolism.

Secondly, we showed that CoA also bound *in vitro* to recombinant Bcl-2, as predicted, with a $K_d$ of about 350 μM. The predicted CoA binding pocket on Bcl-2 is adjacent to the known binding site for Bax-BH3. Indeed, we could show that CoA binds Bcl-2 without displacing Bax-BH3, inferring non-competitive binding. The unique predicted binding pocket of CoA is interesting for drug design purposes, because it is located adjacent to the well-explored Bax-BH3 binding pocket [43], and hence may provide an alternative target site with possible synergetic effects.

Beyond validating our computational predictions, our *in vitro* experiments also suggested the usefulness of *iDTP* for various applications: The case of the CoA:hPPARγ illustrates how *iDTP* might be used to reveal biologically relevant interactions between small-molecules (ligands, cofactors or metabolites) and cellular proteins. Thus, our method

could help establish metabolite-protein pairs for large-scale metabolic analyses, or predicting possible targets for chemical small-molecule pollutants, such as bisphenols. The case of CoA:Bcl-2 might illustrate *iDTP*'s use for drug discovery by suggesting possible lead compounds and novel druggable protein binding pockets. *iDTP* might also provide insights into the binding mechanism of known drugs for which the drug:target complex structure has not been determined.

For example our results suggested (supporting information file PredTargets) that formic acid binds to CYP2E1 (sequence similarity of 81%, RMSD of 0.65°A with the *PPE* of formic acid, mRNA expression match in 75 of 79 tissues, and an overall score of 0.53). CYP2E1 is an enzyme that is known to interact with more than 70 small drugs and xenobiotic compounds [44]. Induction of CYP2E1 has been shown to cause oxidative stress and alcohol induced liver injury in mouse models [45, 46]. Trolox[6-hydroxy,2,5,7,8-tetramethylchroman-2-carboxylic acid], a drug that contains the formic acid structure, has been shown to reduce the above mentioned toxicity [47, 48]. Hence our results suggest a direct interaction between the formic acid moiety of Trolox and CYP2E1 resulting in the reduction of toxicity.

To further evaluate the usefulness of *iDTP* for pharmaceutical purposes we identified the genetic diseases associated with the predicted target proteins for each drug using the OMIM [49] and HGMD [50] databases. For seventeen predicted drug:target pairs (including CoA:hPPAR$\gamma$), a co-citation index with high statistical significance (p-value < 0.005) was found between these molecules and the predicted targets were associated with major human diseases, such as cancer, heart and metabolic dysfunctions (SI Table 3), suggesting opportunities for using these results as basis for drug discovery and drug repositioning. However, the use of *iDTP* for drug repositioning in the strict sense is currently still limited because of the requirement of a relatively large set of 3D structures of known targets to construct a high-confidence *PPE.*

The challenge of identifying new drug-target pairs *in silico* has attracted significant interests from the computational community. To our knowledge, none of the previous studies has utilized the combination of sequence order-independent alignment and probabilistic scoring function to model the drug-protein interaction or has employed the approximated drug delivery profile to filter out the false positive predictions. Most of the previous studies do not assess the performance of their methodologies by predicting the known targets of the drugs and use significantly different datasets and definitions for success rate. Therefore, a direct comparison among the methodologies is not possible. However, it is possible to comment on their methodological advantages and limitations.

Existing methods share one or more of the following limitations. (i) They are known to scale poorly with the size and complexity of the drug and drug binding sites [51]. (ii) These methods do not appropriately account for the different conformations of both the drug and the binding site residues. (iii) They are hampered by the limited availability of the molecular activity profiles in different tissues before and/or after drug treatment. Our method eliminates most of these weaknesses by constructing a structural signature from a set of binding sites instead of a single binding site and employing a probabilistic sequence similarity function, therefore accounting for the different conformations of the drug and the binding site residues (this is analogous to the improvement expected from a multiple sequence alignment as compared to a pairwise alignment). Our method also incorporates the approximated drug delivery profile to identify relevant new targets.

Our method might lead to repositioning existing drugs to treat new diseases, thus expediting the drug discovery process as well as significantly lowering its cost. We also expect that our method will help better understand the side effects and toxicity caused by current drugs due to its ability to identify off-target binding sites. The off-target binding can be mapped to the side-effects observed for each drug using existing graphical approaches [6]. A more comprehensive understanding of the side-effects will help us in identifying safe drug combinations.

## Materials and Methods

### Dataset

We extracted the approved and experimental drugs from the Drugbank database version 3 [52]. For this study, we used all drugs for which at least one 3D structure of a drug:protein complex and more than 40 known drug targets with solved apo structures were available (Table 1). However in principle 30 apo structures and one complex structure should be enough. We expect that our method can be used on much larger set of drugs whose information is not available in public databases.

Protein structures on average have more than 30 pockets (some structures have > 100 pockets) and a majority of the protein-small molecule interactions occur in the three largest pockets [32-34]. A typical pocket involved in protein-small molecule interactions (also known as a druggable pocket) has characteristic values for pocket solvent accessible surface area (300-600 Å$^2$)[28] and pocket volume (400-600 Å$^3$)[53]. We hypothesize that a protein structure that has a minimal number of non-druggable pockets and no druggable pocket is unlikely to interact with a drug. In this study, we extracted the protein structures that had less than three pockets from the CASTp database to form the negative dataset. After redundancy reduction using the PISCES webserver [54] (< 60% pairwise sequence identity), removing the NMR structures, structures with co-crystallized DNA, RNA or ligands (excluding ions like $Zn^{2+}$, $Cl^-$), and structures with druggable pockets, we extracted the surface residues (> 70% of solvent accessible surface area) of the remaining protein structures using the POPS method [55]. These surface residues are aligned with the *PPE's* of all the drugs in the dataset to measure their specificity. Our negative dataset contained a total of 63 protein structures (detailed list of PDB ids can be found supporting information file NegDataset).

**Constructing stochastic pocket ensemble**

After identifying the drug:target complex structure for each drug in the dataset, we extracted the pocket that the drug binds to in the protein structure using the CASTp webserver [31]. We refer to this as the 'bound pocket'. In order to identify the binding site of the drug in the apo structures of known drug targets, we extracted the three largest pockets from the first chain of their respective 3D structures. We chose the pocket that is most similar to the 'bound pocket' by using sequence order-independent alignment.

Dundas *et al.* have established a method to construct the structural signatures for enzyme binding pockets [29]. Their method requires high quality, manually curated enzyme binding sites and is not suitable for high throughput studies. We modified the method described by Dundas *et al.* and used minimal manually curated binding sites (one in most cases) to construct the probabilistic pocket ensemble for each drug. In order to extract the common structural features from the set of binding pockets, ideally a multiple structure alignment method is required. However, no such method currently exists that can handle our dataset.

Therefore, following Dundas *et al.,* we have used the pairwise sequence order-independent structure alignment of surface pockets and hierarchical clustering to extract the common structural features [29]. However, Dundas *et al.* constructed several structural signatures corresponding to the different ligand/ligand binding site conformations at a predefined specific level of the hierarchical tree. In most cases, identifying this cut-off is not trivial and requires expert knowledge about the different conformations of the ligand/ligand binding site. In contrast, we constructed the structural signature at the root of the tree. As a result, the structural signature is an ensemble of more than one unique pockets (corresponding to distinct branches in the hierarchical tree). Each position in the *PPE* has a preservation ratio (how often that particular atom was present in the underlying set of pockets [29]) of at least 0.5. To achieve a minimalistic ensemble and reduce the computational time, we increased the preservation ratio cut-off to 0.6 if the number of atoms in the *PPE* is greater than 110.

For the current implementation of *iDTP,* about 30 apo-structures per drug are used to calculate a high-confidence *PPE.* However, on one hand, future methodological improvements are expected to reduce this number substantially. For example currently establishing the *PPE* involves deriving a tree from pairwise similarity between pockets. Using a widely accepted approach, this tree could be replaced by a phylogenetic tree, which should markedly reduce the number of required apo structures [29]. On the other hand, more and more drugs are expected to meet the requirements for *iDTP,* given the increasing 3D structural and binding data produced from the scientific community, boosted by an increasing number of pharmaceutical companies that make their data openly accessible (http://www.forbes.com/sites/danmunro/2014/04/08/big-pharma-opens-new-chapter-on-big-data-collaboration/). For specific drugs, a stricter conservation ratio for the atoms essential for the binding/action of the respective drugs can be readily incorporated in our method.

**Calculating distance to the probabilistic pocket ensemble**

Each position in the structural signature may be occupied by more than one type of atoms (residues). Therefore, we formulated a probabilistic distance function to accommodate this property. The distance function has a structural and sequence component. The structural component follows Dundas *et al.*'s approach, while the sequence component is based on maximum likelihood.

$$Score = Structural\ similarity + \alpha * (1 - Sequence\ similarity)$$

$$Structural\ similarity = \frac{RMSD}{N^{1/3}}$$

$$Sequence\ similarity = \frac{Sequence\ score}{Best\ sequence\ score}$$

$$Sequence\ score = \sum_i (AtomFreq_i + ResFreq_i)$$

$$Best\ sequence\ score = \sum_i (MaxAtomFreq_i + MaxResFreq_i)$$

where the value for $\alpha$ is set to 1.2 following Dundas *et al.* [29], *RMSD* is the root mean square distance after the alignment, *N* is the number of positions aligned, $AtomFreq_i$ / $ResFreq_i$ is the frequency of aligned atom/residue at position *i*, $MaxAtomFreq_i$ / $MaxResFreq_i$ is the highest frequency of any atom/residue at position *i* and the summation is over all the aligned positions. For example, if a position in the structural signature consisting of *C,C,C,O,N* atoms and *A,A,Y,Y,Y* residues was aligned to an atom *C* and a residue *A*, the *Sequence Score* will be 1 (0.6 + 0.4=1) and the *Best Sequence Score* will be 1.2 (0.6 + 0.6=1.2), resulting in a sequence similarity of 0.83 (1/1.2). An empirical distance cut-off of 0.85 that maps to an RMSD of 0.7 Å and *Sequence Similarity* of 60% for a sequence order-independent alignment of 12-15 atoms is used in this study. An alignment should also contain at least five atoms.

**Cross-validation of the *PPE***

We employed cross-validation (5-fold for drugs that have less than 100 known targets and 3-fold for the drugs with more than 100 targets) to assess whether the *PPE* for each drug captures the essential features for the drug-protein interaction. The known targets of each drug were randomly divided into five (or three) equal sets. Four (or two) sets were used to make the *PPE* and predictions were provided for the fifth (or third) set. We repeated this procedure five (or three) times for each drug.

**Integrating the approximate drug delivery profile (*aDDP*)**

The *aDDP* for each drug is approximated by averaging the mRNA expression of known drug targets in a set of 79 human tissues from Su *et al.* [36]. We mapped each drug:target structure to a gene name by Uniprot ID mapping service [56]. This gene was then searched in the tissue expression dataset compiled by Su *et al.* [36]. In case a protein structure mapped to more than one gene, the average expression of all the mapped genes was used. We classified the expression in 79 human tissues into three classes (low, medium, and high) based on empirical cut-offs for the mRNA expression (< 300, < 1000, ≥ 1000). The new drug target list is reordered by including drug target tissue expression term in the distance function as follows:

$$Score = Structural\ similarity + \alpha * (1 - Sequence\ similarity) + \beta * Tissue\ expression$$

$$Tissue\ expression = 1 - \frac{Number\ of\ tissues\ with\ matching\ expression}{Total\ number\ of\ tissues}$$

where $\beta$ is empirically set to 0.4. If a gene is not present in the dataset compiled by Su *et al.,* we set $\beta$ * tissue expression to 0.2. Our method is not sensitive to the specific value of $\beta$ as the top 10 predicted targets have an almost perfect match between their mRNA expression profile and that of the estimated drug delivery profile.

**Protein expression and purification**

Expression and purification of the ligand binding domain (LBD) of human PPAR$\gamma$ (amino acids Glu196-Tyr477) have been described previously [57]. The sequence, production and purification of the chimeric human Bcl-2 (with the internal loop 51-91 removed and residues 35-50 replaced by residues 33-48 of mouse Bcl-$X_L$) were as described in [43].

**Ligands and peptides**

BRL49653 (Rosiglitazone) and Coenzyme A for hPPARy binding assays were purchased from Sigma-Aldrich (St Quentin Fallavier, France). The fluorescence-labeled peptides FITC-EEPSLLKKLLLAPA, FITC-DPASNLGLEDIIRKALMGSFD, and FITC-TNMGLEAIIRKALMGKYDQWEE, corresponding to the PGC1-NR2, NCoR-RID2 and SMRT-RID2 respectively were purchased from EZbiolab (Westfield, Indiana, USA). For the Bcl-2 interaction studies, the fluorescence-labeled peptide QDASTKKLSE CLRRIGDELDSNMELQRMIAD corresponding to Bax-BH3 (a known ligand for Bcl-2), and the scrambled LD4 peptide (LSDAMETSSLRDALE, a scrambled version of the Bcl-2 ligand LD4 [58]) were purchased from Genscript USA inc. Coenzyme A (CoA) was bought from Calbiochem (VWR, UK).

**Differential scanning fluorimetry (DSF; Thermofluor®)**

This method measures the protein unfolding based on fluorescence detection of the denatured form of the protein [59]. Solutions of 15 μL containing 5 μM hPPARy LBD, varying molar excess of ligands (Rosiglitazone, Coenzyme A, or CD5477), and IX Sypro® Orange in 50 mM Tris pH 8.0, 200 mM NaCl, were added to the wells of a 96-well PCR plate. Final DMSO concentration did not exceed 5% and had no influence on the data. The plates were sealed with an optical sealing tape (Bio-Rad) and heated in an Mx3005P Q-PCR system (Stratagene) from 25 to 95°C at 1°C intervals. Fluorescence changes in the wells were monitored with a photomultiplier tube. The wavelengths for excitation and emission were 545 nm and 568 nm, respectively. The melting temperatures, Tm, were obtained by fitting the fluorescence data with a Boltzmann model using the GraphPad Prism software. The reported data are the average of independent experiments and error bars correspond to standard deviations.

**Differential static light scattering (DSLS)**

DSLS measured by the Stargazer system (Harbinger Biotechnology and Engineering Corporation, Markham, Canada) was used to assess the thermal stability of Bcl-2 in the absence or presence of CoA and control ligands. DSLS measures the specific aggregation temperature, $T_{agg}$ at which a protein aggregates as a result of heat denaturing. Thus, DSLS provides the thermal stability of proteins, which is expected to vary in presence of ligands. Bcl-2 at 0.5 mg/mL was overlaid with mineral oil in a clear bottom 384-well black plate (Corning) were heated from 20 to 85 °C at 1 °C/min and the light scattering was detected by a CCD camera every 0.5 °C in the absence or presence of varying concentrations of CoA, yielding the difference in aggregation temperature at a particular CoA concentration from apo Bcl-2$\Delta T_{agg}$. Data were normalized and $K_d$ values were calculated by plotting $\Delta T_{agg}$ against the CoA concentration. Resulting data were fitted using a binding saturation single site model (GraphPad).

**Fluorescence anisotropy measurements**

Binding affinities of the fluorescent peptides for hPPARy LBD were measured in the presence or absence of ligands using a Safire2 microplate reader (TECAN). For the fluorescence-labeled peptides, excitation wavelength was set at 470 nm and emission measured at 530 nm. The reported data are the average of independent experiments and error bars correspond to standard deviations. The buffer solution for assays was 20 mM Tris-HCl pH 7.5, 150 mM NaCl, 1 mM EDTA, 5 mM dithiothreitol, and 10% (v/v) glycerol. The measurements were initiated at 40 μM of the protein, and then the sample was diluted successively by a factor of 2 with the buffer until the lowest protein concentration reached 9.7 nM. Fluorescent peptides were added to the protein samples at 4 nM, allowing establishment of the titration curve. Ligands, when added, were at final concentration of 80 μM. The binding affinity of the fluorescently labeled Bax-BH3 peptide towards Bcl-2 was determined using a fluorescence spectrometer from Photon Technologies International, USA. The excitation wavelength of fluorescence-labeled peptide was 490 nm and emission was measured at 520 nm. The $K_d$ value for the peptide was fitted using a single binding site model. Competition experiments were performed using the 20nM Bax-BH3 peptide and 400 nM Bcl-2. Fluorescence anisotropy was monitored for concentrations up to 5 mM of CoA.

**Intrinsic tryptophan fluorescence quenching binding assay**

Bcl-2 tryptophans were excited at 280 nM and the emission intensity was measured at 320 nM. 10 μM Bcl-2 was incubated with various dilutions of CoA for 10 min before measurement. The fluorescence emitted was monitored using a PheraStar fluorescence plate reader in 96-well plates. Change in tryptophan fluorescence occurs due to conformational change in the protein, when it is bound to the ligand and the difference in the fluorescence intensity was recorded and analyzed. Data were normalized, and $K_d$ values were calculated by fitting to a Binding-Saturation single site model with GraphPad.

**Acknowledgments**

This research was funded by the King Abdullah University of Science and Technology (KAUST).

**References for Background and Detailed Description of the Invention and Example 1**

1. Reddy, A. & Zhang, S. Polypharmacology: drug discovery for the future. Expert Rev. Clin. Pharmacol. 6, 41-47 (2013).

2. Arrowsmith, J. Trial watch: phase III and submission failures: 2007-2010. Nat. Rev. Drug Discov. 10, 87 (2011).

3. Arrowsmith, J. Trial watch: Phase II failures: 2008-2010. Nat. Rev. Drug Discov. 10, 328-329 (2011).

4. Liebler, D. & Guengerich, F. Elucidating mechanisms of drug-induced toxicity. Nat. Rev. Drug Discov. 4, 410-420 (2005).

5. Mestres, J., Gregori-Puigjane, E., Valverde, S. & Sole R. The topology of drug-target interaction networks: implicit dependence on drug properties and target families. Mol. Biosyst. 5, 1051-1057 (2009).

6. Lounkine, E. et al. Large-scale prediction and testing of drug activity on side-effect targets. Nature 486, 361-367 (2012).

7. Peters, J. Polypharmacology - foe or friend? J. Med. Chem. 56, 8955-8971 (2013).

8. Ashburn, T. & Thor, K. Drug repositioning: identifying and developing new uses for existing drugs. Nat. Rev. Drug Discov. 3, 673-683 (2004).

9. Kinnings, S. et al. Drug discovery using chemical systems biology: repositioning the safe medicine comtan to treat multi-drug and extensively drug resistant tuberculosis. PLoS Comput. Biol. 5, e1000423 (2009).

10. Engin, H., Keskin, O., Nussinov, R. & Gursoy, A. A strategy based on protein-protein interface motifs may help in identifying drug off-targets. J. Chem. Inf. Model. 52, 2273-2286 (2012).

11. Chang, R., Xie, L., Xie, L., Bourne, P. & Palsson, B. Drug off-target effects predicted using structural analysis in the context of a metabolic network model. PLoS Comput. Biol. 6, e1000938 (2010).

12. Li, Y.Y., An, J. & Jones, S.J.M. A computational approach to finding novel targets for existing drugs. PLoS Comput. Biol. 7(9), e1002139 (2011).

13. Iorio, F. et al. Discovery of drug mode of action and drug repositioning from transcriptional responses. Proc. Natl. Acad. Sci USA 107,14621-14626 (2010).

14. Wei, G. et al. Gene expression-based chemical genomics identifies rapamycin as a modulator of MCL1 and glucocorticoid resistance. Cancer Cell 10, 331-342 (2006).

15. Chen, B., Wild, D. & Guha, R. Pubchem as a source of polypharmacology. J. Chem. Inf. Model. 49, 2044-2055 (2009).

16. Hu, G. & Agarwal, P. Human disease-drug network based on genomic expression profiles. PLoS One 4, e6536 (2009).

17. Suthram, S. et al. Network-based elucidation of human disease similarities reveals common functional modules enriched for pluripotent drug targets. PLoS Comput. Biol. 6, e1000662 (2010).

18. Emig, D. et al. Drug target prediction and repositioning using an integrated network-based approach. PLoS One 8(4), e60618 (2013).

19. Lamb, J. et al. The connectivity map: using gene-expression signatures to connect small molecules, genes, and disease. Science 313, 1929-1935 (2006).

20. Keiser, M. et al. Predicting new molecular targets for known drugs. Nature 462, 175-181 (2009).

21. Noeske, T. et al. Predicting compound selectivity by self-organizing maps: cross-activities of metabotropic glutamate receptor antagonists. ChemMedChem 1, 1066-1068 (2006).

22. Qu, X., Gudivada, R., Jegga, A., Neumann, E. & Aronow, B. Inferring novel disease indications for known drugs by semantically linking drug action and disease mechanism relationships. BMC Bioinformatics 10, Suppl 5: S4 (2009).

23. Campillos, M., Kuhn, M., Gavin, A., Jensen, L. & Bork, P. Drug target identification using side-effect similarity. Science 321, 263-266 (2008).

24. Sanseau, P. et al. Use of genome-wide association studies for drug repositioning. Nat. Biotechnol. 30, 317-320 (2012).

25. Wang, Z. & Zhang, H. Rational drug repositioning by medical genetics. Nat. Biotechnol. 31, 1080-1082 (2013).

26. Gottlieb, A., Stein, G.Y., Ruppin, E. & Sharan, R. PREDICT: a method for inferring novel drug indications with application to personalized medicine. Mol. Sys. Biol. 7, 496 (2011).

27. Napolitano, F. et al. Drug repositioning: a machine-learning approach through data integration. J. Cheminform. 5, 30 (2013).

28. Gao, M. & Skolnick, J. A comprehensive survey of small-molecule binding pockets in proteins. PLoS Comput. Biol. 9, e1003302 (2013).

29. Dundas, J., Adamian, L. & Liang, J. Structural signatures of enzyme binding pockets from order-independent surface alignment: a study of metalloendopeptidase and NAD binding proteins. J. Mol. Biol. 406, 713-729 (2011).

30. Tseng, Y. & Liang, J. Estimation of amino acid residue substitution rates at local spatial regions and application in protein function inference: a bayesian monte carlo approach. Mol. Biol. Evol. 23, 421-436 (2006).

31. Dundas, J. et al. CASTp: computed atlas of surface topography of proteins with structural and topographicalmapping of functionally annotated residues. Nucleic Acids Res. 34, W 116-8 (2006).

32. Huang, B.D. & Schroeder, M. LIGSITEcsc: predicting ligand binding sites using the Connolly surface and degree of conservation. BMC Struct. Biol. 6, 19 (2006).

33. Brady, G. Jr & Stouten, P. Fast prediction and visualization of protein binding pockets with PASS. J. Comput. Aided Mol. Des. 14, 383-401 (2000).

34. Peters, K., Fauck, J. & Frommel, C. The automatic search for ligand binding sites in proteins of known three-dimensional structure using only geometric criteria. J. Mol. Biol. 256, 201-213 (1996).

35. Watson, J., Laskowski, R. & Thornton, J. Predicting protein function from sequence and structural data. Curr. Opin. Struct. Biol. 15, 275-284 (2005).

36. Su, A. et al. A gene atlas of the mouse and human protein-encoding transcriptomes. Proc Natl. Acad. Sci. USA 101, 6062-6067 (2004).

37. Jansen, R., Greenbaum, D. & Gerstein, M. Relating whole-genome expression data with protein-protein interactions. Genome Res. 12, 37-46 (2002).

38. Qiao, N., Huang, Y., Naveed, H., Green, C., Han, J. Cociter: an efficient tool to infer gene function by assessing the significance of literature co-citation. PLoS One 8: e74074 (2013).

39. Swedenborg, E., Ruegg, J., Makela, S. & Pongratz, I. Endocrine disruptive chemicals: mechanisms of action and involvement in metabolic disorders. J. Mol. Endocrinol. 43(1), 1-10 (2009).

40. Le Maire et al. Activation of RXR-PPAR heterodimers by organotin environmental endocrine disruptors. EMBO Rep. 10(4), 367-73 (2009).

41. Li, Y., Lambert, M.H. & Xu, H.E. Activation of nuclear receptors: a perspective from structural genomics. Structure 11(7), 741-6 (2003).

42. Leonardi, R., Zhang, Y-M., Rock, C.O. & Jackowski, S. Coenzyme A: Back in action. Progress in Lipid Res. 44(2-3), 125-153 (2005).

43. Ku, B., Liang, C., Jung, J.U. & Oh, B.H. Evidence that inhibition of BAX activation by BCL-2 involves its tight and preferential interaction with the BH3 domain of BAX. Cell Res. 21, 627-641 (2011).

44. Ogu, C. & Maxa, J. Drug interactions due to cytochrome p450. Proc. (Bayl. Univ. Med. Cent.) 13, 421-423 (2000).

45. McGehee, R. Jr, Ronis, M., Cowherd, R., Ingelman-Sundberg, M. & Badger, T. Characterization of cytochrome p450 2e1 induction in a rat hepatoma FGC-4 cell model by ethanol. Biochem. Pharmacol. 48, 1823-1833 (1994).

46. Nanji, A. et al. Markedly enhanced cytochrome p450 2e1 induction and lipid peroxidation is associated with severe liver injury in fish oil-ethanol-fed rats. Alcohol Clin. Exp. Res. 18, 1280-1285 (1994).

47. Wu, D. & Cederbaum, A. Ethanol and arachidonic acid produce toxicity in hepatocytes from pyrazole-treated rats with high levels of CYP2E1. Mol. Cell Biochem. 204, 157-167 (2000).

48. Wu, D. & Cederbaum, A. Cyclosporine a protects against arachidonic acid toxicity in rat hepatocytes: role of CYP2E1 and mitochondria. Hepatology 35, 1420-1430 (2002).

49. Hamosh, A., Scott, A., Amberger, J., Valle, D. & McKusick, V. Online mendelian inheritance in man (OMIM). Hum. Mutat. 15, 57-61 (2000).

50. Stenson, P. et al. The human gene mutation database: building a comprehensive mutation repository for clinical and molecular genetics, diagnostic testing and personalized genomic medicine. Hum. Genet. 133, 1-9 (2014).

51. Diller, D. & Li, R. Kinases, homology models, and high throughput docking. J. Med. Chem. 46, 4638-4647 (2003).

52. Knox, C. et al. Drugbank 3.0: a comprehensive resource for ’omics’ research on drugs. Nucleic Acids Res. 39, D1035-41 (2011).

53. Perot, S., Sperandio, O., Miteva, M.A., Camproux, A.C. & Villoutreix, B.O. Druggable pockets and binding site centric chemical space: a paradigm shift in drug discovery. Drug Disc. Today 15, 656-667 (2010).

54. Wang, G. & Dunbrack, R. Jr. PISCES: a protein sequence culling server. Bioinformatics 19, 1589-1591 (2003).

55. Cavallo, L., Kleinjung, J. & Fraternali, F. POPS: A fast algorithm for solvent accessible surface areas at atomic and residue level. Nucleic Acids Res. 31, 3364-3366 (2003).

56. Wu, C. et al. The universal protein resource (uniprot): an expanding universe of protein information. Nucleic Acids Res. 34, D187-91 (2006).

57. Riu, A. et al. Peroxisome proliferator-activated receptor $\gamma$ is a target for halogenated analogs of bisphenol A. Environ. Health Perspect. 119(9), 1227-32 (2011).

58. Sheibani, N., Tang, Y. & Sorenson, C. M. Paxillin’s LD4 motif interacts with bcl-2. J. Cell. Physiol. 214, 655-661 (2008).

59. Pantoliano, M.W. et al. High-Density Miniaturized Thermal Shift Assays as a General Strategy for Drug Discovery. J. Biomol. Screen. 6(6), 429-440 (2001).

### Example 2

### Evaluation of the *PPE* and Probabilistic Scoring Function

To investigate the capacity of *PPE* to retrieve structurally similar drug binding pockets, we compared the protein pocket bound by 2'-monophosphadenosine 5'-diphosphoribose with the predicted binding pockets of the top 10 predicted targets of this compound. Our sequence order-independent alignment superimposed the ten predicted targets with an average normalized RMSD of 0.27 Å to the constructed drug *PPE* (an alignment contained > 4 atoms). Conversely, using sequence order-dependent structure alignment, only 2 predicted pockets (in PDB id 2BXP and 3ELM) align to the established drug pocket with an average normalized RMSD of 4.06Å. Similarly, using sequence order-dependent alignment the top 10 targets aligned on average 9.5 atoms to the established drug pocket with an average RMSD of 0.68 Å as compared to the *PPE* that resulted in an average alignment length of 12.4 atoms with an average RMSD of 0.63 Å. This result supports that a combination of the minimalistic *PPE* and sequence order-independent alignment is more useful in identifying new drug targets than the combination of the complete binding pocket and sequence order-dependent structure alignment.

Moreover, to determine the effectiveness of the probabilistic sequence similarity function, we compared the probabilistic function with the deterministic similarity function used by Dundas *et al.* [29] and found that the average sequence similarity of the top 10 predicted novel targets of 2'-monophosphadenosine 5'-diphosphoribose using the deterministic function was 55% as compared with the 82% observed using the probabilistic function. These observations supported that the *PPE* is able to extract the non-trivial sequence and structure signatures necessary to capture the promiscuous process of the drug binding to multiple sites and the sites binding to multiple drugs, as described by Gao and Skolnick [28]. Most of the predicted novel targets spatially align with distinct parts of the *PPE's* of the respective drugs (SI Figure 1), suggesting that the *PPE* is indeed an ensemble of several pockets and therefore might be able to accommodate the different conformers of each drug. These results suggest that multiple structural signatures might not be the optimal way to capture the different conformations of drugs in contrast to previous studies [1, 3] and that the problem can be effectively addressed by incorporating a probabilistic scoring function in the structural signatures as demonstrated in this study. Moreover, expert knowledge about the number of binding conformations and hence the number of structural signatures is not required in our methodology.

### 2. Integrating the Drug Delivery Profile to Reduce False Positives

We extracted all the genes whose mRNA tissue expression profile matched the *aDDP* of β-D-glucose targets. We found 99 genes satisfying the above criteria. We constructed 3 sets from the 99 genes, the first set consisted of the 10 genes that had the best sequence and structure similarity scores when aligned to the *PPE* of β-D-glucose, the second set consisted of the 10 genes that had the worst sequence and structure similarity scores when aligned to the *PPE* of β-D-glucose and the third set consisted of the first two sets and 5 randomly chosen genes. Using the Co-citation Index and their p-values, we compared the first two sets with the top 10 predictions by *iDTP* for β-D-glucose and the third set with the top 25 predictions for β-D-glucose obtained by integrating sequence, structure and tissue expression scores (SI Table 1). We found that *aDDP* alone achieves already a 10-12% enrichment of likely targets, according to our criteria, strongly suggesting that it is a valuable discriminator. When *aDDP* was combined with the *PPE,* the number of predictions with statistically significant co-citation index was 2-4 fold higher (36-40% enrichment). Indeed, for the combined approach the top 10 predicted targets (< 60% sequence similarity with any of the known drug targets) of β-D-glucose aligned extremely well with the *PPE* of β-D-glucose (SI Table 2) as reflected by an average sequence similarity score of 82%, RMSD of 0.62Å, a match for mRNA expression profile in 74 of the total of 79 tissues, and an overall average final score of 0.51 (compare to the cut-off value of 0.85).

### References for Example 2

60. Dundas, J., Adamian, L. & Liang, J. Structural signatures of enzyme binding pockets from order-independent surface alignment: a study of metalloendopeptidase and NAD binding proteins. J. Mol. Biol. 406, 713-729 (2011).

61. Gao, M. & Skolnick, J. A comprehensive survey of small-molecule binding pockets in proteins. PLoS Comput. Biol. 9, e1003302 (2013).

62. Tseng, Y. & Liang, J. Estimation of amino acid residue substitution rates at local spatial regions and application in protein function inference: a bayesian monte carlo approach. Mol. Biol. Evol. 23, 421-436 (2006).

**SI Table 1. Co-citation comparison between predictions using tissue expression data only versus the predictions using the sequence, structure, and expression data.** We constructed 3 sets from the 99 genes whose mRNA expression profile matched the *aDDP* of β-D-glucose exactly, the first set consisted of the 10 genes that had the best sequence and structure similarity scores when aligned to the *PPE* of β-D-glucose, the second set consisted of the 10 genes that had the worst sequence and structure similarity scores when aligned to the *PPE* of β-D-glucose and the third set consisted of the first two sets and 5 randomly chosen genes. Based on co-citation, we found that *aDDP* alone achieves already a 10-20% enrichment of likely targets, strongly suggesting that it is a valuable discriminator. When *aDDP* was combined with the *PPE,* the number of predictions with statistically significant co-citation index was 2-4 fold higher (36-40% enrichment).

| | **Sequence+Structure+Expression** | | **Expression** | | |
|---|---|---|---|---|---|
| **P-Value** | **Top** 10 | **Top 25** | **Top 10 (Best)** | **Top 10 (Worst)** | **Top 25** |
| <0.001 | 2 | 5 | 1 | 1 | 2 |
| <0.005 | 3 | 6 | 2 | 1 | 3 |
| <0.01 | 4 | 9 | 2 | 1 | 3 |
| **Enrichment** | **40%** | **36%** | **20%** | **10%** | **12%** |

**SI Table 2. The top 10 predicted target genes of β-D-glucose.** The top 10 predicted target genes of β-D-glucose, their PDB IDs, their sequence similarity score, structure similarity score (RMSD), expression similarity score, combined distance score, the co-citation index, related p-value, and the number of research papers where the drug and gene name are found in the abstract simultaneously.

| | | Alignment | | | | Validation | | |
|---|---|---|---|---|---|---|---|---|
| **Gene** | **PDB** | **Sequence** | **Structure** | **Expression** | **Distance** | **CI** | **P-value** | **Literature** |
| Neprilysin Bifunctional | 2QPJ | 0.90 | 0.69 | 75 | 0.43 | 2.36 | 0.027 | 5 |
| epoxide hydrolase 2 | 3ANT | 0.83 | 0.55 | 73 | 0.46 | 3.10 | 0.012 | 9 |
| Ephrin type-A receptor 3 | 3FXX | 0.81 | 0.54 | 77 | 0.48 | 0.00 | NA | 0 |
| Renin | 2G26 | 0.86 | 0.62 | 73 | 0.48 | 3.58 | 0.004 | 13 |
| Estrogen receptor | 3ERT | 0.82 | 0.65 | 75 | 0.51 | 5.39 | <0.001 | 48 |
| Golgi SNAP receptor complex member 2 | 3EG9 | 0.80 | 0.66 | 75 | 0.54 | 0.00 | NA | 0 |
| Serine/threoni ne-protein kinase Chk2 | 2XM8 | 0.82 | 0.58 | 67 | 0.54 | 0.00 | NA | 0 |
| DCC-interacting protein 13-alpha | 2Q12 | 0.79 | 0.63 | 73 | 0.54 | 3.47 | 0.006 | 12 |
| cGMP-dependent protein kinase | 3NMD | 0.79 | 0.62 | 77 | 0.54 | 4.63 | <0.001 | 28 |
| 3 cGMP-specific 3',5'-cyclic phosphodieste rase | 3B2R | 0.79 | 0.69 | 77 | 0.54 | 0.77 | 0.142 | 1 |

**SI Table 3. Drug - Predicted Target - Disease Relationships.** The predicted targets for which statistically significant co-citation index (p-value < 0.005) was found along with the diseases associated with these genes and the keywords used to find associations between the drugs and the predicted genes.

| Drug | Predicted Target | PDB | Keyword(s) | Associated Diseases |
|---|---|---|---|---|
| Coenzyme A | Peroxisome proliferator-activated receptor gamma | 3ADS | Coenzyme A | Severe Insulin resistance, lipodystrophy, severe obesity, type-2 diabetes, dyslipidaemia and colorectal cancer |
| 2'-Monophosphadenosine 5'-Diphosphoribose | Cellular tumor antigen p53 | 3D0A | NADPH | Several types of cancer |
| | Serum albumin | 2BXP | | Analbuminaemia, familial dysalbuminaemic hyperthyroxinaemia, bisalbuminaemia, dysalbuminemic hyperzincemia |
| | Vascular endothelial growth factor receptor 2 | 3B8Q | | Coronary heart disease, coronary artery lesions of kawasaki disease, infantile haemangioma |
| Nicotinamide-Adenine-Dinucleotide | Nicotinate-nucleotide pyrophosphorylase [carboxylating] | 3LAR | NAD | |
| | Vascular endothelial growth factor receptor 2 | 3EFL | | Coronary heart disease, coronary artery lesions of kawasaki disease, infantile haemangioma |
| Formic Acid | Cytochrome P450 2E1 | 3KOH | Formic / carboxylic acid | Relapsing pneumonia |
| | Cellular tumor antigen p53 | 3OQ5 | carboxylic acid | Several types of cancer |
| Phosphoaminophosphonic Acid-Adenylate Ester | Kinesin-like protein KIF11 | 2FL6 | AMP-PNP | Microcephaly with or without chorioretinopathy, lymphedema, or mental retardation |
| | RAC-beta serine/ threonine-protein kinase | 3D0E | | Type II diabetes mellitus, hypoinsulinemic hypoglycemia with hemihypertrophy, severe insulin resistance |
| Flavin- Adenine Dinucleotide | Insulin | 2OMI | Adenosine, Riboflavin | insulin-dependent diabetes mellitus, neonatal diabetes mellitus, type 1 diabetes mellitus, familial hyperproinsulinemia |
| | Estrogen receptor | 1X7R | | Breast cancer, estrogen resistance, migraine, myocardial infarction, precocious puberty, left ventricular hypertrophy |

(continued)

| Drug | Predicted Target | PDB | Keyword(s) | Associated Diseases |
|---|---|---|---|---|
| Heme | Serum albumin | 2I2Z | Heme | Analbuminaemia, familial dysalbuminaemic hyperthyroxinaemia, bisalbuminaemia, dysalbuminemic hyperzincemia |
| | Cytochrome P450 2A13 | 2PG6 | | Reduced promoter activity, decreased catalytic efficiency |
| β-D-glucose | Renin | 2G26 | Glucose | Familial hyperproreninemia, renal tubular dysgenesis, familial juvenile hyperuricemic nephropathy, anaemia, hypouricosuric hyperuricaemia, kidney failure, hypertension |
| | Estrogen receptor | 3ERT | | Breast cancer, estrogen resistance, migraine, myocardial infarction, precocious puberty, left ventricular hypertrophy |
| | cGMP-dependent protein kinase 1 | 3NMD | | Familial aortic aneurysm |

**SI Table 4. Melting temperatures (Tm) of hPPARγ-LBD.** Tm calculated from thermal denaturation curves of hPPARγ-LBD in presence of varying molar excess of Rosiglitazone or Coenzyme A. Rosiglitazone displays a protective effect (raise of Tm) against thermal denaturation, in contrary of Coenzyme A which decreases the Tm.

| Protein | Ligand | Tm (°C) |
|---|---|---|
| 5 μM hPPARg | Ø | 47.2 |
| | 35 μM Rosiglitazone | 49.2 |
| | 35 μM Coenzyme A | 46.6 |
| | 70 μM Coenzyme A | 46.5 |
| | 140 μM Coenzyme A | 45.5 |
| | 280 μM Coenzyme A | 41.7 |
| | 1400 μM Coenzyme A | 27.5 |

**Example 3**

***De novo* drug discovery**

For a new drug the following protocol can be followed to used our methodology

1. Potential binding partners can be identified from a list of commonly observed protein targets (e.g. Nature Reviews Drug Discovery 5, 821-834 (October 2006)).
2. To test the binding of soluble drugs to the initial list of proteins that can be obtained recombinantly in sufficient quantity, we can use isothermal titration calorimeter experiments or surface plasmon resonance. For less available proteins, or drugs that require particular solvents such as DMSO, we can use methods such as microscale thermophoresis or differential scanning fluorimetry. Many known protein drug targets can be obtained readily commercially, and many expression plasmids are available in the non-profit ADDGENE database.
3. To obtain the binding site of the new drug to one of the above proteins (with sufficiently high interaction strength), we can setup x-ray crystallographic studies.

4. Once the binding site information is available from x-ray crystals and the initial set of protein targets are known from step 2, we can run our algorithm to discover other protein targets.

**Example 4**

**Methods**

**Dataset**

We extracted the approved/experimental drugs from the DrugBank database (version 3) [52]. Eleven drugs selected for use in this study had at least one 3D structure of a drug-protein complex and more than 40 known drug targets with solved apo structures (Table 1). During the *in silico* validation of our method, a 5-fold cross-validation is done on the known targets of each drug to evaluate how well our method can recover the known targets. For example, for a drug with 40 known targets, only 32 structures are used to construct the PPE for each fold. When our method is used to predict new targets, all the known targets are used to construct the PPE for a drug. Therefore, in this study, our dataset contains drugs with 40 or more known targets because our experiments involve 5-fold cross-validation. In practical use of our method, 30 known targets are sufficient. We expect this number to be further reduced in the future. We expect that our method is also suitable for much larger sets of drugs established for proprietary research that are not detailed in public databases.

Protein structures have more than 30 pockets on average (some structures have >100 pockets), and a majority of the small-molecule protein interactions occur in the three largest pockets [32]. A typical pocket involved in small-molecule protein interactions (also known as a druggable pocket) has characteristic values for pocket solvent accessible surface area 300-600 A° 2) and pocket volume (400-600 A° 3) [28; 53]. We hypothesize that a protein structure that has a minimal (i.e. less than three) number of pockets and no druggable pocket is unlikely to interact with a drug. To form the negative dataset, we extracted the protein structures with fewer than three pockets from the CASTp database [31]. We extracted the surface residues (>70% of the solvent-accessible surface area) of these protein structures using POPS after redundancy reduction using the PISCES webserver (<60% pair-wise sequence identity) [55; 54]. We further removed the NMR structures, the structures with cocrystallized DNA, RNA or ligands (excluding ions like Zn2p, Cl-), and the structures with druggable pockets. Our negative dataset contained a total of 63 protein structures (a detailed list of PDB IDs can be found in the Supplementary file NEG dataset -- SI Table 8). Note that our negative dataset thus can still contain protein structures with less than three pockets. However, if they do so, none of the pockets can be druggable. The surface residues of the structures in the negative dataset were aligned with the PPEs of all drugs in the dataset by CPalign [65]. The alignments were then scored by the scoring function defined herein.

Ideally, each of these alignments should have a bad (i.e. high) score, so it will not be predicted to be a drug target. Otherwise, it is counted as a false-positive prediction when we evaluate the specificity of our method.

**Constructing a PPE**

The overview flowchart of our method is shown in Figure 11. After identifying the drug-target complex for each drug in the dataset, we extracted the pocket that the drug binds to in the protein structure using the CASTp webserver [31], which we refer to as the 'bound pocket'. To identify the drug-binding site in apo structures of known drug targets, we extracted the three largest pockets from the first chain of their respective 3D structures. We used sequence order-independent alignment to choose the pocket most similar to the bound pocket [64; 29]. Dundas et al. [29] established a method to construct the structural signatures for enzyme binding pockets that require high-quality, manually curated enzyme binding sites and is, there- fore, not suitable for high-throughput studies. However, we reduced this requirement by using just one manually curated pocket (except for nicotinamide-adenine-dinucleotide where we used both bound structures that are available) and predicting the binding pockets on the rest of the targets from their apo (unbound) structures to construct the PPE for each drug. Conversely, Dundas et al. manually searched the literature to find residues that are important for the interaction and mapped them back onto the apo structures. The PPE represents a unified set of individual pockets that potentially bind to several conformations of the drug.

Extraction of the common structural features from the set of binding pockets is ideally performed using a multiple structure alignment method. However, because no such method currently exists that can handle our dataset, we followed Dundas et al. [29] by first using pairwise sequence order-independent structure alignment of surface pockets and then using hierarchical clustering based on the pairwise similarities. Dundas et al. constructed several structural signatures corresponding to the different ligand/ligand binding site conformations at a predefined specific level of the hierarchical tree. In most cases, identifying this cutoff is nontrivial and requires in-depth knowledge about the different conformations of the ligand/ligand binding site. In contrast, we constructed the structural signature at the root of the tree. The hierarchical tree is used as a guide to recursively combine sibling pockets along the paths from leaf nodes to the root. A signature

pocket is computed as the average of two child (signature) pockets, and the two original child nodes are replaced with a new single leaf node on the hierarchical tree.

As a result, the structural signature is an ensemble of more than one unique pocket (corresponding to distinct branches in the hierarchical tree). Each position in the PPE has a preservation ratio (how often that particular atom was present in the underlying set of pockets) [29] of at least 0.5 (each atom was present in at least half of the structures that have an atom present after alignment at this position). To achieve a minimalistic ensemble and reduce the computational time, we increased the preservation ratio cutoff to 0.6 if the number of atoms in the PPE was greater than 110. For specific drugs, a stricter conservation ratio of atoms essential for binding action of the respective drugs can be readily incorporated in our method.

**Calculating distance to the PPE**

Each position in the structural signature may be occupied by more than one type of atom (which can be from different residues). Therefore, we formulated a probabilistic distance function to accommodate this property. The distance function of a query protein to the already constructed PPE has both structural and sequence components. The structural component follows Dundas et al.'s approach, while the sequence component is based on maximum likelihood.

**iDTP: integrated Drug Target Prediction Method**

$$\text{Score} = \text{Structural score} + \alpha * \text{Sequence score}$$

$$\text{Structural score} = \text{RMSD}*\text{N}^{(-1/3)}$$

$$\text{Sequence score} = 1 - (\text{Sequence similarity}/\text{Best sequence similarity})$$

$$\text{Sequence similarity} = \Sigma_i\ (\text{AtomFreq}_i + \text{ResFreq}_i)$$

$$\text{Best sequence similarity} = \Sigma_i\ (\text{MaxAtomFreq}_i + \text{MaxResFreq}_i)$$

where the value for $\alpha$ is set to 1.2 following Dundas et al. (2011) [29], RMSD is the root mean square distance after the alignment, N is the number of positions aligned, $\text{AtomFreq}_i/\text{ResFreq}_i$ is the frequency of aligned atom/residue at position i, $\text{MaxAtomFreq}_i/\text{MaxResFreq}_i$ is the highest frequency of any atom/residue at position i, and their summation is over all the aligned positions. An empirical distance cutoff of 0.85 that maps to an RMSD of 0.7 A° and pocket sequence similarity of 60% for a sequence order-independent alignment of 12-15 atoms is used in this study. An alignment should also contain at least five atoms.

**Integrating aDDP**

We included an approximation for the DDP as an orthogonal source of structure-independent information. The aDDP for each drug is calculated by averaging the mRNA expression of known drug targets over 79 human tissues from Su et al. [36]. We mapped each drug-target structure to a gene using Uniprot ID mapping service [56]; the gene was then searched in the tissue expression dataset compiled by Su et al. [36]. Because a protein structure could be mapped to more than one gene, the average expression of all the mapped genes was used. We classified expression in 79 human tissues into three classes (low, medium and high) based on empirical cutoffs for mRNA expression (<300, <1000 and >1000, respectively). The new drug target list is reordered by including the drug-target tissue expression term in the distance function as follows:

$$\text{Score} = \text{Structural similarity} + \alpha * (1 - \text{Sequence similarity}) + \beta * \text{Tissue expression}$$

Tissue expression = 1 - (Number of tissue with matching expression/Total number of tissues),

where β is empirically set to 0.4. If a gene is not present in the data- set compiled by Su et al., we set (β*Tissue expression) to 0.2. Our method is not sensitive to the specific value of b in the range of 0.3-0.5. b plays an important role in differentiating true targets from the false targets and the most promising targets from less promising ones. However, it usually does not play an important role in ranking the top 10 targets as the top 10 predicted targets have an almost perfect match between their mRNA expression profile and that of the estimated

**In vitro experimental setup**

Detailed sections on protein expression and purification; ligands and peptides; differential scanning fluorimetry; differential static light scattering; fluorescence anisotropy (FA) measurements; and intrinsic tryptophan fluorescence quenching binding assay can be found in the Supplementary Information below.

**Results**

**Probabilistic Pocket Ensemble**

An inherently promiscuous drug can bind to different protein pockets that have a range of features, making it difficult to establish a general description of a drug's possible binding sites. To capture the essential binding site features of a promiscuous drug, we developed a method to construct its PPE (see Section 2 for details). The PPE represents a unified set of individual pockets that potentially bind to several conformations of the drug. Each position in the PPE can consist of a number of atoms from different residues. The frequency of the atoms and residues at each position is recorded and used to construct a maximum likelihood sequence similarity scoring function. This probabilistic scoring method adequately accounts for the fact that a drug can bind several pockets and a pocket can bind several drugs [28]. The PPEs of formic acid, b-D-glucose and phosphoaminophosphonic acid-adenylate ester are shown in Figure 1; each position in the PPE is labeled with the atom of highest frequency. (The PPE represents a unified set of individual pockets that potentially bind to several conformations of the respective drug.)

**Evaluation of the PPE and the probabilistic scoring function**

To investigate the capacity of PPE to retrieve structurally similar drug-binding pockets, we compared the protein pocket bound by 20 -monophosphadenosine 50 -diphosphoribose with the predicted binding pockets of the top 10 predicted targets of this compound using sequence order-independent and sequence order-dependent alignments (see Supplementary Section S1 below). Our results suggest that a combination of the minimalistic PPE and sequence order-independent alignment is more powerful in identifying new drug targets than the combination of the complete binding pocket with sequence order-dependent structure alignment. Moreover, we showed that the probabilistic similarity function performs better than the deterministic similarity function used by Dundas et al. (2011) [29] (see Supplementary Section S1). The PPE is able to extract non-trivial sequence and structure signatures that are necessary for capturing the promiscuous process of a drug binding to multiple sites and the sites binding to multiple drugs. Most of the predicted targets spatially align with distinct parts of a respective drug's PPE (see Figures 6a-c), suggesting that the PPE is indeed an ensemble of several pockets and, therefore, can accommodate different conformations of each drug. In contrast to previous studies [29; 30], these results suggest that multiple structural signatures may not be optimal for capturing different drug conformations, but instead, this can be achieved by the incorporation of a probabilistic scoring function in structural signatures. Moreover, our methodology does not require in-depth details about the number of binding conformations or the number of structural signatures.

In the next step, we used cross-validation (see Supplementary section S3 for details) to assess whether the PPE for each drug predicted targets spatially align with distinct parts of a respective drug's PPE (see Figures 6a-c), suggesting that the PPE is indeed an ensemble of several pockets and, therefore, can accommodate different conformations of each drug. In contrast to previous studies [29; 30], these results suggest that multiple structural signatures may not be optimal for capturing different drug conformations, but instead, this can be achieved by the incorporation of a probabilistic scoring function in structural signatures. Moreover, our methodology does not require in-depth details about the number of binding conformations or the number of structural signatures.

In the next step, we used cross-validation (see Supplementary section S3 for details) to assess whether the PPE for each drug.

## Integrating the DDP to reduce false positives

To identify new drug targets, we downloaded the CASTp database (Dundas et al., 2006), which consists of 75 000 protein structures and their pockets. We extracted the three largest pockets, reported to account for more than 80% of a protein's small-molecular binding sites (32; 67; 69; 34), from each of these protein structures. We aligned the pockets of each protein structure with the PPE (constructed using all the known drug targets) of each drug in our dataset using sequence order-independent structure alignment. This resulted in several thousand hits, a number similar to those of other drug repositioning studies [20]. Although our method has high specificity for the curated dataset, the false-positive rate is expected to be higher in a general database search because our construction of the PPE is minimalistic, and therefore it can align with several unrelated protein surfaces randomly [29; 35].

To reduce the false positive rate of our method, we included an approximation for the aDDP as an orthogonal source of structure- independent information. Given that the actual tissue delivery profile for a specific drug is generally not available, we reasoned that the intracellular delivery profile of this drug has to be compatible with the mRNA expression profile of its established targets. In other words a protein can only be a target of a given drug if the drug is delivered into (or produced in) the tissues in which the protein is expressed at significant levels. For each candidate drug, we therefore approximate its delivery profile by averaging the mRNA expression profiles of all its known targets in a set of 79 human tissues [36].

For the drugs tested, the mRNA expression profiles of the known target proteins are similar for the same drug (e.g. the Pearson correlation coefficient of aDDP of CoA with its known targets is 0.56), but are different between different drugs (e.g. the average Pearson correlation coefficient of aDDP of CoA with the aDDP's of the rest of the drugs is 0.44) as shown in Figure 2. The mRNA expression profiles not only provide information about protein localization but also provide information about protein-protein interactions and pathways [37]. Thus, the comparison of the average tissue expression profile of the established drug targets with the expression profiles of the predicted target is expected to reflect the likelihood for drug-target interactions in a particular set of tissues and hence can be used as a proxy for the drug delivery.

## Validation using in silico experiments

We used text mining to investigate the capacity of an aDDP to predict drug-target interactions. A cocitation index finds the association between two terms (in this case the name of a drug and a gene) by comparing the number of times the two terms appear in the abstract of studies in the PubMed library when compared with two random terms [38]. We found that, when the aDDP was combined with the PPE, the number of predictions with a statistically significant cocitation index was 2- to 4-fold higher than using aDDP alone (see Supplementary Information Section 2 and SI Table 1). The top 10 predicted targets (<60% sequence similarity with any of the known drug targets) of b-D-glucose using the combined approach aligned extremely well with its PPE (SI Table 2). Similar results were observed for all the drugs in the dataset (see Supplementary Information, below, and SI Tables 7 and 8). Moreover, six of the top ten predictions for b-D-glucose and four predictions for CoA have a statistically significant cocitation index (P-value < 0.05). We found a total of 34 predicted targets with cocitation index values of statistical significance (P-value < 0.05) for all the drugs in our dataset. For the top 10 predicted targets of all the drugs in the dataset, the range of the average sequence-similarity score between the PPEs and the predicted pocket on the targets was between 80 and 87%, the range of the average RMSD between the PPEs and the predicted pocket on the targets was between 0.62 and 0.66 A°, the range of the average match of mRNA expression profile between 72 and 76 out of the 79 tissues and the range of the average final score was between 0.45 and 0.56 (compared to our cutoff value of 0.85 in our cross-validation study). These results support that the combination of PPE and aDDP into iDTP allows identifying novel proteins that have high structural (binding site) and system-level similarity with known drug targets.

## Validation using in vitro binding experiments

To provide an experimental assessment of the performance of iDTP, we chose to test the predicted targets for CoA because it has the least number of known binding proteins in our dataset and hence has the least well-defined PPE. We used multiple in vitro binding experiments to test binding site and affinity for two top hits from iDTP, namely the peroxisome proliferator-activated receptor gamma (PPARc) and B-cell lymphoma 2 (Bcl-2).

PPARc is a nuclear hormone receptor that regulates numerous biological functions including adipogenesis and cell differentiation. Its dysregulation is involved in the onset of diabetes and obesity [39]. The interaction of the ligand binding domain (LBD) of human PPARc (hPPARc-LBD) with CoA is one of our most promising predictions because this interaction achieved a high iDTP score and a statistically significant cocitation index (SI Table 3). The pocket predicted by iDTP to bind CoA overlaps with the known ligand binding site of PPARc. To test this prediction in vitro, we used differential scanning fluorimetry to measure the melting temperature (Tm) of hPPARc-LBD in the pre ence or absence

of CoA or rosiglitazone, an antidiabetic drug known to act as a ligand of hPPARc-LBD (Fig. 2a and SI Table 4). At a molar excess of seven, rosiglitazone had a protective effect by raising the Tm of hPPARc-LBD’s by 2o C from that of the apo protein, while CoA displayed a destabilizing effect, lowering the Tm by 0.8o C from that of the apo protein, suggesting a direct interaction with hPPARc-LBD. Next, we used fluorescence anisotropy (FA) to characterize the interaction between hPPARc-LBD and its natural partner proteins upon binding with CoA. We measured Kd between hPPARc-LBD and fluorescein-labeled peptides derived from a coactivator protein (PGC1) and two corepressor proteins (NCoR and SMRT). These experiments were performed in the presence or absence of increasing molar excess of CoA or the reference hPPARc- LBD agonist rosiglitazone or antagonist CD5477 [68]. If an increasing molar excess of the ligand causes the fluorescently labeled coactivator/corepressor Kd to increase, we can infer that ligand binding is taking place because the ligand disturbs binding to coactivators or corepressors. The nature of the ligand-hPPARc-LBD interaction can also be inferred: an agonist ligand enhances binding to a coactivator and decreases binding to a corepressor; an inverse agonist causes the opposite effect; and a neutral antagonist decreases binding for both coactivators and corepressors. Accordingly, adding a 2-10 M excess of the agonist rosiglitazone hPPARc-LBD raised the affinity of hPPARc-LBD for the coactivator PGC1 (Figures 3a and b, SI Table 5). Conversely, a 2 M excess of the antagonist CD5577 lowered the affinity of hPPARc- LBD for the coactivator PGC1 (Figures 3a and b, SI Table 5), whereas the addition of 2-10 M excess of CoA lowered the affinity of hPPARc-LBD for both coactivator PGC1 (Figure 3b) and corepressors NCoR and SMRT (Figures 3c and d), SI Table 5). Collectively, our experiments confirm a direct interaction between CoA and hPPARc-LBD in which CoA behaves as a neutral an- tagonist. From its potency in competing with known ligands and its dose-dependent stabilization of hPPARc-LBD, we estimate an apparent Kd of <500 mM.

We also tested direct binding of CoA to recombinant Bcl-2 (see Supplementary Information, below, and SI Tables 7 and 8). Bcl-2, the founding member of the Bcl-2 family of proteins that control cell death, is an important anti-apoptotic protein and is classified as an oncogene. Using differential static light scattering, we observed that the aggregation temperature Tagg for 0.5 mg/ml apo Bcl-2 was rv57o C. Four hundred nanomoles of the known ligand, Bax-BH3, significantly increased the Tagg to 67o C, whereas the presence of 1 lM of the scrambled LD4 peptide (a negative control) did not alter Tagg. Increasing concentrations of CoA increased Tagg for Bcl-2 up to 62o C, suggesting a direct interaction (Figures 4a and 4b, SI Table 6). By measuring the quenching of intrinsic tryptophan fluorescence of Bcl-2 in the presence of increasing CoA concentrations, we established the Kd of the CoA:Bcl-2 interaction to be 0.38 mM (Figures 4c and d), whereas the Kd of the fluorescent-labeled Bax-BH3 peptide was 128 6 21 nM (Figure 2a). According to iDTP, the CoA binding pocket is adjacent to the Bax-BH binding site with no notable over- lap (Figure 2b). In agreement even 4.7 mM CoA did not reduce the FA of Bax-BH3 (at a concentration of 20 nM, 235 000 times less than CoA), supporting the prediction that the binding sites of CoA and Bax-BH do not overlap (Figure 3). Thus, our in vitro binding experiments strongly support our computational predictions.

**Discussion**

We have developed a computational method to extract implicit structural signatures of a drug binding site from an ensemble of structures of proteins to which this drug binds. We showed that such a PPE, can be built using as few as one structure of a drug-protein complex and a set of apo structures of other known drug-binding proteins. The PPE of a given drug is constructed using sequence order-independent alignments and a probabilistic scoring function, which allows weakly conserved but significant structural patterns of the interactions between the drug and its several target proteins to emerge and be quantified. Thus, our PPE is able to encode features related to promiscuous target interactions and structural flexibility of a drug. The validity of 11 PPEs was confirmed by illustrating that they reliably identify known targets of the respective drugs. We found that by combining a PPE with an aDDP as an orthogonal source of structure-independent information, the resulting method, iDTP, enables large-scale prediction of novel drug targets. The challenge of identifying new drug-target pairs in silico has attracted significant interest from the computational community. However, compared with other algorithms, iDTP includes unprecedented features, because no previous studies have combined sequence order-independent alignment and probabilistic scoring function to model the drug-protein interaction, nor have they employed the aDDP to filter out false positive predictions. Most previous studies have not assessed the performance of their methodolo- gies by exploiting known drug targets, as we did here to validate the success rate of PPE. Because other studies used considerably different datasets and their programs are not publicly available, a direct comparison among methodologies is unfortunately impossible. However, compared with iDTP, other existing methods including conventional docking or structure-based virtual screening, share one or more of the following limitations: (i) they are known to scale poorly with the size and complexity of drugs and drug binding sites [51] and (ii) their algorithms do not appropriately account for the different conformations of both drug and binding site residues. Our method addresses these concerns by constructing a structural signature from a set of binding sites, instead of a single binding site, and by using a probabilistic sequence similarity function that allows accounting for the different conformations of drugs and binding site residues. (The improvement expected from this methodology is analogous to the improvement from a multiple sequence alignment compared to a pairwise alignment.) We also incorporated the aDDP to identify relevant new targets.

The predictive power of iDTP was supported by both computational cross-validation and text mining. Additionally, we validated two of our predicted interactions by in vitro experiments. First, we showed that CoA bound to hPPARc-LBD with an apparent Kd of less than 500 mM, displaying characteristics of a neutral antagonist. CoA is a ubiquitous cofactor that can reach high concentrations in eukaryotes depending on cell type and subcellular localization (rv0. 14, 0.7 and 5 mM in animal cytosol, peroxysomes and mito- chondria, respectively) [42]. It is therefore possible that this predicted interaction plays a currently unrecognized biological role in fatty acid signaling and metabolism. iDTP predicted that the CoA binding site on hPPARc-LBD is the receptor's ligand-binding pocket, which also binds rosiglitazone and CD5477. Indeed, the ligand-binding pocket of hPPARc is one of the largest among the nuclear receptor protein family [41], allowing hPPARc to bind a variety of ligands. Thus, CoA may trigger a con- formational change that disrupts or unsettles the binding surface of both coactivators and corepressors, producing the characteristics of a neutral antagonist. However, we cannot strictly rule out that CoA binds to the surface where coactivators and corepressors would normally bind, creating competition for the binding site.

Second, we verified another CoA interaction predicted by iDTP by showing that CoA binds in vitro to recombinant Bcl-2 with a Kd of rv350 1M. The predicted CoA binding pocket on Bcl-2 is adjacent to the known binding site for Bax-BH3. Because we showed that CoA binds Bcl-2 without displacing Bax-BH3, we can indeed infer non-competitive binding. The predicted binding pocket of CoA is interesting for drug design purposes, because it is located adjacent to the well-explored Bax-BH3 binding pocket [43]; therefore, it may provide an alternative target site with possible synergetic effects.

Beyond validating our computational predictions, our in vitro experiments also suggest the usefulness of iDTP for various applications: The case of the CoA-hPPARc illustrates how iDTP might be used to reveal biologically relevant interactions between small molecules (ligands, cofactors or metabolites) and cellular proteins. Thus, our method could help establish metabolite-protein pairs for large-scale metabolic analyses or for predicting possible targets for chemical small-molecule pollutants such as bisphenols. The interaction between CoA and Bcl-2 illustrates how iDTP could be used for drug discovery by suggesting possible lead compounds and novel druggable protein binding pockets. In addition, iDTP could provide insight into the binding mechanisms of known drugs for which the drug-target complex has not yet been determined. For example, our results suggest that formic acid binds to CYP2E1 (Supplementary Information, below, and SI Tables 7 and 8). CYP2E1 is an enzyme known to interact with more than 70 small drugs and xenobiotic compounds (Ogu and Maxa, 2000). Induction of CYP2E1 has been shown to cause oxidative stress and alcohol-induced liver injury in mouse models [45; 46]; however, Trolox[6-hydroxy,2,5,7,8-tetramethylchroman-2-carboxylic acid], a drug that contains the formic acid structure, has been shown to reduce the aforementioned toxicity [47; 48]. Hence, our results suggest a direct interaction between the Trolox formic acid moiety and CYP2E1 that results in reduced toxicity.

To further evaluate the usefulness of iDTP for pharmaceutical purposes, we identified the genetic diseases associated with the predicted target proteins for each drug using the databases-Online Mendelian Inheritance in Man [49] and Human Gene Mutation Database [50]. A cocitation index with high statistical significance ($P < 0.005$) was found for 16 predicted drug-target pairs (including CoA-hPPARc). The predicted drug targets were associated with major human diseases, such as cancer, heart problems and metabolic dysfunctions (SI Table 3), making these results a potentially valuable basis for drug discovery and repositioning. However, the use of iDTP for drug repositioning, in the strict sense of re-using a FDA-approved chemical compound, remains currently limited, as a relatively large set of 3D structures of known targets is required to construct a high-confidence PPE. The rapid pace of experimental determination of protein structures will reduce this limitation in the future.

**Additional References for Example 4**

63. Alam, T. et al. (2014) How to find a leucine in a haystack? Structure, ligand recognition and regulation of Leucine-Aspartic acid (LD) motifs. Biochem. J., 460, 317-329.

64. Cui, X. et al. (2015) Finding optimal interaction interface alignments between biological complexes. Bioinformatics, 31(12): i133-i141.

65. Dundas, J. et al. (2007) Topology independent protein structural alignment BMC Bioinformatics, 8, 388.

66. Dundas, J. et al. (2011) Structural signatures of enzyme binding pockets from order-independent surface alignment: a study of metalloendopeptidase and NAD binding proteins. J. Mol. Biol., 406, 713-729.

67. Laskowski, R.A. (1995) SURFNET: A program for visualizing molecular surfaces, cavities, and intermolecular interactions. J. Mol. Graph., 13, 323-330.

68. LeMaire, A. et al. (2009) Activation of RXR-PPAR heterodimers by organotin environmental endocrine disruptors. EMBO Rep., 10, 367-373.

69. Liang, J. et al. (1998) Anatomy of protein pockets and cavities: Measurement of binding site geometry and implications for ligand design. Prot. Sci., 7, 1884-1897.

70. Peters, J. (2013) Polypharmacology-foe or friend? J. Med. Chem., 56, 8955-8971.

71. Wang, G. and Dunbrack,R.Jr (2003) PISCES: a protein sequence culling server. Bioinformatics, 19, 1589-1591.

72. Wu, C. et al. (2006) The universal protein resource (uniprot): an expanding universe of protein information. Nucleic Acids Res., 34, D187-91.

**Supplementary Information**

**1. Evaluation of the PPE and Probabilistic Scoring Function**

To investigate the capacity of a PPE to retrieve structurally similar drug-binding pockets, we compared the protein pocket bound by 2'-monophosphadenosine 5'-diphosphoribose with the predicted binding pockets of the top 10 predicted targets of this compound. Our sequence order-independent alignment superimposed the top 10 predicted targets with an average normalized RMSD of 0.27 Å to the constructed drug PPE (an alignment contained > four atoms). Conversely, sequence order-dependent alignment superimposed only three predicted pockets (in PDB ID-1EM2, 2BXP and 3ELM) with an average normalized RMSD of 4.02 Å to the 2'-monophosphadenosine 5'-diphosphoribose bound pocket. Similarly, only 20 predicted pockets among the 110 predicted pockets for the 11 drugs using sequence order-dependent superimposed to the 2'-monophosphadenosine 5'-diphosphoribose bound pocket with an average normalized RMSD of 4.05 Å (average alignment length of 8.35), as compared to the sequence order-independent alignment superimposed the 110 predicted pockets with an average normalized RMSD of 0.28 Å (average alignment length of 12.11) to the constructed drug PPE. This result supports that a combination of the minimalistic PPE and sequence order-independent alignment is more useful in identifying new drug targets than the combination of the complete binding pocket and sequence order-dependent structure alignment. Moreover, to determine the effectiveness of the probabilistic sequence similarity function we compared the probabilistic function with the deterministic similarity function used by Dundas *et al.* [29]. We found that the average sequence similarity of the top 10 predicted novel targets of 2'-monophosphadenosine 5'-diphosphoribose to 55% and 82% for the deterministic and probabilistic functions, respectively. This trend was similar across the 11 drugs in the data set (average sequence similarity of 54% using deterministic function and 82% using probabilistic function). These observations support the ability of a PPE to extract the nontrivial sequence and structure signatures necessary to capture the promiscuous process of a drug binding to multiple sites and vice versa as described by Gao and Skolnick [28]. Most of the predicted targets spatially aligned with distinct parts of the PPE's of the respective drugs (see process diagram of Figure 11), suggesting that the PPE is an ensemble of several pockets and, therefore, possibly accommodates the different conformations of each drug. In contrast with previous studies [1; 3], these results suggest that multiple structural signatures are unlikely to be optimal for capturing different conformations of drugs; instead, the problem can be effectively addressed by incorporating a probabilistic scoring function in the structural signatures. Moreover, our methodology does not require in-depth knowledge about the number of binding conformations or subsequent structural signatures.

**2. Integrating the Drug Delivery Profile to Reduce False Positives**

We extracted all genes whose mRNA tissue expression profile matched the aDDP of β-D-glucose targets. We found 99 genes satisfying the above criteria, from which we constructed three sets. The first set consisted of the 10 genes with the best sequence and structure similarity scores when aligned to the PPE of β-D-glucose, the second set consisted of the 10 genes that had the worst sequence and structure similarity scores when aligned to the PPE of β-D-glucose, and the third set consisted of the first two sets and five randomly chosen genes. Using the cocitation index and their p-values, we compared the first two sets with the top 10 predictions by iDTP for β-D-glucose and the third set with the top 25 predictions for β-D-glucose obtained by integrating sequence, structure, and tissue expression scores (SI Table 2). According to our criteria, aDDP alone had a 10-12% enrichment of likely targets, strongly suggesting that it is a valuable discriminator. When aDDP was combined with the PPE, the number of predictions with statistically significant cocitation index was two- to four-fold higher (36-40% enrichment). Using the combined approach, the top 10 predicted targets (< 60% sequence similarity with any of the known drug targets) of β-D-glucose aligned extremely well with the PPE of β-D-glucose (SI Table 3) as reflected by an average sequence similarity score of 82%, an RMSD of 0.62 Å, a

match for mRNA expression profile in 74 of the total of 79 tissues, and an overall average final score of 0.51 (compare to the cutoff value of 0.85).

### 3. Cross-validation of the *PPE*

We employed cross-validation (five-fold for drugs that have less than 100 known targets and three-fold for drugs with more than 100 targets) to assess whether the PPE for each drug captures the essential features for the drug-protein interaction. The known targets of each drug were randomly divided into five (or three) equal sets. Four (or two) sets were used to make the PPE and predictions were provided for the fifth (or third) set. We repeated this procedure five (or three) times for each drug.

### 4. Discussion on negative dataset

A known target for another drug can also be a target for the drug in our dataset. As negative results are usually not published in literature, it was impossible for us to build a more comprehensive negative dataset. A more comprehensive negative dataset might even help improve the scoring function, but due to the lack of available data we have settled on the current negative dataset. We plan to manually curate a negative dataset from existing literature to construct a more comprehensive Negative dataset for each drug in the dataset.

### 5. *In vitro* experimental setup

**Protein expression and purification.** Expression and purification of the ligand-binding domain (LBD) of human PPARγ (amino acids Glu196-Tyr477) have been described previously [57]. Ku *et al.* described the sequence, production, and purification of chimeric human Bcl-2 (with the internal loop 51-91 removed and residues 35-50 replaced by residues 33-48 of mouse Bcl-XL) [43].

**Ligands and peptides.** BRL49653 (Rosiglitazone) and Coenzyme A for hPPARy binding assays were purchased from Sigma-Aldrich (St Quentin Fallavier, France). The fluorescent-labeled peptides FITC-EEPSLLKKLLLAPA, FITC-DPASNLGLEDIIRKALMGSFD, and FITC-TNMGLEAIIRKALMGKYDQWEE, corresponding to the PGC1-NR2, NCoR-RID2, and SMRT-RID2, respectively, were purchased from EZbiolab (Westfield, Indiana, USA). For the Bcl-2 interaction studies, the fluorescent-labeled peptide QDASTKKLSE CLRRIGDELDSNMELQRMIAD corresponding to Bax-BH3 (a known ligand for Bcl-2), and the scrambled LD4 peptide (LSDAMETSSLRDALE, a scrambled version of the Bcl-2 ligand LD4) [58] was purchased from Genscript USA inc. Coenzyme A (CoA) was bought from Calbiochem (VWR, UK).

**Differential scanning fluorimetry (DSF or Thermofluor).** This method measures protein unfolding based on fluorescence detection of the denatured form of the protein (Pantoliano *et al.,* 2001). Solutions of 15 μL containing 5 μM hPPARγ LBD, varying molar excess of ligands (Rosiglitazone, Coenzyme A, or CD5477), 1X Sypro Orange in 50 mM Tris pH 8.0, and 200 mM NaCl were added to the wells of a 96-well PCR plate. Final DMSO concentration did not exceed 5% and had no influence on the data. The plates were sealed with an optical sealing tape (Bio-Rad) and heated in an Mx3005P Q-PCR system (Stratagene) from 25°C to 95°C at 1°C intervals. Fluorescence changes in the wells were monitored with a photomultiplier tube. The wavelengths for excitation and emission were 545 nm and 568 nm, respectively. The melting temperatures, $T_m$, were obtained by fitting the fluorescence data with a Boltzmann model using GraphPad Prism software. The data reported here are the averages of independent experiments and error bars correspond to standard deviations.

**Differential static light scattering (DSLS).** DSLS measured by the Stargazer system (Harbinger Biotechnology and Engineering Corporation, Markham, Canada) was used to assess the thermal stability of Bcl-2 in the presence or absence of CoA and to control ligands. DSLS measures the specific aggregation temperature, $T_{agg}$, at which a protein aggregates as a result of heat denaturing. Thus, DSLS provides proteins with thermal stability, which is expected to vary in the presence of ligands. Bcl-2 at 0.5 mg/mL was overlaid with mineral oil in a clear bottom 384-well black plate (Corning), and heated from 20 to 85 °C at 1 °C/min; light scattering was detected by a CCD camera every 0.5 °C in the presence or absence of varying concentrations of CoA, yielding the difference in aggregation temperature at a particular CoA concentration from apo Bcl-2 $\Delta T_{agg}$. Data were normalized and $K_d$ values were calculated by plotting $\Delta T_{agg}$ against the CoA concentration. Resulting data were fitted using a binding-saturation single-site model (GraphPad).

**Fluorescence anisotropy measurements.** Binding affinities of the fluorescent peptides for hPPARγ LBD were measured in the presence or absence of ligands using a Safire2 microplate reader (TECAN). Fluorescent-labeled peptides were measured using an excitation wavelength of 470 nm; emission was measured at 530 nm. Data are the average of independent experiments and error bars correspond to standard deviations. The buffer solution for assays consisted of 20 mM Tris-HCl pH 7.5, 150 mM NaCl, 1 mM EDTA, 5 mM dithiothreitol, and 10% (v/v) glycerol. Measurements began at 40 μM of the protein, followed by successive twofold sample dilutions with buffer, until reaching the lowest protein

concentration (9.7 nM). Fluorescent peptides were added to the protein samples at 4 nM to allow the establishment of the titration curve. Added ligands were at a final concentration of 80 μM. The binding affinity of the fluorescently labeled Bax-BH3 peptide towards Bcl-2 was determined using a fluorescence spectrometer from Photon Technologies International, USA. The fluorescent-labeled peptide excitation wavelength was 490 nm and its emission was measured at 520 nm. The $K_d$ value for the peptide was fitted using a single-binding-site model. Competition experiments were performed using the 20nM Bax-BH3 peptide and 400 nM Bcl-2. Fluorescence anisotropy was monitored for concentrations up to 5 mM of CoA.

**Intrinsic tryptophan fluorescence quenching binding assay.** Bcl-2 tryptophans were excited at 280 nM and the emission intensity was measured at 320 nM. 10 μM Bcl-2 was incubated with various dilutions of CoA for 10 min before measurement. Emitted fluorescence was monitored using a PheraStar fluorescence plate reader in 96-well plates. Change in tryptophan fluorescence occurs due to conformational changes in the protein when it is bound to the ligand; differences in the fluorescence intensity were recorded and analyzed. Data were normalized and $K_d$ values were calculated by fitting to a Binding- Saturation single-site model with GraphPad.

**SI Table 5. Dissociation constants measured by fluorescence anisotropy on hPPARγ-LBD.** Mean dissociation constants (Kd) and standard deviations (SD) values measured from fluorescence anisotropy titrations between fluorescent-labelled PGC1-NR2 or N-CORNR2 or S-CORNR2 peptides and hPPARγ LBD in the absence of ligand or in the presence of increasing molar excess of Rosiglitazone, Coenzyme A or CD5477.

| Peptide | Ligand | Molar Excess | Kd | SD |
|---|---|---|---|---|
| **NCoR ID2** | Ø | | 2.22 | 0.57 |
| | **Coenzyme A** | 2 | 2.66 | 0.9 |
| | | 5 | 3.76 | 1.36 |
| | | 10 | 11.41 | 3.61 |
| | | 40 | >100 | NA |
| **SMRT ID2** | Ø | | 0.39 | 0.05 |
| | **Coenzyme A** | 2 | 0.42 | 0.05 |
| | | 5 | 0.46 | 0.03 |
| | | 10 | 0.69 | 0.09 |
| | | 40 | 0.8 | 0.06 |
| **PGC1 NR2** | Ø | | 1.97 | 0.4 |
| | **Rosiglitazone** | 2 | 1.38 | 0.28 |
| | | 5 | 1.1 | 0.11 |
| | | 10 | 1.11 | 0.24 |
| | **Coenzyme A** | 2 | 1.89 | 0.28 |
| | | 5 | 2.98 | 0.55 |
| | | 10 | 3.61 | 1.56 |
| | **CD 5577** | 2 | 6.7 | 1.32 |

**SI Table 6:** Bax-BH3 peptide is a strong interacting partner of Bcl-2 which stabilizes the protein by increase in aggregation temperature and Coenzyme A also found to be increasing the thermal stability of Bcl-2 based on $T_{agg}$ values.

| Protein | Ligand | $T_{agg}$ (°C) |
|---|---|---|
| 0.5 mg/mL of Bcl-2 | - | 56.85 ± 0.14 |
| | 0.1 mM Coenzyme A | 57.65 ± 0.19 |
| | 0.25 mM Coenzyme A | 59.72 ± 1.011 |
| | 0.5 mM Coenzyme A | 59.83 ± 0.21 |
| | 0.75 mM Coenzyme A | 60.49 ± 0.46 |
| | 1 mM Coenzyme A | 60.88 ± 0.46 |
| | 1.5 mM Coenzyme A | 61.12 ± 0.58 |
| | 2 mM Coenzyme A | 61.25 ± 0.66 |
| | 3 mM Coenzyme A | 61.78 ± 0.45 |
| | 400 nM Bax-BH3 peptide | 67.30 ± 1.02 |
| | 1 uM scrambled LD4 peptide | 57.05 ± 0.15 |

**SI Table 7:** Table of predicted targets containing information that can be used in performing the methods described herein.

## Coenzyme A (1XVT)

| PDB | Pocket | Aligned | RMSD | Seq | Distance | :xpressior | Final | Gene Name | Terms | CI | P-value | Literature |
|---|---|---|---|---|---|---|---|---|---|---|---|---|
| 2bdf | 70 | 12 | 0.68 | 0.87 | 0.46 | 0 | 0.46 | Proto-oncogene tyrosine-protein kinase Src | Coenzyme A | 0 | NA | 0 |
| 3pmt | 7 | 11 | 0.63 | 0.86 | 0.45 | 0.02 | 0.47 | Tudor domain-containing protein 3 | | 0 | NA | 0 |
| 3adx | 64 | 12 | 0.52 | 0.79 | 0.48 | 0.01 | 0.49 | Peroxisome proliferator-activated receptor gamma | | 2.7824 | 0.002 | 6 |
| 2o21 | 28 | 12 | 0.6 | 0.8 | 0.5 | 0.03 | 0.53 | Apoptosis regulator Bcl-2 | | 1.56 | 0.007 | 2 |
| 3mr2 | 57 | 12 | 0.49 | 0.74 | 0.52 | 0.01 | 0.53 | DNA polymerase eta | | 0 | NA | 0 |
| 2a5u | 140 | 11 | 0.69 | 0.81 | 0.54 | 0 | 0.54 | ›hatidylinositol 4,5-bisphosphate 3-kinase catalytic subunit gamma isoform | | 0.975 | 0.017 | 1 |
| 1fic | 79 | 11 | 0.59 | 0.8 | 0.51 | 0.04 | 0.55 | Fibrinogen gamma chain | | 0 | NA | 0 |
| 3k75 | 95 | 11 | 0.61 | 0.79 | 0.53 | 0.02 | 0.55 | DNA polymerase beta, DNA repair protein XRCC1 | | 0 | NA | 0 |
| 2xw1 | 158 | 15 | 0.73 | 0.82 | 0.51 | 0.04 | 0.55 | Serum albumin | | 0 | NA | 0 |
| 1afo | 6 | 11 | 0.65 | 0.8 | 0.53 | 0.02 | 0.55 | Glycophorin-A | | 0 | NA | 0 |
| 2j14 | | | 0.67 | 0.8 | 0.52 | 0.03 | 0.55 | Peroxisome proliferator-activated receptor delta | | 2.5463 | .005 | 5 |

## Formic Acid (1B93)

| PDB | Pocket | Aligned | RMSD | Seq | Distance | :xpressior | Final | Gene Name | Terms | CI | P-value | Literature |
|---|---|---|---|---|---|---|---|---|---|---|---|---|
| 3ipx | 36 | 11 | 0.62 | 0.86 | 0.44 | 0.06 | 0.5 | Deoxycytidine kinase | Formic Acid | 0 | NA | 0 |
| 1nbq | 48 | 11 | 0.67 | 0.83 | 0.5 | 0.01 | 0.51 | Junctional adhesion molecule A | | 0 | NA | 0 |
| 1os4 | 40 | 11 | 0.63 | 0.82 | 0.5 | 0.01 | 0.51 | Insulin | | 0 | NA | 0 |
| 1fls | 37 | 11 | 0.65 | 0.83 | 0.5 | 0.01 | 0.51 | Collagenase 3 | | 0 | NA | 0 |
| 1s3w | 24 | 11 | 0.59 | 0.84 | 0.47 | 0.05 | 0.52 | Dihydrofolate reductase | | 0 | NA | 0 |
| 2o2m | 30 | 12 | 0.66 | 0.84 | 0.49 | 0.03 | 0.52 | Bcl-2-like protein 1 | | 0 | NA | 0 |
| 3oq5 | 98 | 11 | 0.54 | 0.8 | 0.49 | 0.03 | 0.52 | Cellular tumor antigen p53 | | 1.5714 | 0.002 | 2 |
| 3km0 | 71 | 11 | 0.64 | 0.81 | 0.51 | 0.01 | 0.52 | tradiol 17-beta-dehydrogenase 1 | | 0 | NA | 0 |
| 1t31 | 30 | 11 | 0.61 | 0.8 | 0.51 | 0.01 | 0.52 | Chymase | | 0 | NA | 0 |
| 3koh | 166 | 12 | 0.65 | 0.81 | 0.51 | 0.02 | 0.53 | Cytochrome P450 2E1 | | 1.9864 | 0.001 | 3 |

## Beta-D-glucose (1PIG)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2qpj | 87 | 14 | 0.69 | 0.9 | 0.41 | 0.02 | 0.43 | Neprilysin | glucose | 2.3552 | 0.027 | 5 |
| 3ant | 112 | 13 | 0.55 | 0.83 | 0.43 | 0.03 | 0.46 | Bifunctional epoxide hydrolase 2 | | 3.0992 | 0.012 | 9 |
| 3fxx | 36 | 11 | 0.54 | 0.81 | 0.47 | 0.01 | 0.48 | Ephrin type-A receptor 3 | | 0 | NA | 0 |
| 2g26 | 82 | 11 | 0.62 | 0.86 | 0.45 | 0.03 | 0.48 | Renin | | 3.5776 | 0.004 | 13 |
| 3ert | 32 | 13 | 0.65 | 0.82 | 0.49 | 0.02 | 0.51 | Estrogen receptor | | 5.385 | <0.001 | 48 |
| 3eg9 | 244 | 14 | 0.66 | 0.8 | 0.52 | 0.02 | 0.54 | Golgi SNAP receptor complex member 2 | | 0 | NA | 0 |
| 2xm8 | 37 | 11 | 0.58 | 0.82 | 0.48 | 0.06 | 0.54 | Serine/threonine-protein kinase Chk2 | | 0 | NA | 0 |
| 2q12 | 25 | 14 | 0.63 | 0.79 | 0.51 | 0.03 | 0.54 | DCC-interacting protein 13-alpha | | 3.4707 | 0.006 | 12 |
| 3nmd | 14 | 11 | 0.62 | 0.79 | 0.53 | 0.01 | 0.54 | cGMP-dependent protein kinase 1 | | 4.6282 | <0.001 | 28 |
| 3b2r | 79 | 15 | 0.69 | 0.79 | 0.53 | 0.01 | 0.54 | MP-specific 3',5'-cyclic phosphodiesterase | | 0.7702 | 0.142 | 1 |
| 3b2r | | | 0.69 | 0.79 | 0.53 | 0.01 | 0.54 | cGMP-specific 3',5'-cyclic phosphodiesterase | | 0.7702 | 0.142 | 1 |

## NAD(1OG3,2BGL)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2azt | 75 | 11 | 0.63 | 0.88 | 0.43 | 0.03 | 0.46 | Glycine N-methyltransferase | NAD | 0 | NA | 0 |
| 3qaq | 158 | 12 | 0.58 | 0.79 | 0.5 | 0 | 0.5 | Phosphatidylinositol 4,5-bisphosphate 3-kinase catalytic subunit gamma isoform | | 0 | NA | 0 |
| 2z0n | 28 | 12 | 0.56 | 0.8 | 0.48 | 0.03 | 0.51 | DCC-interacting protein 13-alpha | | 0 | NA | 0 |
| 1u9e | 62 | 12 | 0.7 | 0.84 | 0.49 | 0.02 | 0.51 | Estrogen receptor beta | | 0.9692 | 0.025 | 1 |
| 1jst | 121 | 12 | 0.61 | 0.83 | 0.47 | 0.05 | 0.52 | Cyclin-dependent kinase 2, Cyclin-A2 | | 0 | NA | 0 |
| 3cbl | 40 | 12 | 0.61 | 0.8 | 0.51 | 0.02 | 0.53 | Tyrosine-protein kinase Fes/Fps | | 0 | NA | 0 |
| 3lar | 188 | 14 | 0.71 | 0.84 | 0.49 | 0.04 | 0.53 | Nicotinate-nucleotide pyrophosphorylase [carboxylating] | | 1.9692 | 0.002 | 3 |
| 1ca9 | 140 | 11 | 0.52 | 0.78 | 0.5 | 0.04 | 0.54 | Tumor necrosis factor receptor superfamily member 1B, TNF receptor-associated factor 2 | | 0 | NA | 0 |
| 3efl | 61 | 14 | 0.61 | 0.78 | 0.52 | 0.02 | 0.54 | Vascular endothelial growth factor receptor 2 | | 1.5542 | 0.003 | 2 |
| 2jgy | 61 | 11 | 0.63 | 0.85 | 0.46 | 0.08 | 0.54 | Uridine 5'-monophosphate synthase | | 0 | NA | 0 |

## Heme (1GGE)

| PDB | Pocket | Aligned | RMSD | Seq | Distance | Expression | Final | Gene Name | Terms | CI | P-value | Literature |
|---|---|---|---|---|---|---|---|---|---|---|---|---|
| 1s8o | 66 | 12 | 0.56 | 0.9 | 0.36 | 0.03 | 0.39 | Bifunctional epoxide hydrolase 2 | Heme | 0 | NA | 0 |
| 2zrs | 175 | 13 | 0.54 | 0.91 | 0.34 | 0.07 | 0.41 | Programmed cell death protein 6 | | 0 | NA | 0 |
| 3lpp | 617 | 11 | 0.6 | 0.86 | 0.44 | 0 | 0.44 | Sucrase-isomaltase, intestinal | | 0 | NA | 0 |
| 1hap | 35 | 14 | 0.7 | 0.88 | 0.43 | 0.03 | 0.46 | Prothrombin | | 0 | NA | 0 |
| 2i2z | 74 | 17 | 0.65 | 0.86 | 0.42 | 0.04 | 0.46 | Serum albumin | | 3.1284 | 0.002 | 8 |
| 1qvn | 65 | 13 | 0.62 | 0.84 | 0.46 | 0 | 0.46 | Interleukin-2 | | 0.9585 | 0.026 | 1 |
| 2pg6 | 263 | 14 | 0.67 | 0.87 | 0.43 | 0.03 | 0.46 | Cytochrome P450 2A13 | | 2.9585 | 0.002 | 7 |
| 3iec | 189 | 11 | 0.58 | 0.85 | 0.44 | 0.02 | 0.46 | Serine/threonine-protein kinase MARK2 | | 0 | NA | 0 |
| 3adw | 76 | 12 | 0.67 | 0.86 | 0.46 | 0.01 | 0.47 | Peptostreptococcal albumin-binding protein | | 0 | NA | 0 |
| 1r80 | 39 | 16 | 0.66 | 0.83 | 0.46 | 0.01 | 0.47 | Histo-blood group ABO system transferase | | 0 | NA | 0 |
| 2z0n | | | 0.6 | 0.85 | 0.44 | 0.03 | 0.47 | DCC-interacting protein 13-alpha | | 0 | NA | 0 |
| 2gaq | | | 0.65 | 0.86 | 0.46 | 0.01 | 0.47 | Serine/threonine-protein kinasemTOR | | 2.2182 | 0.007 | 4 |

## Adenosine-5'-Diphosphate (1UC9)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2xf0 | 37 | 11 | 0.64 | 0.92 | 0.39 | 0.02 | 0.41 | Serine/threonine-protein kinase Chk1 | denosine Diphosphat | 0 | NA | 0 |
| 1gzn | 44 | 12 | 0.64 | 0.87 | 0.44 | 0.02 | 0.46 | RAC-beta serine/threonine-protein kinase | | 0.9944 | 0.005 | 1 |
| 3com | 84 | 13 | 0.68 | 0.89 | 0.43 | 0.03 | 0.46 | Serine/threonine-protein kinase 4 | | 0 | NA | 0 |
| 2h3n | 53 | 13 | 0.74 | 0.88 | 0.46 | 0.01 | 0.47 | unoglobulin lambda-like polypeptide 1, Immunoglobulin iota chain | | 0 | NA | 0 |
| 3jzq | 31 | 14 | 0.65 | 0.85 | 0.45 | 0.02 | 0.47 | Protein Mdm4 | | 0 | NA | 0 |
| 3be2 | 39 | 11 | 0.67 | 0.87 | 0.46 | 0.02 | 0.48 | Vascular endothelial growth factor receptor 2 | | 0.9944 | 0.005 | 1 |
| 2o7n | 26 | 13 | 0.59 | 0.84 | 0.45 | 0.04 | 0.49 | Integrin alpha-L | | 0 | NA | 0 |
| 2l6u | 16 | 11 | 0.66 | 0.85 | 0.48 | 0.01 | 0.49 | Mediator of RNA polymerase II transcription subunit 25 | | 0 | NA | 0 |
| 2jfa | 57 | 11 | 0.62 | 0.82 | 0.49 | 0.02 | 0.51 | Estrogen receptor | | 0.9944 | 0.005 | 1 |
| 1oj6 | 72 | 11 | 0.69 | 0.84 | 0.51 | 0 | 0.51 | Neuroglobin | | 0 | NA | 0 |

## Flavin-Adenine Dinucleotide (1FNB)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2omi | 39 | 12 | 0.64 | 0.88 | 0.42 | 0.01 | 0.43 | Insulin | Adenosine, Riboflavin | 2.9059 | 0.003 | 7 |
| 3f68 | 40 | 11 | 0.66 | 0.9 | 0.42 | 0.03 | 0.45 | Prothrombin | | 2.2278 | 0.007 | 4 |
| 1xwd | 127 | 11 | 0.64 | 0.85 | 0.47 | 0.02 | 0.49 | Glycoprotein hormones alpha chain | | 3.1573 | 0.008 | 10 |
| 3cjf | 33 | 11 | 0.63 | 0.84 | 0.47 | 0.02 | 0.49 | Vascular endothelial growth factor receptor 2 | | 2.2278 | 0.007 | 4 |
| 1x7r | 37 | 11 | 0.64 | 0.83 | 0.48 | 0.02 | 0.5 | Estrogen receptor | | 3.0758 | 0.003 | 8 |
| 1y8o | 63 | 12 | 0.74 | 0.88 | 0.47 | 0.04 | 0.51 | kinase isozyme 3, mitochondrial | | 0.8052 | 0.129 | 1 |
| 1xp0 | 48 | 14 | 0.72 | 0.83 | 0.51 | 0.01 | 0.52 | cGMP-specific 3',5'-cyclic phosphodiesterase | | 1.9059 | 0.011 | 3 |
| 3bv7 | 75 | 11 | 0.67 | 0.85 | 0.48 | 0.04 | 0.52 | 3-oxo-5-beta-steroid 4-dehydrogenase | | 0 | NA | 0 |
| 1lwy | 31 | 12 | 0.66 | 0.82 | 0.5 | 0.03 | 0.53 | N-glycosylase/DNA lyase | | 0.9059 | 0.069 | 1 |
| 2dkw | 21 | 11 | 0.51 | 0.78 | 0.5 | 0.03 | 0.53 | ATPase family AAA domain-containing protein 2B | | 0 | NA | 0 |
| 1e04 | | | 0.59 | 0.78 | 0.51 | 0.02 | 0.53 | Antithrombin-III | | 0.0869 | 0.079 | 1 |

## Citric Acid (1HTO)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2hby | 39 | 11 | 0.58 | 0.91 | 0.37 | 0.09 | 0.46 | Caspase-1 | Citric Acid | 0 | NA | 0 |
| 2dmz | 13 | 14 | 0.59 | 0.81 | 0.47 | 0 | 0.47 | InaD-like protein | | 0 | NA | 0 |
| 1f5q | 207 | 13 | 0.62 | 0.84 | 0.45 | 0.03 | 0.48 | Cyclin-dependent kinase 2 | | 0 | NA | 0 |
| 2stw | 33 | 11 | 0.62 | 0.84 | 0.47 | 0.02 | 0.49 | Protein C-ets-1 | | 0 | NA | 0 |
| 3m1n | 42 | 13 | 0.63 | 0.8 | 0.51 | 0 | 0.51 | Sonic hedgehog protein | | 0 | NA | 0 |
| 3l16 | 153 | 16 | 0.65 | 0.78 | 0.52 | 0 | 0.52 | Phosphatidylinositol 4,5-bisphosphate 3-kinase catalytic subunit gamma isoform | | 0 | NA | 0 |
| 2pgy | 45 | 11 | 0.65 | 0.81 | 0.52 | 0.01 | 0.53 | Histo-blood group ABO system transferase | | 0 | NA | 0 |
| 2igp | 12 | 11 | 0.63 | 0.79 | 0.53 | 0 | 0.53 | Kinetochore protein NDC80 homolog | | 0 | NA | 0 |
| 3knv | 15 | 13 | 0.72 | 0.81 | 0.53 | 0.01 | 0.54 | TNF receptor-associated factor 2 | | 0 | NA | 0 |
| 1ozs | 17 | 12 | 0.63 | 0.81 | 0.5 | 0.04 | 0.54 | Troponin I, cardiac muscle | | 0 | NA | 0 |
| 2oxw | | | 0.59 | 0.83 | 0.47 | 0.08 | 0.55 | Macrophage metalloelastase | | 0 | NA | 0 |

## 2'-Monophosphadenosine 5'-Diphosphoribose (1DJL)

| PDB | Pocket | Aligned | RMSD | Seq | Distance | Expression | Final | Gene Name | Terms | CI | P-value | Literature |
|---|---|---|---|---|---|---|---|---|---|---|---|---|
| 3d0a | 146 | 12 | 0.69 | 0.84 | 0.49 | 0.03 | 0.52 | Cellular tumor antigen p53 | NADPH | 3.9734 | <0.001 | 15 |
| 1em2 | 38 | 12 | 0.54 | 0.8 | 0.47 | 0.05 | 0.52 | StAR-related lipid transfer protein 3 | | 0 | NA | 0 |
| 2bxp | 72 | 13 | 0.6 | 0.81 | 0.48 | 0.04 | 0.52 | Serum albumin | | 2.5585 | 0.001 | 5 |
| 1je4 | 21 | 11 | 0.64 | 0.88 | 0.43 | 0.09 | 0.52 | C-C motif chemokine 4 | | 0.9734 | 0.02 | 1 |
| 1xuc | 36 | 11 | 0.64 | 0.81 | 0.52 | 0.01 | 0.53 | Collagenase 3 | | 1.5584 | 0.007 | 2 |
| 3mo0 | 75 | 16 | 0.62 | 0.8 | 0.49 | 0.04 | 0.53 | Histone-lysine N-methyltransferase EHMT1 | | 0 | NA | 0 |
| 1urw | 31 | 11 | 0.63 | 0.81 | 0.51 | 0.03 | 0.54 | Cyclin-dependent kinase 2 | | 0 | NA | 0 |
| 3elm | 31 | 14 | 0.62 | 0.77 | 0.53 | 0.01 | 0.54 | Collagenase 3 | | 1.5584 | 0.007 | 2 |
| 2q2z | 102 | 12 | 0.71 | 0.83 | 0.52 | 0.02 | 0.54 | Kinesin-like protein KIF11 | | 0 | NA | 0 |
| 3b8q | 56 | 12 | 0.61 | 0.79 | 0.52 | 0.02 | 0.54 | Vascular endothelial growth factor receptor 2 | | 1.9734 | 0.004 | 3 |

## Riboflavin Monophosphate (1SZF)

| PDB | Pocket | Aligned | RMSD | Seq | Distance | Expression | Final | Gene Name | CI | P-value | Literature |
|---|---|---|---|---|---|---|---|---|---|---|---|
| 2c2h | 25 | 11 | 0.63 | 0.83 | 0.49 | 0 | 0.49 | Ras-related C3 botulinum toxin substrate 3 | 0 | NA | 0 |
| 1y2a | 59 | 11 | 0.59 | 0.84 | 0.46 | 0.04 | 0.5 | Phospholipid scramblase 1 | 0 | NA | 0 |
| 1zxa | 13 | 12 | 0.76 | 0.86 | 0.5 | 0.01 | 0.51 | cGMP-dependent protein kinase 1 | 0 | NA | 0 |
| 2stw | 31 | 11 | 0.7 | 0.85 | 0.5 | 0.02 | 0.52 | Protein C-ets-1 | 0 | NA | 0 |
| 2qhm | 49 | 12 | 0.67 | 0.83 | 0.5 | 0.02 | 0.52 | Serine/threonine-protein kinase Chk1 | 0 | NA | 0 |
| 3jtc | 45 | 11 | 0.66 | 0.82 | 0.52 | 0.01 | 0.53 | Endothelial protein C receptor | 0 | NA | 0 |
| 2qgw | 67 | 13 | 0.55 | 0.76 | 0.52 | 0.02 | 0.54 | Estrogen Receptor | 0 | NA | 0 |
| 2ve7 | 151 | 14 | 0.62 | 0.76 | 0.54 | 0 | 0.54 | Kinetochore protein Spc25 | 0 | NA | 0 |
| 3ps6 | 151 | 11 | 0.51 | 0.75 | 0.54 | 0 | 0.54 | Phosphatidylinositol 4,5-bisphosphate 3-kinase catalytic subunit gamma isoform | 0 | NA | 0 |
| 1bnd | 29 | 11 | 0.78 | 0.85 | 0.53 | 0.01 | 0.54 | Neurotrophin-3 | 0 | NA | 0 |

## Phosphoaminophosphonic Acid-Adenylate Ester (1TQM)

| PDB | Pocket | Aligned | RMSD | Seq | Distance | Expression | Final | Gene Name | Terms | CI | P-value | Literature |
|---|---|---|---|---|---|---|---|---|---|---|---|---|
| 2o1g | 35 | 15 | 0.57 | 0.76 | 0.52 | 0.01 | 0.53 | Histo-blood group ABO system transferase | AMP-PNP | 0 | NA | 0 |
| 1xdt | 64 | 11 | 0.67 | 0.82 | 0.52 | 0.02 | 0.54 | Proheparin-binding EGF-like growth factor | | 0 | NA | 0 |
| 1dm2 | 24 | 11 | 0.69 | 0.84 | 0.51 | 0.03 | 0.54 | Cyclin-dependent kinase 2 | | 0 | NA | 0 |
| 3kpw | 77 | 11 | 0.68 | 0.81 | 0.54 | 0.02 | 0.56 | Phenylethanolamine N-methyltransferase | | 0 | NA | 0 |
| 2qa6 | 85 | 12 | 0.59 | 0.77 | 0.54 | 0.02 | 0.56 | Estrogen receptor, Nuclear receptor coactivator 2 | | 0 | NA | 0 |
| 2fl6 | 95 | 13 | 0.76 | 0.82 | 0.54 | 0.02 | 0.56 | Kinesin-like protein KIF11 | | 0.997 | 0.003 | 1 |
| 3d0e | 69 | 11 | 0.71 | 0.82 | 0.54 | 0.02 | 0.56 | RAC-beta serine/threonine-protein kinase | | 0.997 | 0.003 | 1 |
| 2nte | 53 | 13 | 0.64 | 0.76 | 0.56 | 0 | 0.56 | BRCA1-associated RING domain protein 1 | | 0 | NA | 0 |
| 3d4l | 186 | 11 | 0.68 | 0.8 | 0.55 | 0.02 | 0.57 | Dipeptidyl peptidase 4 | | 0 | NA | 0 |
| 1olg | 24 | 11 | 0.6 | 0.77 | 0.55 | 0.03 | 0.58 | Cellular tumor antigen p53 | | 0 | NA | 0 |

**SI Table 8**

| Negative Dataset | | | | | |
|---|---|---|---|---|---|
| **IDs** | **length** | **Exptl.** | **resolution** | **R-factor** | **FreeRvalue** |
| 1TTZ | 87 | XRAY | 2.11 | 0.19 | 0.21 |
| 1USD | 45 | XRAY | 1.7 | 0.21 | 0.22 |
| 1DF4 | 68 | XRAY | 1.45 | 0.2 | 0.24 |
| 2MLT | 54 | XRAY | 2 | 0.2 | 1 |
| 2WFV | 48 | XRAY | 1.85 | 0.2 | 0.23 |
| 1X8Y | 86 | XRAY | 2.2 | 0.28 | 0.3 |
| 3JQH | 170 | XRAY | 2.2 | 0.2 | 0.22 |
| 1ONJ | 61 | XRAY | 1.56 | 0.17 | 0.19 |

(continued)

| IDs | length | Exptl. | resolution | R-factor | FreeRvalue |
|---|---|---|---|---|---|
| 3DMW | 126 | XRAY | 2.3 | 0.25 | 0.27 |
| 1UJ0 | 62 | XRAY | 1.7 | 0.22 | 0.23 |
| 1K51 | 72 | XRAY | 1.8 | 0.19 | 0.21 |
| 3E21 | 45 | XRAY | 1.73 | 0.22 | 0.22 |
| 1Q8H | 49 | XRAY | 2 | 0.27 | 0.28 |
| 1ZX6 | 58 | XRAY | 1.6 | 0.18 | 0.22 |
| 2UUX | 55 | XRAY | 1.4 | 0.17 | 0.22 |
| 2IJI | 63 | XRAY | 2.3 | 0.24 | 0.29 |
| 1YPA | 64 | XRAY | 2 | 0.18 | 1 |
| 1I1N | 226 | XRAY | 1.5 | 0.18 | 0.21 |
| 2OO2 | 86 | XRAY | 1.8 | 0.24 | 0.29 |
| 3FDT | 74 | XRAY | 2 | 0.2 | 0.26 |
| 1I6B | 689 | XRAY | 3.2 | 0.23 | 0.29 |
| 2DS7 | 51 | XRAY | 2.5 | 0.24 | 0.27 |
| 3L37 | 47 | XRAY | 1.45 | 0.24 | 0.26 |
| 1MN4 | 282 | XRAY | 2.2 | 0.23 | 0.27 |
| 1Q7D | 69 | XRAY | 1.8 | 0.24 | 0.27 |
| 1SMU | 54 | XRAY | 1.43 | 0.19 | 0.21 |
| 2CMP | 63 | XRAY | 1.58 | 0.23 | 0.28 |
| 1LB5 | 160 | XRAY | 2.4 | 0.21 | 0.24 |
| 1KS9 | 291 | XRAY | 1.7 | 0.23 | 0.29 |
| 2OQQ | 84 | XRAY | 2 | 0.24 | 0.3 |
| 3CP0 | 82 | XRAY | 1.65 | 0.17 | 0.22 |
| 1IXM | 384 | XRAY | 2.6 | 0.22 | 0.31 |
| 1T6F | 74 | XRAY | 1.47 | 0.18 | 0.22 |
| 1P9G | 41 | XRAY | 0.84 | 0.07 | 0.08 |
| 2GI9 | 56 | XRAY | 1.14 | 0.19 | 0.18 |
| 1LOE | 466 | XRAY | 1.9 | 0.18 | 1 |
| 1QM7 | 61 | XRAY | 2.1 | 0.2 | 0.22 |
| 1KGG | 258 | XRAY | 2.3 | 0.18 | 0.26 |
| 1J8E | 44 | XRAY | 1.85 | 0.19 | 0.22 |
| 1SSH | 60 | XRAY | 1.4 | 0.17 | 0.2 |
| 1MJN | 179 | XRAY | 1.3 | 0.16 | 0.2 |
| 2W6B | 56 | XRAY | 2.8 | 0.28 | 0.3 |
| 1LF1 | 308 | XRAY | 1.7 | 0.19 | 0.22 |
| 1J73 | 102 | XRAY | 2 | 0.2 | 0.27 |
| 1YZM | 51 | XRAY | 1.5 | 0.21 | 0.2 |
| 1K0F | 277 | XRAY | 2.5 | 0.21 | 0.25 |

(continued)

| IDs | length | Exptl. | resolution | R-factor | FreeRvalue |
|---|---|---|---|---|---|
| 1Z96 | 80 | XRAY | 1.8 | 0.17 | 0.2 |
| 1OAI | 59 | XRAY | 1 | 0.15 | 0.16 |
| 1MYL | 318 | XRAY | 2.4 | 0.21 | 0.29 |
| 3HRO | 44 | XRAY | 1.9 | 0.22 | 0.23 |
| 1YK4 | 52 | XRAY | 0.69 | 0.1 | 0.11 |
| 1B7I | 66 | XRAY | 1.65 | 0.22 | 0.24 |
| 3LE4 | 79 | XRAY | 1.7 | 0.18 | 0.21 |
| 3JSC | 105 | XRAY | 1.5 | 0.17 | 0.2 |
| 3NGP | 62 | XRAY | 1.08 | 0.17 | 0.18 |
| 1KFN | 56 | XRAY | 1.65 | 0.24 | 0.27 |
| 1WKX | 43 | XRAY | 1.7 | 0.16 | 0.19 |
| 2XF6 | 51 | XRAY | 2.12 | 0.21 | 0.23 |
| 1VBV | 105 | XRAY | 2.7 | 0.23 | 0.29 |
| 2PNE | 81 | XRAY | 0.98 | 0.14 | 0.16 |
| 3P46 | 60 | XRAY | 1.7 | 0.2 | 0.24 |
| 1T3J | 96 | XRAY | 2.5 | 0.24 | 0.29 |
| 3IUF | 48 | XRAY | 1.8 | 0.2 | 0.24 |

## Claims

**1.** A method for identifying a ligand binding site of a protein, the method comprising the steps of:

(a) generating stochastic basis sets of values representative of putative ligand-protein binding sites by (1) identifying information in a protein structure database, the information corresponding to known protein signature pockets and representing at least protein signature pocket atomic coordinates and conformation, and (2) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and known protein signature pockets;
(b) generating basis sets of values representative of nucleotide expression levels of genes that have been correlated to known ligand-protein binding sites in a predetermined number of biological samples by mapping known ligand-protein binding site information to a gene name and identifying in a gene-biological sample expression database information representative of nucleotide expression levels corresponding to the gene;
(c) selecting overlapping values from the basis sets generated in steps (a) and (b) as information which correlates to at least the atomic coordinates and conformation of one or more ligand-protein binding sites; and
(d) validating the one or more ligand-protein binding sites determined in step (c) *in vitro* by measuring the affinity of the ligand for the one or more ligand-protein binding sites.

**2.** The method of claim 1, wherein the biological samples are tissue samples, the nucleotide is mRNA and:

(1) the distance function defined by the structural similarity and likely sequence similarity of putative ligand-protein binding sites and known protein signature pockets is represented by the formula:

$$Score = Structural\ similarity + \alpha * (1\text{-}Sequence\ similarity)$$

$$Structural\ similarity = \frac{RMSD}{N^{1/3}}$$

$$Sequence\ similarity = \frac{Sequence\ score}{Best\ sequence\ score}$$

$$Sequence\ score = \sum_i (AtomFreq_i + ResFreq_i)$$

$$Best\ sequence\ score = \sum_i (MaxAtomFreq_i + MaxResFreq_i)$$

where $\alpha$ is 1.2, *RMSD* is the root mean square distance after the alignment, *N* is the number of positions aligned, *AtomFreqi* / *ResFreqi* is the frequency of aligned atom/residue at position *i*, and *MaxAtomFreqi* / *MoxResFreqi* is the highest frequency of any atom/residue at position *i* and the summation is over all the aligned positions; and
(2) the distance function defined by the structural similarity, likely sequence similarity of putative ligand-protein binding sites and known protein signature pockets and relative tissue mRNA expression levels in the predetermined number of tissues is represented by the formula:

$$Score = Structural\ similarity + \alpha^*(1\text{-}Sequence\ similarity) + \beta^*Tissue\ expression$$

$$Tissue\ expression = 1\text{-}\frac{Number\ of\ tissues\ with\ matching\ expression}{Total\ number\ of\ tissues}$$

wherein $\alpha$ and the structural similarity term and sequence similarity term are as defined above and $\beta$ is a value between 0.1 and 0.9.

3. The method of claims 1 or 2, wherein the protein structure database is the $CAST_p$ database.

4. The method of claims 1-3, wherein between about 20 to about 40 apo structures per ligand are used to generate stochastic basis sets of values representative of putative ligand-protein binding sites.

5. The method of claims 1-4, wherein known protein signature pockets are determined by identifying the three largest pockets of the first chain of the protein's three-dimensional structure.

6. The method of claims 1-5, wherein the number of atoms in the putative ligand-protein binding sites is between about 50 to about 100.

7. The method of claims 1-6, wherein the biological samples are tissue samples, the nucleotide is mRNA, mRNA expression levels of genes that have been correlated to known ligand-protein binding sites in a predetermined number of tissues are average values and the known ligand-protein binding sites are mapped to more than one gene.

8. The method of claim 7, wherein average mRNA expression levels are determined using Uniprot ID mapping.

9. The method of claims 1-8, wherein the affinity of the ligand for the one or more ligand-protein binding sites is measured using fluorescence anisotropy.

10. The method of claims 1-9, wherein the ligand is selected from the group consisting of a small molecule or a nucleic acid.

11. The method of claims 1-10, wherein at least one step is done *in silico.*

**12.** A computer system for identifying a ligand binding site of a protein comprising a processor, a memory and a non-transitory computer-readable storage medium that stores instructions that, when executed by the processor, cause the system to:

(a) generate stochastic basis sets of values representative of putative ligand-protein binding sites by (1) identifying information in a protein structure database, the information corresponding to known protein signature pockets and representing at least protein signature pocket atomic coordinates and conformation, and (2) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and known protein signature pockets;
(b) generate basis sets of values representative of nucleotide expression levels of genes that have been correlated to known ligand-protein binding sites in a predetermined number of biological samples (preferably tissue or cell samples) by mapping known ligand-protein binding site information to a gene name and identifying in a gene- biological sample expression database information representative of nucleotide expression levels corresponding to the gene; and
(c) select overlapping values from the basis sets generated in steps (a) and (b) as information which correlates to at least the atomic coordinates and conformation of one or more ligand-protein binding sites.

**13.** A method for identifying protein binding sites for a novel ligand, the method comprising the steps of:

(a) evaluating the binding of the novel ligand to one or more known protein binding sites, selecting protein binding sites which bind the novel ligand within a specified selectivity and identifying information representing at least the atomic coordinates and conformation of protein signature pockets which bind the ligand within a specified selectivity;
(b) generating stochastic basis sets of values representative of putative ligand-protein binding sites by performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and identified protein signature pockets;
(c) generating basis sets of values representative of nucleotide expression levels of genes that have been correlated to known ligand-protein binding sites in a predetermined number of biological samples by mapping known ligand-protein binding site information to a gene name and identifying in a gene-biological sample expression database information representative of nucleotide expression levels corresponding to the gene;
(d) selecting overlapping values from the basis sets generated in steps (b) and (c) as information which correlates to at least the atomic coordinates and conformation of one or more ligand-protein binding sites; and
(e) validating the one or more ligand-protein binding sites determined in step (d) *in vitro* by measuring the affinity of the ligand for the one or more ligand-protein binding sites.

**14.** The method of claim 13, wherein the nucleotide is mRNA, the biological samples are tissue samples and:

(1) the distance function defined by the structural similarity and likely sequence similarity of putative ligand-protein binding sites and identified protein signature pockets is represented by the formula:

$$Score = Structural\ similarity + \alpha * (1\text{-}Sequence\ similarity)$$

$$Structural\ similarity = \frac{RMSD}{N^{1/3}}$$

$$Sequence\ similarity = \frac{Sequence\ score}{Best\ sequence\ score}$$

$$Sequence\ score = \sum_i (AtomFreq_i + ResFreq_i)$$

$$Best\ sequence\ score = \sum_i (MaxAtomFreq_i + MaxResFreq_i)$$

where α is 1.2, *RMSD* is the root mean square distance after the alignment, *N* is the number of positions aligned, *AtomFreqi* / *ResFreqi* is the frequency of aligned atom/residue at position *i*, and *MaxAtomFreqi* / *MoxResFreqi* is the highest frequency of any atom/residue at position *i* and the summation is over all the aligned positions; and (2) the distance function defined by the structural similarity, likely sequence similarity of putative ligand-protein binding sites and identified protein signature pockets and relative tissue mRNA expression levels in the predetermined number of tissues is represented by the formula:

$$Score = Structural\ similarity + \alpha^*(1\text{-}Sequence\ similarity) + \beta^*Tissue\ expression$$

$$Tissue\ expression = 1\text{-}\frac{Number\ of\ tissues\ with\ matching\ expression}{Total\ number\ of\ tissues}$$

wherein α and the structural similarity term and sequence similarity term are as defined above and β is a value between 0.1 and 0.9.

**15.** A network system for identifying a ligand binding site of a protein, the network system comprising:

(a) computer system comprising a processor, a memory and a non-transitory computer-readable storage medium that stores instructions that, when executed by the processor, cause the system to:

(1) generate stochastic basis sets of values representative of putative ligand-protein binding sites by (i) identifying information in a protein structure database, the information corresponding to known protein signature pockets and representing at least protein signature pocket atomic coordinates and conformation, and (ii) performing pair-wise sequence order-independent structure alignment, divisive hierarchical clustering, and probabilistic sequence similarity operations on the identified protein signature pocket information to select the stochastic basis sets of values representative of putative ligand-protein binding sites, the probabilistic sequence similarity operation including solution of a distance function defined by the structural similarity and likely sequence similarity of putative ligand-protein binding sites and known protein signature pockets;
(2) generate basis sets of values representative of nucleotide expression levels of genes that have been correlated to known ligand-protein binding sites in a predetermined number of biological samples (preferably tissue or cell samples) by mapping known ligand-protein binding site information to a gene name and identifying in a gene- biological sample expression database information representative of nucleotide expression levels corresponding to the gene; and
(3) select overlapping values from the basis sets generated in steps (1) and (2) as information which correlates to at least the atomic coordinates and conformation of one or more ligand-protein binding sites; and

(b) a high-throughput assay which is networked to the computer system for the transmission of information representative of validated ligand-protein binding sites determined by measuring the affinity of the ligand for the predicted ligand-protein binding sites.

**Patentansprüche**

**1.** Verfahren zum Identifizieren einer Ligandbindungsstelle eines Proteins, wobei das Verfahren die Schritte umfasst:

(a) Erzeugen von stochastischen Basissätzen von Werten, die für putative Ligand-Protein-Bindungsstellen repräsentativ sind, durch (1) Identifizieren von Informationen in einer Proteinstrukturdatenbank, wobei die Informationen bekannten Proteinsignaturbindetaschen entsprechen und zumindest Proteinsignaturbindetaschen-Atomkoordinaten und - konformation darstellen, und (2) Durchführen von Operationen einer paarweisen sequenzreihenfolgenunabhängigen Strukturalignierung, eines teilenden hierarchischen Clusterings und probalistischer Sequenzähnlichkeit an den identifizierten Proteinsignaturbindetascheninformationen, um die stochastischen Basissätze von Werten auszuwählen, die für putative Ligand-Protein-Bindungsstellen repräsentativ sind, wobei die probalistische Sequenzähnlichkeitsoperation eine Lösung einer Distanzfunktion beinhaltet, die durch die Strukturähnlichkeit und wahrscheinliche Sequenzähnlichkeit von putativen Ligand-Protein-Bindungsstellen und bekannten Proteinsignaturbindetaschen definiert ist;

(b) Erzeugen von Basissätzen von Werten, die für Nukleotidexpressionsspiegel von Genen repräsentativ sind, die mit bekannten Ligand-Protein-Bindungsstellen in einer vorbestimmten Anzahl biologischer Proben korreliert wurden, durch Kartieren bekannter Informationen zu Ligand-Protein-Bindungsstellen zu einem Gennamen und Identifizieren von Informationen, die für die dem Gen entsprechenden Nukleotidexpressionsspiegel repräsentativ sind, in einer Datenbank zur Expression von biologischen Proben durch Gene;

(c) Auswählen von überlappenden Werten aus den in den Schritten (a) und (b) erzeugten Basissätzen als Informationen, die mit zumindest den Atomkoordinaten und der Konformation einer oder mehrerer Ligand-Protein-Bindungsstellen korrelieren; und

(d) Validieren der einen oder mehreren Ligand-Protein-Bindungsstellen, die in Schritt (c) bestimmt wurden, *in vitro* durch Messen der Affinität des Liganden für die eine oder mehreren Ligand-Protein-Bindungsstellen.

**2.** Verfahren nach Anspruch 1, wobei die biologischen Proben Gewebeproben sind, das Nukleotid mRNA ist und:

(1) die Distanzfunktion, die durch die Strukturähnlichkeit und wahrscheinliche Sequenzähnlichkeit putativer Ligand-Protein-Bindungsstellen und bekannte Proteinsignaturbindetaschen definiert ist, durch die Formel dargestellt ist:

$$Score = Strukturähnlichkeit + \alpha * (1 - Sequenzähnlichkeit)$$

$$Strukturähnlichkeit = RMSD / N^{1/3}$$

$$Sequenzähnlichkeit = \frac{Sequenz\text{-}Score}{Bester\ Sequenz\text{-}Score}$$

$$Sequenz\text{-}Score = \sum_i (Atomhäuf._i + \mathrm{Rest}häuf._i)$$

$$Bester\ Sequenz\text{-}Score = \sum_i (Max.\ Atomhäuf._i + Max.\ \mathrm{Rest}häuf._i)$$

wobei $\alpha$ 1,2 ist, *RMSD* das quadratische Mittel der Distanz nach Alignierung ist, *N* die Anzahl von alignierten Positionen ist, $Atomhäuf._i$ / $Resthäuf._i$ die Häufigkeit alignierten Atoms/Rests an Position *i* ist und $Max.\ Atomhäuf._i$ / $Max\ Resthäuf._i$ die höchste Häufigkeit eines beliebigen Atoms/Rests an Position *i* ist und die Summierung über alle alignierten Positionen erfolgt; und

(2) die Distanzfunktion, die durch die Strukturähnlichkeit und wahrscheinliche Sequenzähnlichkeit putativer Ligand-Protein-Bindungsstellen und bekannter Proteinsignaturbindetaschen und relative Gewebe-mRNA-Expressionshöhen in der vorbestimmten Anzahl von Geweben definiert ist, durch die Formel dargestellt ist:

$$Score = Strukturähnlichkeit + \alpha * (1 - Sequenzähnlichkeit) + \beta * Gewebeexpression$$

$$Gewebeexpression = 1 - \frac{Anzahl\ von\ Geweben\ mit\ übereinstimmender\ Expression}{Gesamtanzahl\ von\ Geweben}$$

wobei $\alpha$ und der Strukturähnlichkeitsterm und der Sequenzähnlichkeitsterm wie oben definiert sind und $\beta$ ein Wert zwischen 0,1 und 0,9 ist.

**3.** Verfahren nach Anspruch 1 oder 2, wobei die Proteinstrukturdatenbank die $CAST_p$-Datenbank ist.

**4.** Verfahren nach den Ansprüchen 1 bis 3, wobei zwischen etwa 20 und etwa 40 Apo-Strukturen pro Ligand verwendet werden, um stochastische Basissätzen von Werten zu erzeugen, die für putative Ligand-Protein-Bindungsstellen repräsentativ sind.

**5.** Verfahren nach den Ansprüchen 1 bis 4, wobei bekannte Proteinsignaturbindetaschen durch Identifizieren der drei größten Bindetaschen der ersten Kette der dreidimensionalen Struktur des Proteins bestimmt werden.

**6.** Verfahren nach den Ansprüchen 1 bis 5, wobei die Anzahl von Atomen in den putativen Ligand-Protein-Bindungsstellen zwischen etwa 50 und etwa 100 liegt.

**7.** Verfahren nach den Ansprüchen 1 bis 6, wobei die biologischen Proben Gewebeproben sind, das Nukleotid mRNA ist, mRNA-Expressionshöhen von Genen, die mit bekannten Ligand-Protein-Bindungsstellen in einer vorbestimmten Anzahl von Geweben korreliert wurden, Durchschnittswerte sind und die bekannten Ligand-Protein-Bindungsstellen mehr als einem Gen kartiert werden.

**8.** Verfahren nach Anspruch 7, wobei die durchschnittlichen mRNA-Expressionshöhen mittels Uniprot-ID-Kartierung bestimmt werden.

**9.** Verfahren nach den Ansprüchen 1 bis 8, wobei die Affinität des Liganden für die eine oder mehreren Ligand-Protein-Bindungsstellen mittels Fluoreszenzanisotropie gemessen wird.

**10.** Verfahren nach den Ansprüchen 1 bis 9, wobei der Ligand aus der Gruppe ausgewählt ist, bestehend aus kleinem Molekül oder einer Nukleinsäure.

**11.** Verfahren nach den Ansprüchen 1 bis 10, wobei zumindest ein Schritt *in silico* erfolgt.

**12.** Computersystem zum Identifizieren einer Ligandbindungsstelle eines Proteins, umfassend einen Prozessor, einen Speicher und ein nichtflüchtiges computerlesbares Speichermedium, auf dem Anweisungen gespeichert sind, die, wenn sie von dem Prozessor ausgeführt werden, das System veranlassen zum:

(a) Erzeugen von stochastischen Basissätzen von Werten, die für putative Ligand-Protein-Bindungsstellen repräsentativ sind, durch (1) Identifizieren von Informationen in einer Proteinstrukturdatenbank, wobei die Informationen bekannten Proteinsignaturbindetaschen entsprechen und zumindest Proteinsignaturbindetaschen-Atomkoordinaten und - konformation darstellen, und (2) Durchführen von Operationen einer paarweisen sequenzreihenfolgenunabhängigen Strukturalignierung, eines teilenden hierarchischen Clusterings und probalistischer Sequenzähnlichkeit an den identifizierten Proteinsignaturbindetascheninformationen, um die stochastischen Basissätze von Werten auszuwählen, die für putative Ligand-Protein-Bindungsstellen repräsentativ sind, wobei die probalistische Sequenzähnlichkeitsoperation eine Lösung einer Distanzfunktion beinhaltet, die durch die Strukturähnlichkeit und wahrscheinliche Sequenzähnlichkeit von putativen Ligand-Protein-Bindungsstellen und bekannten Proteinsignaturbindetaschen definiert ist;
(b) Erzeugen von Basissätzen von Werten, die für Nukleotidexpressionsspiegel von Genen repräsentativ sind, die mit bekannten Ligand-Protein-Bindungsstellen in einer vorbestimmten Anzahl biologischer Proben (vorzugsweise Gewebe- oder Zellproben) korreliert wurden, durch Kartieren bekannter Informationen zu Ligand-Protein-Bindungsstellen zu einem Gennamen und Identifizieren von Informationen, die für die dem Gen entsprechenden Nukleotidexpressionsspiegel repräsentativ sind, in einer Datenbank zur Expression von biologischen Proben durch Gene; und
(c) Auswählen von überlappenden Werten aus den in den Schritten (a) und (b) erzeugten Basissätzen als Informationen, die mit zumindest den Atomkoordinaten und der Konformation einer oder mehrerer Ligand-Protein-Bindungsstellen korrelieren.

**13.** Verfahren zum Identifizieren von Proteinbindungsstellen für einen neuen Liganden, wobei das Verfahren die Schritte umfasst:

(a) Evaluieren der Bindung des neuen Liganden an eine oder mehrere bekannte Proteinbindungsstellen, Auswählen der Proteinbindungsstellen, die den neuen Liganden innerhalb einer spezifizierten Selektivität binden, und Identifizieren von Informationen, die zumindest die Atomkoordinaten und Konformation von Proteinsignaturbindetaschen repräsentieren, die den Liganden innerhalb einer spezifizierten Selektivität binden;

(b) Erzeugen von stochastischen Basissätzen von Werten, die für putative Ligand-Protein-Bindungsstellen repräsentativ sind, durch Durchführen von Operationen einer paarweisen sequenzreihenfolgenunabhängigen Strukturalignierung, eines teilenden hierarchischen Clusterings und probalistischer Sequenzähnlichkeit an den identifizierten Proteinsignaturbindetascheninformationen, um die stochastischen Basissätze von Werten auszuwählen, die für putative Ligand-Protein-Bindungsstellen repräsentativ sind, wobei die probalistische Sequenzähnlichkeitsoperation eine Lösung einer Distanzfunktion beinhaltet, die durch die Strukturähnlichkeit und wahrscheinliche Sequenzähnlichkeit von putativen Ligand-Protein-Bindungsstellen und identifizierten Proteinsignaturbindetaschen definiert ist;

(c) Erzeugen von Basissätzen von Werten, die für Nukleotidexpressionsspiegel von Genen repräsentativ sind, die mit bekannten Ligand-Protein-Bindungsstellen in einer vorbestimmten Anzahl biologischer Proben korreliert wurden, durch Kartieren bekannter Informationen zu Ligand-Protein-Bindungsstellen zu einem Gennamen und Identifizieren von Informationen, die für die dem Gen entsprechenden Nukleotidexpressionsspiegel repräsentativ sind, in einer Datenbank zur Expression von biologischen Proben durch Gene;

(d) Auswählen von überlappenden Werten aus den in den Schritten (a) und (b) erzeugten Basissätzen als Informationen, die mit zumindest den Atomkoordinaten und der Konformation einer oder mehrerer Ligand-Protein-Bindungsstellen korrelieren.

(e) Validieren der einen oder mehreren Ligand-Protein-Bindungsstellen, die in Schritt (d) bestimmt wurden, *in vitro* durch Messen der Affinität des Liganden für die eine oder mehreren Ligand-Protein-Bindungsstellen.

**14.** Verfahren nach Anspruch 13, wobei das Nukleotid mRNA ist, die biologischen Proben Gewebeproben sind und:

(1) die Distanzfunktion, die durch die Strukturähnlichkeit und wahrscheinliche Sequenzähnlichkeit putativer Ligand-Protein-Bindungsstellen und identifizierte Proteinsignaturbindetaschen definiert ist, durch die Formel dargestellt ist:

$$\textit{Score} = \textit{Strukturähnlichkeit} + \alpha * (1 - \textit{Sequenzähnlichkeit})$$

$$\textit{Strukturähnlichkeit} = \textit{RMSD} / N^{1/3}$$

$$\textit{Sequenzähnlichkeit} = \frac{\textit{Sequenz-Score}}{\textit{Bester Sequenz-Score}}$$

$$\textit{Sequenz-Score} = \sum_i(\textit{Atomhäuf.}_i + \text{Rest}\textit{häuf.}_i)$$

$$\textit{Bester Sequenz-Score} = \sum_i(\textit{Max. Atomhäuf.}_i + \textit{Max.}\ \text{Rest}\textit{häuf.}_i)$$

wobei $\alpha$ 1,2 ist, *RMSD* das quadratische Mittel der Distanz nach Alignierung ist, *N* die Anzahl von alignierten Positionen ist, $\textit{Atomhäuf.}_i$ / $\textit{Resthäuf.}_i$ die Häufigkeit alignierten Atoms/Rests an Position *i* ist und $\textit{Max. Atomhäuf.}_i$ / $\textit{Max Resthäuf.}_i$ die höchste Häufigkeit eines beliebigen Atoms/Rests an Position *i* ist und die Summierung über alle alignierten Positionen erfolgt; und

(2) die Distanzfunktion, die durch die Strukturähnlichkeit und wahrscheinliche Sequenzähnlichkeit putativer Ligand-Protein-Bindungsstellen und identifizierte Proteinsignaturbindetaschen und relative Gewebe-mRNA-Expressionshöhen in der vorbestimmten Anzahl von Geweben definiert ist, durch die Formel dargestellt ist:

$$\textit{Score} = \textit{Strukturähnlichkeit} + \alpha * (1 - \textit{Sequenzähnlichkeit}) + \beta * \textit{Gewebeexpression}$$

$$\textit{Gewebeexpression} = 1 - \frac{\textit{Anzahl von Geweben mit übereinstimmender Expression}}{\textit{Gesamtanzahl von Geweben}}$$

wobei $\alpha$ und der Strukturähnlichkeitsterm und der Sequenzähnlichkeitsterm wie oben definiert sind und $\beta$ ein Wert zwischen 0,1 und 0,9 ist.

**15.** Netzwerksystem zum Identifizieren einer Ligandbindungsstelle eines Proteins, wobei das Netzwerksystem umfasst:

(a) ein Computersystem, umfassend einen Prozessor, einen Speicher und ein nichtflüchtiges computerlesbares Speichermedium, auf dem Anweisungen gespeichert sind, die, wenn sie von dem Prozessor ausgeführt werden, das System veranlassen zum:

(1) Erzeugen von stochastischen Basissätzen von Werten, die für putative Ligand-Protein-Bindungsstellen repräsentativ sind, durch (i) Identifizieren von Informationen in einer Proteinstrukturdatenbank, wobei die Informationen bekannten Proteinsignaturbindetaschen entsprechen und zumindest Proteinsignaturbindetaschen-Atomkoordinaten und - konformation darstellen, und (ii) Durchführen von Operationen einer paarweisen sequenzreihenfolgenunabhängigen Strukturalignierung, eines teilenden hierarchischen Clusterings und probalistischer Sequenzähnlichkeit an den identifizierten Proteinsignaturbindetascheninformationen, um die stochastischen Basissätze von Werten auszuwählen, die für putative Ligand-Protein-Bindungsstellen repräsentativ sind, wobei die probalistische Sequenzähnlichkeitsoperation eine Lösung einer Distanzfunktion beinhaltet, die durch die Strukturähnlichkeit und wahrscheinliche Sequenzähnlichkeit von putativen Ligand-Protein-Bindungsstellen und bekannten Proteinsignaturbindetaschen definiert ist;
(2) Erzeugen von Basissätzen von Werten, die für Nukleotidexpressionsspiegel von Genen repräsentativ sind, die mit bekannten Ligand-Protein-Bindungsstellen in einer vorbestimmten Anzahl biologischer Proben (vorzugsweise Gewebe- oder Zellproben) korreliert wurden, durch Kartieren bekannter Informationen zu Ligand-Protein-Bindungsstellen zu einem Gennamen und Identifizieren von Informationen, die für die dem Gen entsprechenden Nukleotidexpressionsspiegel repräsentativ sind, in einer Datenbank zur Expression von biologischen Proben durch Gene; und
(3) Auswählen von überlappenden Werten aus den in den Schritten (1) und (2) erzeugten Basissätzen als Informationen, die mit zumindest den Atomkoordinaten und der Konformation einer oder mehrerer Ligand-Protein-Bindungsstellen korrelieren; und

(b) einen Hochdurchsatzassay, der mit dem Computersystem vernetzt ist, um Informationen zu übertragen, die für validierte Ligand-Protein-Bindungsstellen repräsentativ sind, die durch Messen der Affinität des Liganden für die prognostizierten Ligand-Protein-Bindungsstellen bestimmt wurden.

## Revendications

**1.** Procédé d'identification d'un site de liaison de ligand d'une protéine, le procédé comprenant les étapes de :

(a) génération d'ensembles de base stochastiques de valeurs représentatives de sites de liaison ligand-protéine putatifs par (1) identification d'informations dans une base de données de structures de protéine, les informations correspondant à des poches de signature protéique connues et représentant au moins des coordonnées atomiques et une conformation de poche de signature protéique, et (2) conduite d'opérations d'alignement apparié de structures indépendant de l'ordre de séquence, de regroupement hiérarchique par division, et de similarité de séquence probabilistique sur les informations de poche de signature protéique identifiées pour sélectionner les ensembles de base stochastiques de valeurs représentatives de sites de liaison ligand-protéine putatifs, l'opération de similarité de séquence probabilistique comprenant la résolution d'une fonction de la distance définie par la similarité structurale et la similarité de séquence probable de sites de liaison ligand-protéine putatifs et de poches de signature protéique connues ;
(b) génération d'ensembles de base de valeurs représentatives de taux d'expression nucléotidique de gènes qui ont été corrélés à des sites de liaison ligand-protéine connus dans un nombre prédéterminé d'échantillons biologiques par mise en correspondance d'informations de site de liaison ligand-protéine connu avec un nom de gène et identification dans une base de données d'expression génique d'échantillon biologique d'informations représentatives de taux d'expression nucléotidique correspondant au gène ;
(c) sélection de valeurs de chevauchement à partir des ensembles de base générés dans les étapes (a) et (b) en tant qu'informations qui corrèlent au moins les coordonnées atomiques et la conformation d'un ou plusieurs sites de liaison ligand-protéine ; et
(d) validation des un ou plusieurs sites de liaison ligand-protéine déterminés dans l'étape (c) *in vitro* par mesure de l'affinité du ligand pour les un ou plusieurs sites de liaison ligand-protéine.

**2.** Procédé selon la revendication 1, dans lequel les échantillons biologiques sont des échantillons de tissu, le nucléotide est un ARNm et :

(1) la fonction de la distance définie par la similarité structurale et la similarité de séquence probable de sites de liaison ligand-protéine putatifs et de poches de signature protéique connues est représentée par la formule :

$$Score = Similarit\acute{e}\ structurale + \alpha^*(1\text{-}Similarit\acute{e}\ de\ s\acute{e}quence)$$

$$Similarit\acute{e}\ structurale = \frac{RMSD}{N^{1/3}}$$

$$Similarit\acute{e}\ de\ s\acute{e}quence = \frac{Score\ de\ s\acute{e}quence}{Meilleur\ score\ de\ s\acute{e}quence}$$

$$Score\ de\ s\acute{e}quence = \sum_i (AtomFreq_i + ResFreq_i)$$

$$Meilleur\ score\ de\ s\acute{e}quence = \sum_i (MaxAtomFreq_i + MaxResFreq_i)$$

où $\alpha$ est 1,2, *RMSD* est la distance quadratique moyenne après l'alignement, *N* est le nombre de positions alignées, $AtomFreq_i$ / $ResFreq_i$ est la fréquence d'atome/résidu aligné à la position *i*, et $MaxAtomFreq_i$ / $MaxResFreq_i$ est la fréquence la plus élevée d'un atome/résidu quelconque à la position *i* et la sommation est effectuée sur toutes les positions alignées ; et

(2) la fonction de la distance définie par la similarité structurale, la similarité de séquence probable de sites de liaison ligand-protéine putatifs et de poches de signature protéique identifiées et les taux d'expression d'ARNm tissulaires relatifs dans le nombre prédéterminé de tissus est représentée par la formule :

$$Score = Similarit\acute{e}\ structurale + \alpha^*(1\text{-}Similarit\acute{e}\ de\ s\acute{e}quence + \beta^*Expression\ tissulaire)$$

$$Expression\ tissulaire = 1 - \frac{Nombre\ de\ tissus\ avec\ expression\ correspondante}{Nombre\ total\ de\ tissus}$$

où $\alpha$ et le terme de similarité structurale et le terme de similarité de séquence sont tels que définis ci-dessus et $\beta$ est une valeur comprise entre 0,1 et 0,9.

**3.** Procédé selon les revendications 1 ou 2, dans lequel la base de données de structures de protéine est la base de données $CAST_p$.

**4.** Procédé selon les revendications 1 à 3, dans lequel entre environ 20 et environ 40 structures apo par ligand sont utilisées pour générer des ensembles de base stochastiques de valeurs représentatives de sites de liaison ligand-protéine putatifs.

**5.** Procédé selon les revendications 1 à 4, dans lequel des poches de signature protéique connues sont déterminées par identification des trois plus grandes poches de la première chaîne de la structure tridimensionnelle d'une protéine.

**6.** Procédé selon les revendications 1 à 5, dans lequel le nombre d'atomes dans les sites de liaison ligand-protéine putatifs est compris entre environ 50 et environ 100.

**7.** Procédé selon les revendications 1 à 6, dans lequel les échantillons biologiques sont des échantillons de tissu, le

nucléotide est un ARNm, les taux d'expression d'ARNm de gènes qui ont été corrélés à des sites de liaison ligand-protéine connus dans un nombre prédéterminé de tissus sont des valeurs moyennes et les sites de liaison ligand-protéine connus sont mis en correspondance avec plusieurs gènes.

**8.** Procédé selon la revendication 7, dans lequel les taux moyens d'expression d'ARNm sont déterminés au moyen de la mise en correspondance d'identifiant Uniprot.

**9.** Procédé selon les revendications 1 à 8, dans lequel l'affinité du ligand pour les un ou plusieurs sites de liaison ligand-protéine est mesurée au moyen de l'anisotropie de fluorescence.

**10.** Procédé selon les revendications 1 à 9, dans lequel le ligand est choisi dans le groupe constitué d'une petite molécule ou d'un acide nucléique.

**11.** Procédé selon les revendications 1 à 10, dans lequel au moins une étape est conduite *in silico.*

**12.** Système informatique pour identifier un site de liaison de ligand d'une protéine comprenant un processeur, une mémoire et un support de stockage non transitoire lisible par ordinateur qui stocke des instructions qui, lorsqu'elles sont exécutées par le processeur, amènent le système à :

(a) générer des ensembles de base stochastiques de valeurs représentatives de sites de liaison ligand-protéine putatifs par (1) identification d'informations dans une base de données de structures de protéine, les informations correspondant à des poches de signature protéique connues et représentant au moins des coordonnées atomiques et une conformation de poche de signature protéique, et (2) conduite d'opérations d'alignement de structures apparié indépendant de l'ordre de séquence, de regroupement hiérarchique par division, et de similarité de séquence probabilistique sur les informations de poche de signature protéique identifiées pour sélectionner les ensembles de base stochastiques de valeurs représentatives de sites de liaison ligand-protéine putatifs, l'opération de similarité de séquence probabilistique comprenant la résolution d'une fonction de la distance définie par la similarité structurale et la similarité de séquence probable de sites de liaison ligand-protéine putatifs et de poches de signature protéique connues ;
(b) générer des ensembles de base de valeurs représentatives de taux d'expression nucléotidique de gènes qui ont été corrélés à des sites de liaison ligand-protéine connus dans un nombre prédéterminé d'échantillons biologiques (de préférence des échantillons de tissu ou de cellules) par mise en correspondance d'informations de site de liaison ligand-protéine connu avec un nom de gène et identification dans une base de données d'expression génique d'échantillon biologique d'informations représentatives du taux d'expression nucléotidique correspondant au gène ; et
(c) sélection de valeurs de chevauchement à partir des ensembles de base générés dans les étapes (a) et (b) en tant qu'informations qui corrèlent au moins les coordonnées atomiques et la conformation d'un ou plusieurs sites de liaison ligand-protéine.

**13.** Procédé d'identification de sites de liaison de protéine pour un nouveau ligand, le procédé comprenant les étapes de :

(a) évaluation de la liaison du nouveau ligand à un ou plusieurs sites de liaison de protéine connus, sélection de sites de liaison de protéine qui se lient au nouveau ligand avec une sélectivité spécifiée et identification d'informations représentant au moins les coordonnées atomiques et la conformation de poches de signature protéique qui se lient au ligand avec une sélectivité spécifiée ;
(b) génération d'ensembles de base stochastiques de valeurs représentatives de sites de liaison ligand-protéine putatifs par conduite d'alignement de structures apparié indépendant de l'ordre de séquence, de regroupement hiérarchique par division, et de similarité de séquence probabilistique sur les informations de poche de signature protéique identifiées pour sélectionner les ensembles de base stochastiques de valeurs représentatives de sites de liaison ligand-protéine putatifs, l'opération de similarité de séquence probabilistique comprenant la résolution d'une fonction de la distance définie par la similarité structurale et la similarité de séquence probable de sites de liaison ligand-protéine putatifs et identifié poches de signature protéique ;
(c) génération d'ensembles de base de valeurs représentatives de taux d'expression nucléotidique de gènes qui ont été corrélés à des sites de liaison ligand-protéine connus dans un nombre prédéterminé d'échantillons biologiques par mise en correspondance d'informations de site de liaison ligand-protéine connu avec un nom de gène et identification dans une base de données d'expression génique d'échantillon biologique d'informations représentatives de taux d'expression nucléotidique correspondant au gène ;
(d) sélection de valeurs de chevauchement à partir des ensembles de base générés dans les étapes (b) et (c)

en tant qu'informations qui corrèlent au moins les coordonnées atomiques et la conformation d'un ou plusieurs sites de liaison ligand-protéine ; et
(e) validation des un ou plusieurs sites de liaison ligand-protéine déterminés dans l'étape (d) *in vitro* par mesure de l'affinité du ligand pour les un ou plusieurs sites de liaison ligand-protéine.

**14.** Procédé selon la revendication 13, dans lequel le nucléotide est un ARNm, les échantillons biologiques sont des échantillons de tissu et :

(1) la fonction de la distance définie par la similarité structurale et la similarité de séquence probable de sites de liaison ligand-protéine putatifs et de poches de signature protéique identifiées est représentée par la formule :

$$Score = Similarit\acute{e}\ structurale + \alpha^*(1 - Similarit\acute{e}\ de\ s\acute{e}quence)$$

$$Similarit\acute{e}\ structurale = \frac{RMSD}{N^{1/3}}$$

$$Similarit\acute{e}\ de\ s\acute{e}quence = \frac{Score\ de\ s\acute{e}quence}{Meilleur\ score\ de\ s\acute{e}quence}$$

$$Score\ de\ s\acute{e}quence = \sum_i (AtomFreq_i + ResFreq_i)$$

$$Meilleur\ score\ de\ s\acute{e}quence = \sum_i (MaxAtomFreq_i + MaxResFreq_i)$$

où $\alpha$ est 1,2, *RMSD* est la distance quadratique moyenne après l'alignement, *N* est le nombre de positions alignées, $AtomFreq_i$ / $ResFreq_i$ est la fréquence d'atome/résidu aligné à la position *i*, et $MaxAtomFreq_i$ / $MaxResFreq_i$ est la fréquence la plus élevée d'un atome/résidu quelconque à la position *i* et la sommation est effectuée sur toutes les positions alignées ; et
(2) la fonction de la distance définie par la similarité structurale, la similarité de séquence probable de sites de liaison ligand-protéine putatifs et de poches de signature protéique identifiées et les taux d'expression d'ARNm tissulaires relatifs dans le nombre prédéterminé de tissus est représentée par la formule :

$$Score = Similarit\acute{e}\ structurale + \alpha^*(1 - Similarit\acute{e}\ de\ s\acute{e}quence + \beta^*Expression\ tissulaire)$$

$$Expression\ tissulaire = 1 - \frac{Nombre\ de\ tissus\ avec\ expression\ correspondante}{Nombre\ total\ de\ tissus}$$

où $\alpha$ et le terme de similarité structurale et le terme de similarité de séquence sont tels que définis ci-dessus et $\beta$ est une valeur comprise entre 0,1 et 0,9.

**15.** Système de réseau pour identifier un site de liaison de ligand d'une protéine, le système de réseau comprenant :

(a) un système informatique comprenant un processeur, une mémoire et un support de stockage non transitoire lisible par ordinateur qui stocke des instructions qui, lorsqu'elles sont exécutées par le processeur, amènent le système à :

(1) générer des ensembles de base stochastiques de valeurs représentatives de sites de liaison ligand-protéine putatifs par (i) identification d'informations dans une base de données de structures de protéine, les informations correspondant à des poches de signature protéique connues et représentant au moins

des coordonnées atomiques et une conformation de poche de signature protéique, et (ii) conduite d'opérations d'alignement apparié de structures indépendant de l'ordre de séquence, de regroupement hiérarchique par division, et de similarité de séquence probabilistique sur les informations de poche de signature protéique identifiées pour sélectionner les ensembles de base stochastiques de valeurs représentatives de sites de liaison ligand-protéine putatifs, l'opération de similarité de séquence probabilistique comprenant la résolution d'une fonction de la distance définie par la similarité structurale et la similarité de séquence probable de sites de liaison ligand-protéine putatifs et de poches de signature protéique connues ;

(2) générer des ensembles de base de valeurs représentatives de taux d'expression nucléotidique de gènes qui ont été corrélés à des sites de liaison ligand-protéine connus dans un nombre prédéterminé d'échantillons biologiques (de préférence des échantillons de tissu ou de cellules) par mise en correspondance d'informations de site de liaison ligand-protéine connu avec un nom de gène et identification dans une base de données d'expression génique d'échantillon biologique représentatives de taux d'expression nucléotidique correspondant au gène ; et

(3) sélection de valeurs de chevauchement à partir des ensembles de base générés dans les étapes (1) et (2) en tant qu'informations qui corrèlent au moins les coordonnées atomiques et la conformation d'un ou plusieurs sites de liaison ligand-protéine ; et

(b) un dosage à haut débit qui est mis en réseau avec le système informatique pour la transmission d'informations représentatives de sites de liaison ligand-protéine validés déterminées par mesure de l'affinité du ligand pour les sites de liaison ligand-protéine prédits.

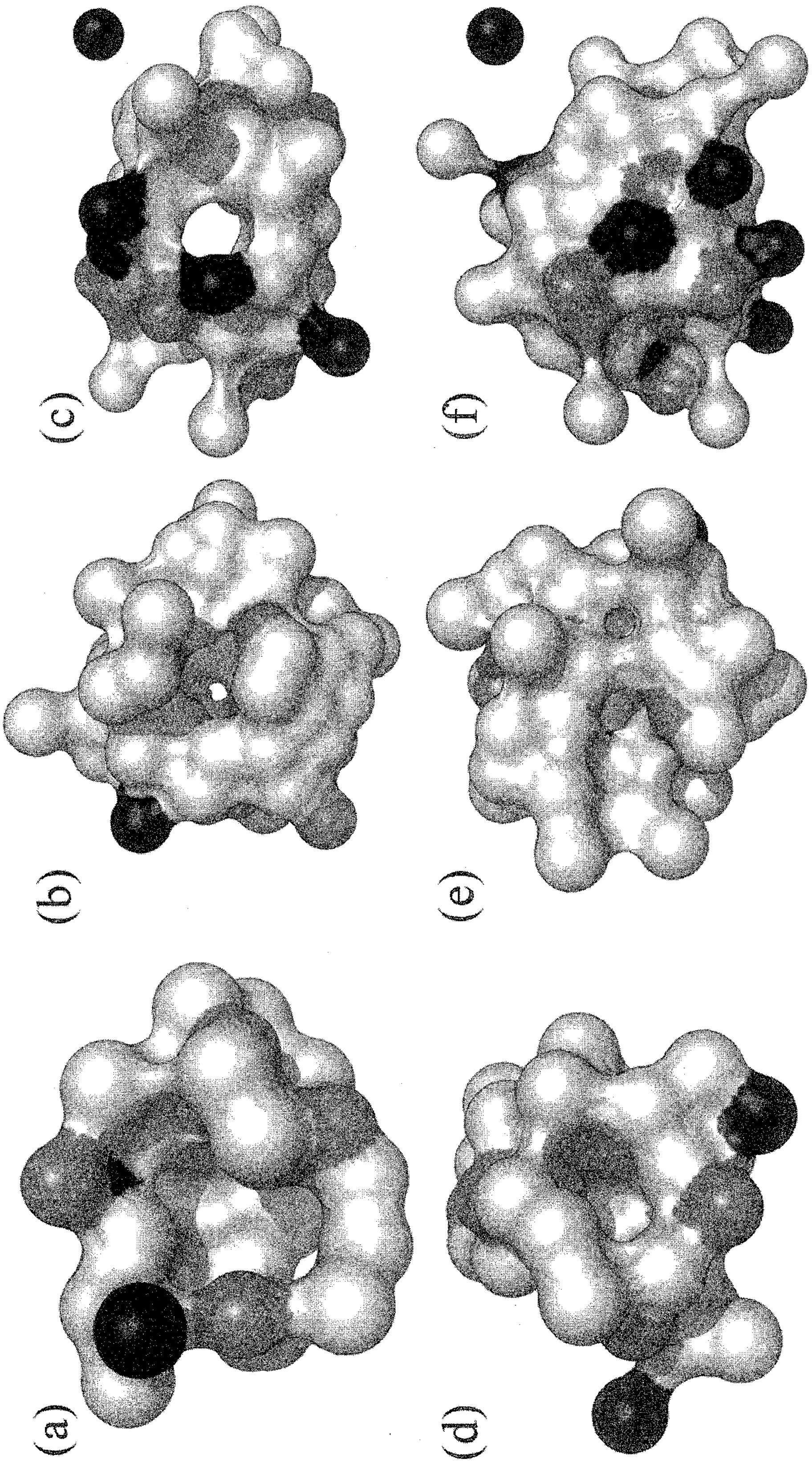


FIGURE 1

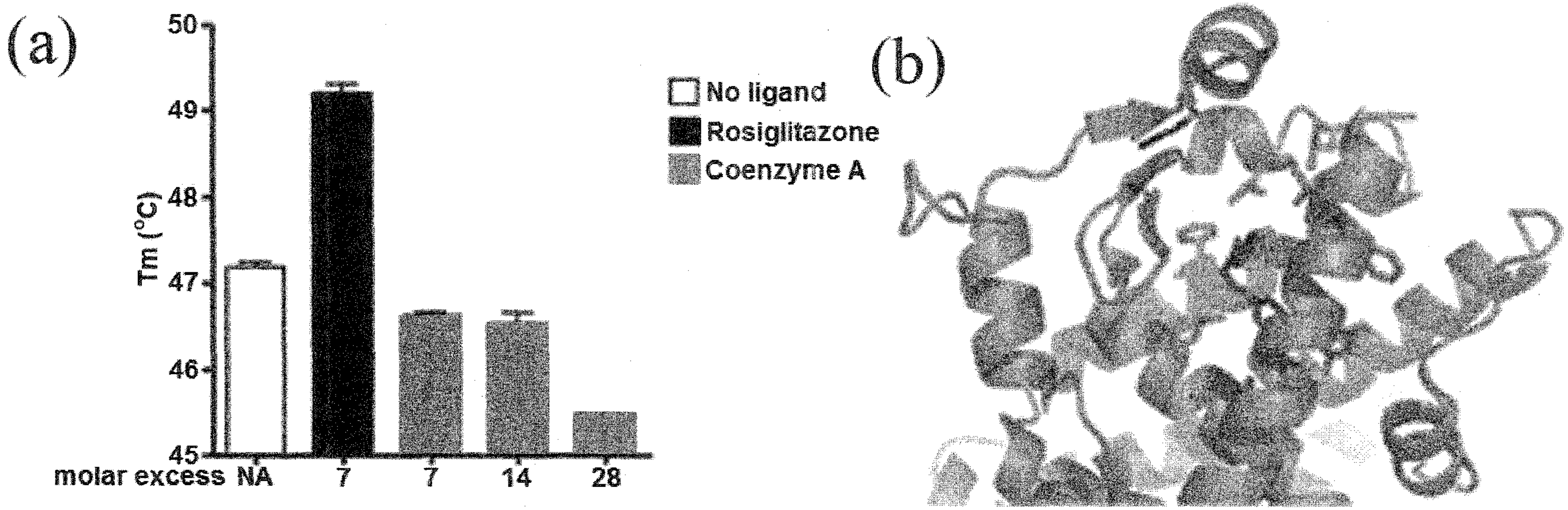
(a)
Tm (°C)
50
49
48
47
46
45
molar excess NA
7
7
14
28
No ligand
Rosiglitazone
Coenzyme A
(b)

FIGURE 2

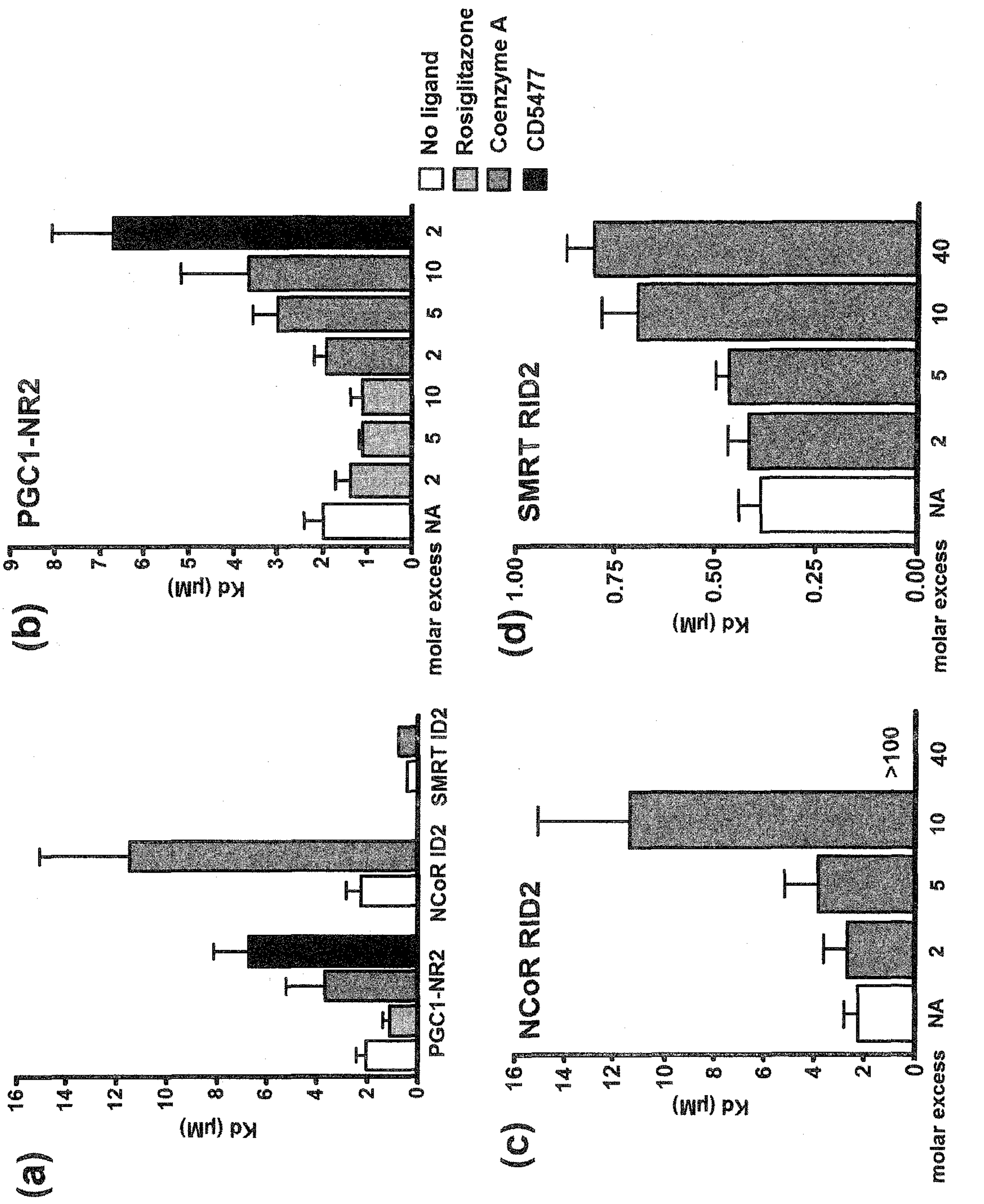


FIGURES 3a-3d

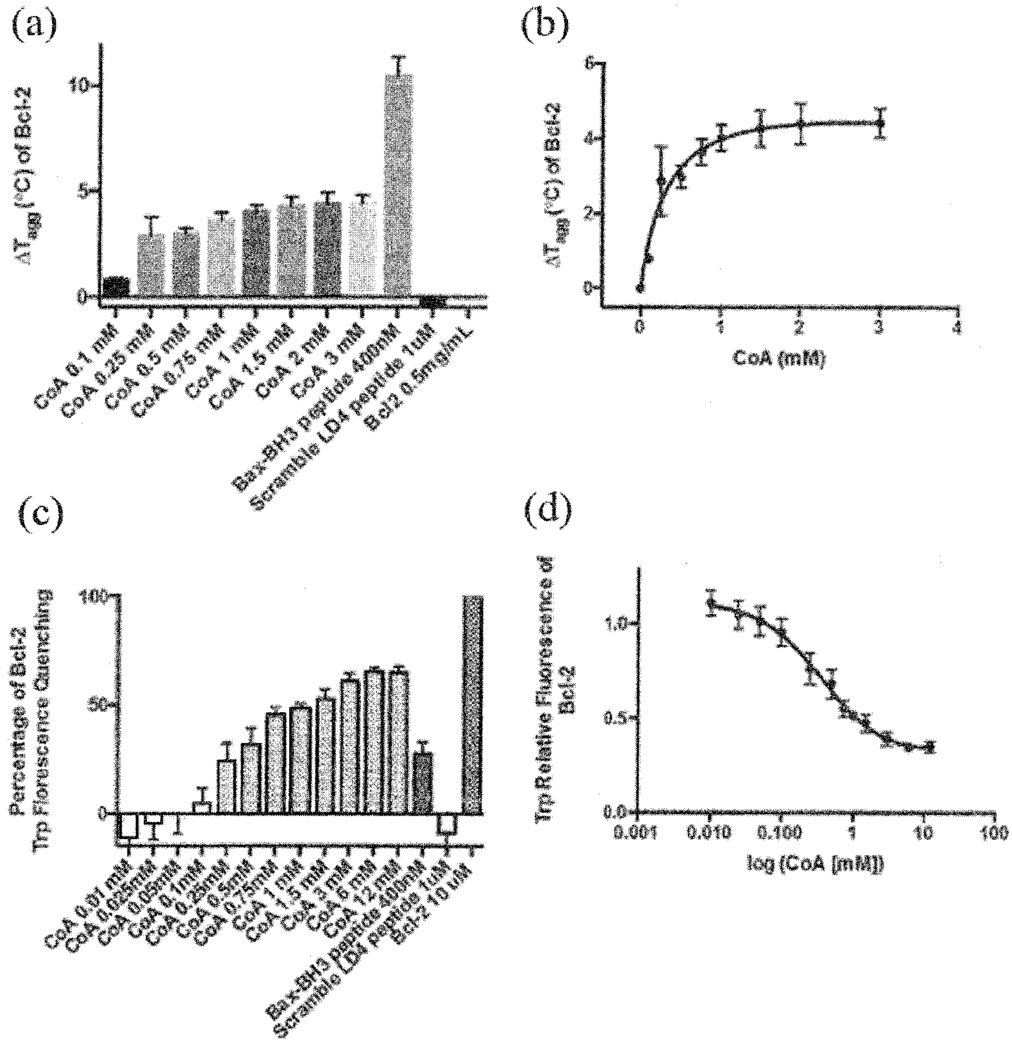
(a)
ΔT_agg (°C) of Bcl-2
10
5
0
CoA 0.1 mM
CoA 0.25 mM
CoA 0.5 mM
CoA 0.75 mM
CoA 1 mM
CoA 1.5 mM
CoA 2 mM
CoA 3 mM
Bax-BH3 peptide 400nM
Scramble LD4 peptide 1uM
Bcl2 0.5mg/mL
(b)
ΔT_agg (°C) of Bcl-2
6
4
2
0
0
1
2
3
4
CoA (mM)
(c)
Percentage of Bcl-2
Trp Florescence Quenching
100
50
0
CoA 0.01 mM
CoA 0.025mM
CoA 0.05mM
CoA 0.1mM
CoA 0.25mM
CoA 0.5mM
CoA 0.75mM
CoA 1 mM
CoA 1.5 mM
CoA 3 mM
CoA 6 mM
CoA 12 mM
Bax-BH3 peptide 400nM
Scramble LD4 peptide 1uM
Bcl-2 10 uM
(d)
Trp Relative Fluorescence of
Bcl-2
1.0
0.5
0.0
0.001
0.010
0.100
1
10
100
log (CoA [mM])

# FIGURE 4

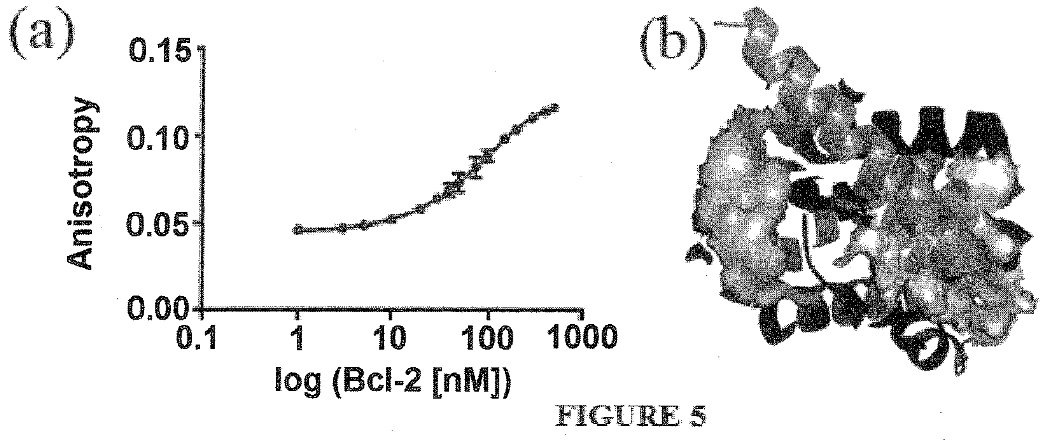


FIGURE 5

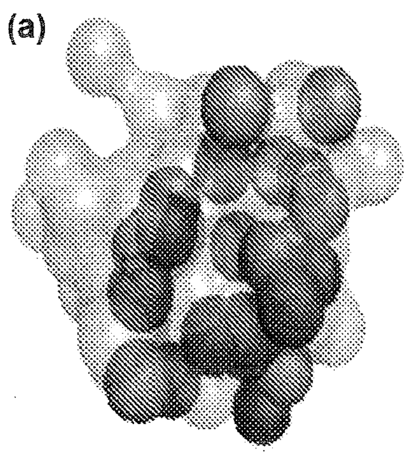


FIGURE 6a

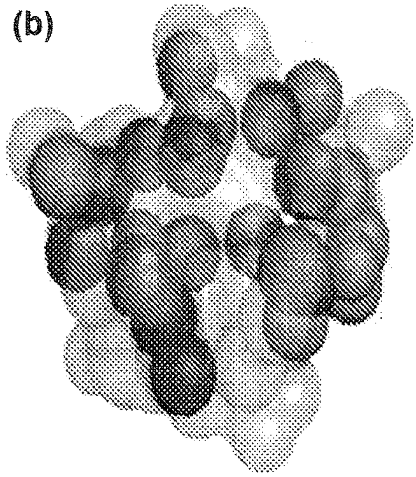


FIGURE 6b

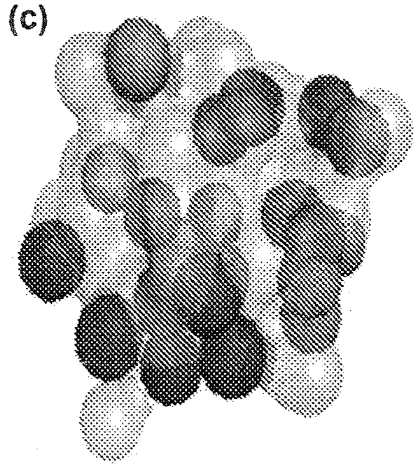


FIGURE 6c

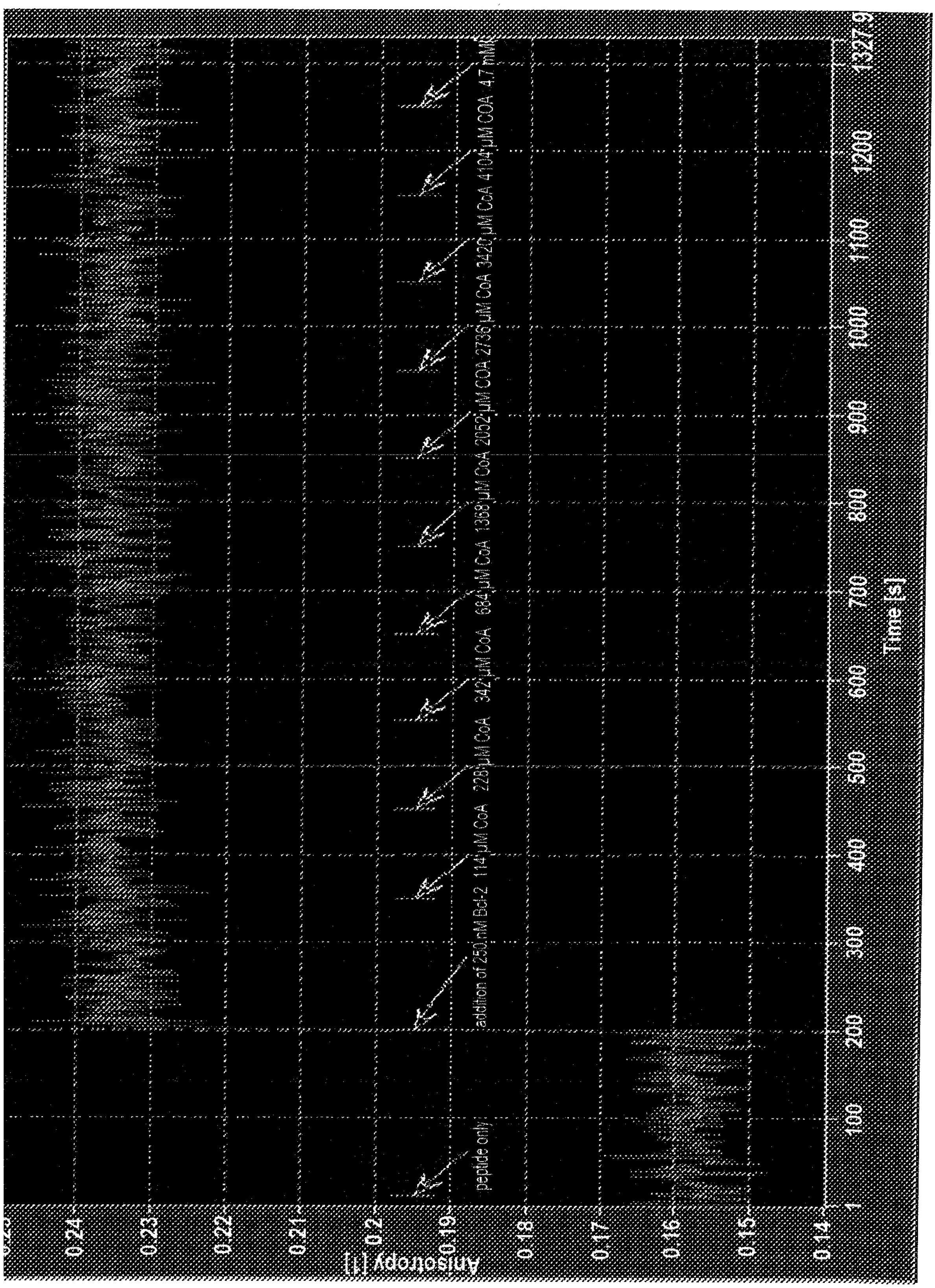

peptide only
addition of 250 nM Bcl-2
114 µM CoA
228 µM CoA
342 µM CoA
684 µM CoA
1368 µM CoA
2052 µM COA
2736 µM COA
3420 µM CoA
4104 µM CoA
4.7 mM
Anisotropy [1]
Time [s]
0.24
0.23
0.22
0.21
0.2
0.19
0.18
0.17
0.16
0.15
0.14
1
100
200
300
400
500
600
700
800
900
1000
1100
1200
1327.9


FIGURE 7

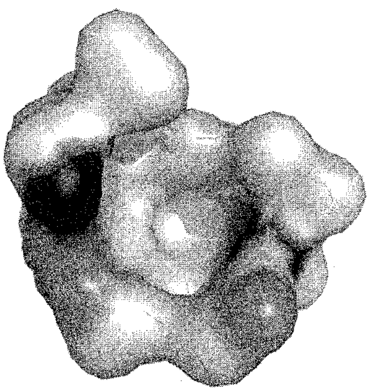

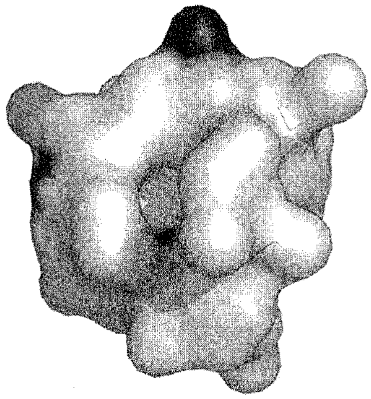

| 3D Structure | Drug | Known Targets | Sensitivity | Specificity |
|---|---|---|---|---|
| 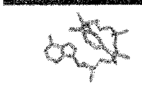 | Nicotinamide-Adenine-Dinucleotide | 129 | 74% | 87% |
| NA | Heme | 104 | 77% | 78% |
| 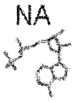 | Adenosine-5'-Diphosphate | 101 | 63% | 67% |
| 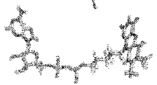 | Flavin-Adenine Dinucleotide | 73 | 64% | 81% |
| 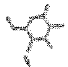 | Beta-D-Glucose | 67 | 66% | 82% |
| 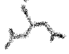 | Citric Acid | 62 | 65% | 71% |
| 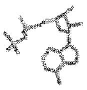 | 2'-Monophosphoadenosine 5'-Diphosphoribose | 52 | 55% | 84% |
| 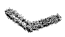 | Formic Acid | 47 | 57% | 77% |
| 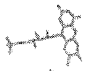 | Riboflavin Monophosphate | 47 | 71% | 72% |
| 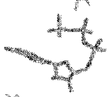 | Phosphoaminophosphonic Acid-Adenylate Ester | 42 | 45% | 88% |
| 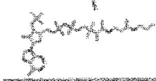 | Coenzyme A | 40 | 60% | 87% |
| | Average | 69 | 63% | 79% |

# FIGURE 8

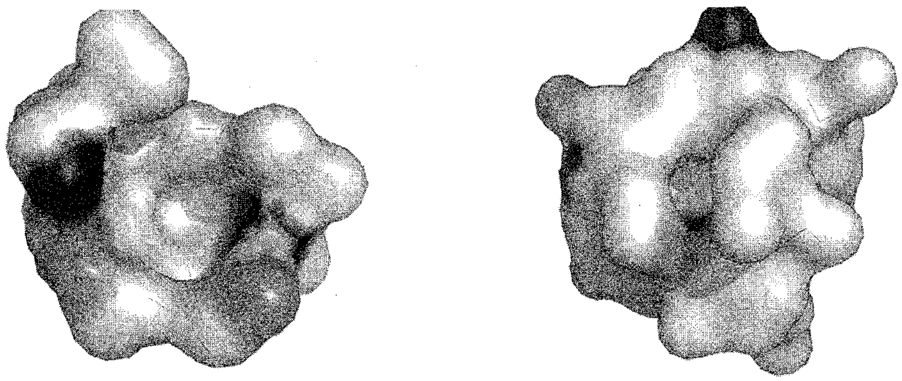

| Drug | Known Targets | Sensitivity |
|---|---|---|
| Flavin-Adenine Dinucleotide | 73 | 64% |
| Beta-D-Glucose | 68 | 66% |
| Citric Acid | 63 | 65% |
| 2'-Monophosphoadenosine 5'-Diphosphoribose | 52 | 55% |
| Formic Acid | 48 | 57% |
| Riboflavin Monophosphate | 47 | 71% |
| Phosphoaminophosphonic Acid-Adenylate Ester | 42 | 45% |
| Average | 56 | 60% |

FIGURE 9

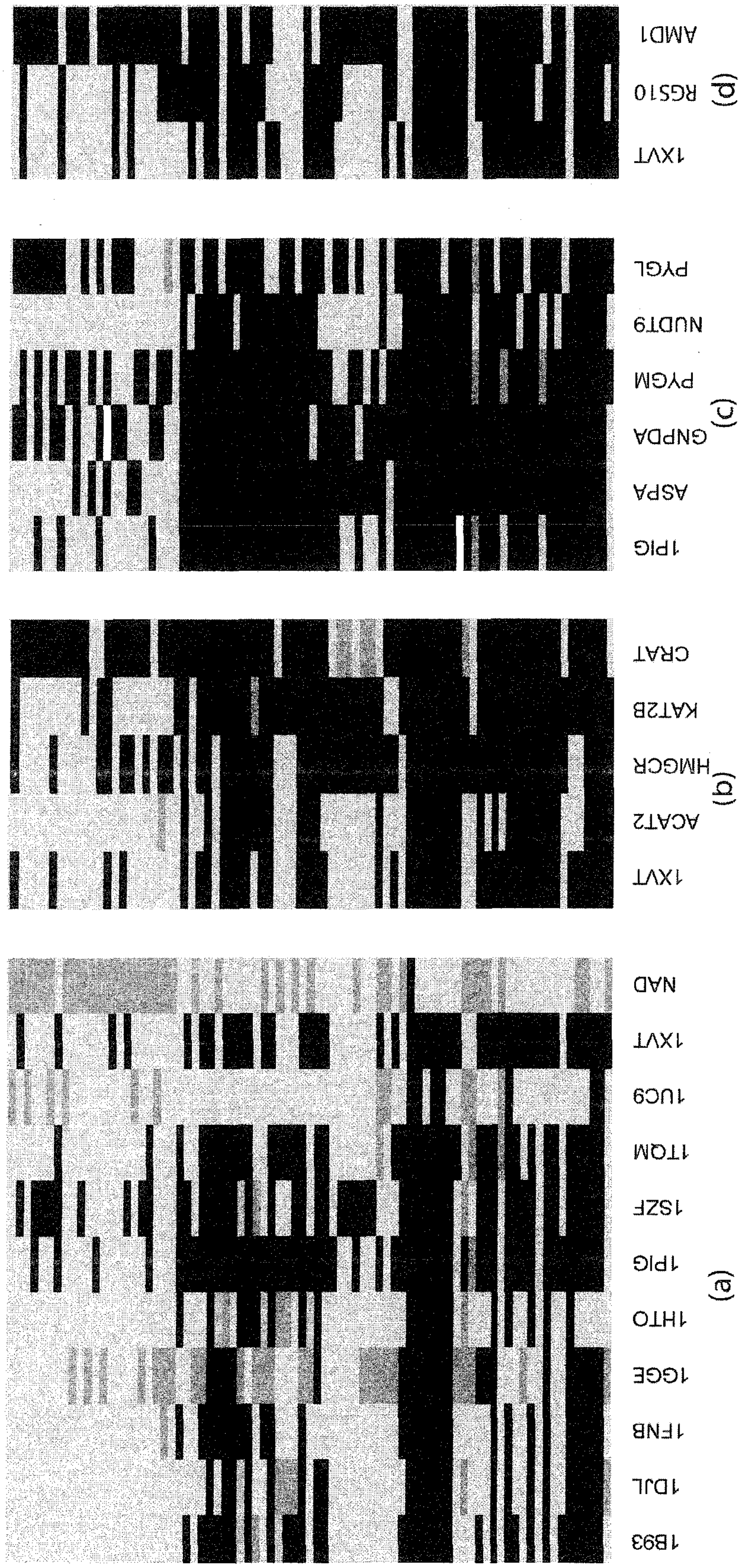
(a)
1B93
1DJL
1FNB
1GGE
1HTO
1PIG
1SZF
1TQM
1UC9
1XVT
NAD
(b)
1XVT
ACAT2
HMGCR
KAT2B
CRAT
(c)
1PIG
ASPA
GNPDA
PYGM
NUDT9
PYGL
(d)
1XVT
RGS10
AMD1

FIGURE 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130280238 A
- US 20140275487 A
- US 20110274692 A
- US 20140316116 A
- WO 04083443 A1
- US 20030138829 A1
- US 20030008308 A1
- WO 04067175 A1
- US 2004083443 A1
- US 20040110275 A1
- US 20040121364 A1


### Non-patent literature cited in the description


- **SAMBROOK et al.** Molecular Cloning: A Laboratory Manual. 2001
- Current Protocols in Molecular Biology. 1994, vol. I-III
- Cell Biology: A Laboratory Handbook. 1994, vol. I-III
- Current Protocols in Immunology. 1984, vol. I-III
- Oligonucleotide Synthesis. 1985
- Nucleic Acid Hybridization. 1984
- Transcription And Translation
- Animal Cell Culture. IRL Press, 1986
- **PERBAL.** Immobilized Cells And Enzymes. 1984
- A Practical Guide To Molecular Cloning
- **DUNDAS et al.** Structural Signatures of Enzyme Binding Pockets from Order-Independent Surface Alignment: A Study of Metalloendopeptidase and NAD Binding Proteins. *J Mol Biol.,* 11 March 2011, vol. 406 (5), 713-729
- **DUNDAS.** Protein Function Prediction for Omics Era. Springer Science & Business Media, 19 April 2011
- **SU, A. et al.** A gene atlas of the mouse and human protein-encoding transcriptomes. *Proc Natl. Acad. Sci. USA,* 2004, vol. 101, 6062-6067
- **GRASSOT et al.** RTKdb: database of receptor tyrosine kinase. *Nucleic Acids Res,* 2003, vol. 31, 353-358
- **HORN et al.** GPCRDB information system for G protein-coupled receptors. *Nucleic Acids Res,* 2003, vol. 31, 294-297
- **SKOUFOS et al.** Olfactory receptor database: a sensory chemoreceptor resource. *Nucleic Acids Res,* 2000, vol. 28, 341-343
- **FUHRER et al.** The thyrotropin receptor mutation database: update 2003. *Thyroid,* 2003, vol. 13, 1123-1126
- **PATTERSON et al.** The androgen receptor gene mutations database. *Nucleic Acids Res,* 1994, vol. 22, 3560-3562
- **GOTTLIEB et al.** The androgen receptor gene mutations database. *Nucleic Acids Res,* 1998, vol. 26, 234-238
- **NAKATA et al.** Development of the receptor database (RDB): application to the endocrine disruptor problem. *Bioinformatics,* 1999, vol. 15, 544-552
- **JOSHI-TOPE et al.** Reactome: a knowledgebase of biological pathways. *Nucleic Acids Res,* 2005, vol. 33, D428-D432
- **PERI et al.** Development of human protein reference database as an initial platform for approaching systems biology in humans. *Genome Res,* 2003, vol. 13, 2363-2371
- **POROLLO et al.** Versatile annotation and publication quality visualization of protein complexes using POLYVIEW-3D. *BMC Bioinformatics,* 2007, vol. 8, 316
- Fluorescence anisotropy measurements in solution: Methods and reference materials (IUPAC Technical Report). *Pure Appl. Chem.,* 2013, vol. 85 (3), 589-608
- **REDDY, A. ; ZHANG, S.** Polypharmacology: drug discovery for the future. *Expert Rev. Clin. Pharmacol.,* 2013, vol. 6, 41-47
- **ARROWSMITH, J.** Trial watch: phase III and submission failures: 2007-2010. *Nat. Rev. Drug Discov.,* 2011, vol. 10, 87
- **ARROWSMITH, J.** Trial watch: Phase II failures: 2008-2010. *Nat. Rev. Drug Discov.,* 2011, vol. 10, 328-329
- **LIEBLER, D. ; GUENGERICH, F.** Elucidating mechanisms of drug-induced toxicity. *Nat. Rev. Drug Discov.,* 2005, vol. 4, 410-420

- **MESTRES, J. ; GREGORI-PUIGJANE, E. ; VALVERDE, S. ; SOLE R.** The topology of drug-target interaction networks: implicit dependence on drug properties and target families. *Mol. Biosyst.,* 2009, vol. 5, 1051-1057
- **LOUNKINE, E. et al.** Large-scale prediction and testing of drug activity on side-effect targets. *Nature,* 2012, vol. 486, 361-367
- **PETERS.** J. Polypharmacology - foe or friend?. *J. Med. Chem.,* 2013, vol. 56, 8955-8971
- **ASHBURN, T. ; THOR, K.** Drug repositioning: identifying and developing new uses for existing drugs. *Nat. Rev. Drug Discov.,* 2004, vol. 3, 673-683
- **KINNINGS, S. et al.** Drug discovery using chemical systems biology: repositioning the safe medicine comtan to treat multi-drug and extensively drug resistant tuberculosis. *PLoS Comput. Biol.,* 2009, vol. 5, e1000423
- **ENGIN, H. ; KESKIN, O. ; NUSSINOV, R. ; GURSOY, A.** A strategy based on protein-protein interface motifs may help in identifying drug off-targets. *J. Chem. Inf. Model.,* 2012, vol. 52, 2273-2286
- **CHANG, R. ; XIE, L. ; XIE, L. ; BOURNE, P. ; PALSSON, B.** Drug off-target effects predicted using structural analysis in the context of a metabolic network model. *PLoS Comput. Biol.,* 2010, vol. 6, e1000938
- **LI, Y.Y. ; AN, J. ; JONES, S.J.M.** A computational approach to finding novel targets for existing drugs. *PLoS Comput. Biol.,* 2011, vol. 7 (9), e1002139
- **IORIO, F. et al.** Discovery of drug mode of action and drug repositioning from transcriptional responses. *Proc. Natl. Acad. Sci USA,* 2010, vol. 107, 14621-14626
- **WEI, G. et al.** Gene expression-based chemical genomics identifies rapamycin as a modulator of MCL1 and glucocorticoid resistance. *Cancer Cell,* 2006, vol. 10, 331-342
- **CHEN, B. ; WILD, D. ; GUHA, R.** Pubchem as a source of polypharmacology. *J. Chem. Inf. Model.,* 2009, vol. 49, 2044-2055
- **HU, G. ; AGARWAL, P.** Human disease-drug network based on genomic expression profiles. *PLoS One,* 2009, vol. 4, e6536
- **SUTHRAM, S. et al.** Network-based elucidation of human disease similarities reveals common functional modules enriched for pluripotent drug targets. *PLoS Comput. Biol.,* 2010, vol. 6, e1000662
- **EMIG, D. et al.** Drug target prediction and repositioning using an integrated network-based approach. *PLoS One,* 2013, vol. 8 (4), e60618
- **LAMB, J. et al.** The connectivity map: using gene-expression signatures to connect small molecules, genes, and disease. *Science,* 2006, vol. 313, 1929-1935
- **KEISER, M. et al.** Predicting new molecular targets for known drugs. *Nature,* 2009, vol. 462, 175-181
- **NOESKE, T. et al.** Predicting compound selectivity by self-organizing maps: cross-activities of metabotropic glutamate receptor antagonists. *ChemMedChem,* 2006, vol. 1, 1066-1068
- **QU, X. ; GUDIVADA, R. ; JEGGA, A. ; NEUMANN, E. ; ARONOW, B.** Inferring novel disease indications for known drugs by semantically linking drug action and disease mechanism relationships. *BMC Bioinformatics,* 2009, vol. 10 (5), S4
- **CAMPILLOS, M. ; KUHN, M. ; GAVIN, A. ; JENSEN, L. ; BORK, P.** Drug target identification using side-effect similarity. *Science,* 2008, vol. 321, 263-266
- **SANSEAU, P. et al.** Use of genome-wide association studies for drug repositioning. *Nat. Biotechnol.,* 2012, vol. 30, 317-320
- **WANG, Z. ; ZHANG, H.** Rational drug repositioning by medical genetics. *Nat. Biotechnol.,* 2013, vol. 31, 1080-1082
- **GOTTLIEB, A. ; STEIN, G.Y. ; RUPPIN, E. ; SHARAN, R.** PREDICT: a method for inferring novel drug indications with application to personalized medicine. *Mol. Sys. Biol.,* 2011, vol. 7, 496
- **NAPOLITANO, F. et al.** Drug repositioning: a machine-learning approach through data integration. *J. Cheminform.,* 2013, vol. 5, 30
- **GAO, M. ; SKOLNICK, J.** A comprehensive survey of small-molecule binding pockets in proteins. *PLoS Comput. Biol.,* 2013, vol. 9, e1003302
- **DUNDAS, J. ; ADAMIAN, L. ; LIANG, J.** Structural signatures of enzyme binding pockets from order-independent surface alignment: a study of metalloendopeptidase and NAD binding proteins. *J. Mol. Biol.,* 2011, vol. 406, 713-729
- **TSENG, Y. ; LIANG, J.** Estimation of amino acid residue substitution rates at local spatial regions and application in protein function inference: a bayesian monte carlo approach. *Mol. Biol. Evol.,* 2006, vol. 23, 421-436
- **DUNDAS, J. et al.** CASTp: computed atlas of surface topography of proteins with structural and topographicalmapping of functionally annotated residues. *Nucleic Acids Res.,* 2006, vol. 34, W 116-8
- **HUANG, B.D. ; SCHROEDER, M.** LIGSITEcsc: predicting ligand binding sites using the Connolly surface and degree of conservation. *BMC Struct. Biol.,* 2006, vol. 6, 19
- **BRADY, G. JR ; STOUTEN, P.** Fast prediction and visualization of protein binding pockets with PASS. *J. Comput. Aided Mol. Des.,* 2000, vol. 14, 383-401

- **PETERS, K. ; FAUCK, J. ; FROMMEL, C.** The automatic search for ligand binding sites in proteins of known three-dimensional structure using only geometric criteria. *J. Mol. Biol.,* 1996, vol. 256, 201-213
- **WATSON, J. ; LASKOWSKI, R. ; THORNTON, J.** Predicting protein function from sequence and structural data. *Curr. Opin. Struct. Biol.,* 2005, vol. 15, 275-284
- **JANSEN, R. ; GREENBAUM, D. ; GERSTEIN, M.** Relating whole-genome expression data with protein-protein interactions. *Genome Res.,* 2002, vol. 12, 37-46
- **QIAO, N. ; HUANG, Y. ; NAVEED, H. ; GREEN, C. ; HAN, J.** Cociter: an efficient tool to infer gene function by assessing the significance of literature co-citation. *PLoS One,* 2013, vol. 8, e74074
- **SWEDENBORG, E. ; RUEGG, J. ; MAKELA, S. ; PONGRATZ, I.** Endocrine disruptive chemicals: mechanisms of action and involvement in metabolic disorders. *J. Mol. Endocrinol.,* 2009, vol. 43 (1), 1-10
- **LE MAIRE et al.** Activation of RXR-PPAR heterodimers by organotin environmental endocrine disruptors. *EMBO Rep.,* 2009, vol. 10 (4), 367-73
- **LI, Y. ; LAMBERT, M.H. ; XU, H.E.** Activation of nuclear receptors: a perspective from structural genomics. *Structure,* 2003, vol. 11 (7), 741-6
- **LEONARDI, R. ; ZHANG, Y-M. ; ROCK, C.O. ; JACKOWSKI, S.** Coenzyme A: Back in action. *Progress in Lipid Res.,* 2005, vol. 44 (2-3), 125-153
- **KU, B. ; LIANG, C. ; JUNG, J.U. ; OH, B.H.** Evidence that inhibition of BAX activation by BCL-2 involves its tight and preferential interaction with the BH3 domain of BAX. *Cell Res.,* 2011, vol. 21, 627-641
- **OGU, C. ; MAXA, J.** Drug interactions due to cytochrome p450. *Proc. (Bayl. Univ. Med. Cent.),* 2000, vol. 13, 421-423
- **MCGEHEE, R. JR ; RONIS, M. ; COWHERD, R. ; INGELMAN-SUNDBERG, M. ; BADGER, T.** Characterization of cytochrome p450 2e1 induction in a rat hepatoma FGC-4 cell model by ethanol. *Biochem. Pharmacol.,* 1994, vol. 48, 1823-1833
- **NANJI, A. et al.** Markedly enhanced cytochrome p450 2e1 induction and lipid peroxidation is associated with severe liver injury in fish oil-ethanol-fed rats. *Alcohol Clin. Exp. Res.,* 1994, vol. 18, 1280-1285
- **WU, D. ; CEDERBAUM, A.** Ethanol and arachidonic acid produce toxicity in hepatocytes from pyrazole-treated rats with high levels of CYP2E1. *Mol. Cell Biochem.,* 2000, vol. 204, 157-167
- **WU, D. ; CEDERBAUM, A.** Cyclosporine a protects against arachidonic acid toxicity in rat hepatocytes: role of CYP2E1 and mitochondria. *Hepatology,* 2002, vol. 35, 1420-1430
- **HAMOSH, A. ; SCOTT, A. ; AMBERGER, J. ; VALLE, D. ; MCKUSICK, V.** Online mendelian inheritance in man (OMIM). *Hum. Mutat.,* 2000, vol. 15, 57-61
- **STENSON, P. et al.** The human gene mutation database: building a comprehensive mutation repository for clinical and molecular genetics, diagnostic testing and personalized genomic medicine. *Hum. Genet.,* 2014, vol. 133, 1-9
- **DILLER, D. ; LI, R.** Kinases, homology models, and high throughput docking. *J. Med. Chem.,* 2003, vol. 46, 4638-4647
- **KNOX, C. et al.** Drugbank 3.0: a comprehensive resource for 'omics' research on drugs. *Nucleic Acids Res.,* 2011, vol. 39, D1035-41
- **PEROT, S. ; SPERANDIO, O. ; MITEVA, M.A. ; CAMPROUX, A.C. ; VILLOUTREIX, B.O.** Druggable pockets and binding site centric chemical space: a paradigm shift in drug discovery. *Drug Disc. Today,* 2010, vol. 15, 656-667
- **WANG, G. ; DUNBRACK, R.** Jr. PISCES: a protein sequence culling server. *Bioinformatics,* 2003, vol. 19, 1589-1591
- **CAVALLO, L. ; KLEINJUNG, J. ; FRATERNALI, F.** POPS: A fast algorithm for solvent accessible surface areas at atomic and residue level. *Nucleic Acids Res.,* 2003, vol. 31, 3364-3366
- **WU, C. et al.** The universal protein resource (uniprot): an expanding universe of protein information. *Nucleic Acids Res.,* 2006, vol. 34, D187-91
- **RIU, A. et al.** Peroxisome proliferator-activated receptor γ is a target for halogenated analogs of bisphenol A. *Environ. Health Perspect.,* 2011, vol. 119 (9), 1227-32
- **SHEIBANI, N. ; TANG, Y. ; SORENSON, C. M.** Paxillin's LD4 motif interacts with bcl-2. *J. Cell. Physiol.,* 2008, vol. 214, 655-661
- **PANTOLIANO, M.W. et al.** High-Density Miniaturized Thermal Shift Assays as a General Strategy for Drug Discovery. *J. Biomol. Screen.,* 2001, vol. 6 (6), 429-440
- *Nature Reviews Drug Discovery,* October 2006, vol. 5, 821-834
- **ALAM, T. et al.** How to find a leucine in a haystack? Structure, ligand recognition and regulation of Leucine-Aspartic acid (LD) motifs. *Biochem. J.,* 2014, vol. 460, 317-329
- **CUI, X. et al.** Finding optimal interaction interface alignments between biological complexes. *Bioinformatics,* 2015, vol. 31 (12), i133-i141
- **DUNDAS, J. et al.** Topology independent protein structural alignment. *BMC Bioinformatics,* 2007, vol. 8, 388
- **DUNDAS, J. et al.** Structural signatures of enzyme binding pockets from order-independent surface alignment: a study of metalloendopeptidase and NAD binding proteins. *J. Mol. Biol.,* 2011, vol. 406, 713-729

- **LASKOWSKI, R.A.** SURFNET: A program for visualizing molecular surfaces, cavities, and intermolecular interactions. *J. Mol. Graph.,* 1995, vol. 13, 323-330
- **LEMAIRE, A. et al.** Activation of RXR-PPAR heterodimers by organotin environmental endocrine disruptors. *EMBO Rep.,* 2009, vol. 10, 367-373
- **LIANG, J. et al.** Anatomy of protein pockets and cavities: Measurement of binding site geometry and implications for ligand design. *Prot. Sci.,* 1998, vol. 7, 1884-1897
- **PETERS, J.** Polypharmacology-foe or friend?. *J. Med. Chem.,* 2013, vol. 56, 8955-8971
- **WANG, G. ; DUNBRACK,R.JR.** PISCES: a protein sequence culling server. *Bioinformatics,* 2003, vol. 19, 1589-1591